(19) Europäisches Patentamt
European Patent Office
Office européen des brevets

(11) EP 4 328 244 A1

(12) EUROPEAN PATENT APPLICATION
published in accordance with Art. 153(4) EPC

(43) Date of publication:
**28.02.2024 Bulletin 2024/09**

(21) Application number: **22791777.0**

(22) Date of filing: **21.04.2022**

(51) International Patent Classification (IPC):
***C07K 16/30*** (2006.01) ***A61K 39/395*** (2006.01)
***A61K 47/68*** (2017.01) ***A61K 51/02*** (2006.01)
***A61K 51/10*** (2006.01) ***A61P 35/00*** (2006.01)
***A61P 35/04*** (2006.01) ***C07K 16/46*** (2006.01)
***C12N 15/13*** (2006.01)

(52) Cooperative Patent Classification (CPC):
**A61K 39/395; A61K 47/68; A61K 51/02; A61K 51/10; A61P 35/00; A61P 35/04; C07K 16/00; C07K 16/30; C07K 16/46**

(86) International application number:
**PCT/JP2022/018413**

(87) International publication number:
**WO 2022/225006 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.04.2021 JP 2021072201**

(71) Applicants:
- **Nihon Medi-Physics Co., Ltd.**
  **Tokyo 136-0075 (JP)**
- **Sumitomo Pharma Co., Ltd.**
  **Osaka 541-0045 (JP)**

(72) Inventors:
- **YAMADA, Masato**
  **Tokyo 136-0075 (JP)**
- **TAKENAKA, Fumiaki**
  **Tokyo 136-0075 (JP)**
- **HARADA, Kotaro**
  **Tokyo 136-0075 (JP)**
- **OMURA, Momoko**
  **Tokyo 136-0075 (JP)**
- **MATONO, Mitsuhiro**
  **Suita-shi, Osaka 564-0053 (JP)**
- **OTSUKI, Kumiko**
  **Suita-shi, Osaka 564-0053 (JP)**
- **OCHIAI, Yasushi**
  **Tokyo 104-8356 (JP)**
- **MURAKAMI, Takayuki**
  **Suzuka-shi, Mie 513-0818 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) HUMANISED ANTIBODIES LABELLED WITH RADIONUCLIDES THAT EMIT ?-RAYS

(57) The present invention aims to provide a radionuclide-labeled anti-MUC5AC humanized antibody that is superior in the specificity for mucin subtype 5AC (MUC5AC) and accumulation in tumor. The present invention provides a conjugate of a radionuclide and an antibody, wherein the radionuclide is a nuclide that emits β-rays, and the antibody is a humanized antibody that specifically binds to mucin subtype 5AC and has a heavy chain variable region consisting of the amino acid sequence shown in any of SEQ ID NOs: 1 to 4, and a light chain variable region consisting of the amino acid sequence shown in any of SEQ ID NOs: 5 to 8.

EP 4 328 244 A1

## Description

[Technical Field]

The present invention relates to conjugates of a radionuclide or a chelating agent chelated with a radionuclide, and a mucin subtype 5AC specific humanized antibody, radiopharmaceuticals containing same, and use thereof.

[Background Art]

Mucin is the main component of mucus secreted from epithelial cell and the like of animal and is a glycoprotein containing a large amount of sugar with a molecular weight of 1 - 10 million. Mucin includes secretory mucin produced by epithelial cell and the like and membrane-bound mucin that has a hydrophobic transmembrane site and exists while being bound to the cell membrane. The core proteins of mucin are collectively called MUC, and it is known that there are at least 20 types of genes encoding core proteins. One of them, mucin subtype 5AC (MUC5AC), belongs to secretory mucin.

MUC5AC is expressed in the stomach and trachea in normal tissues, and overexpression in pancreatic cancer has been reported. Overexpression has also been reported in thyroid cancer, liver cancer, colorectal cancer, gastric cancer, urothelial cancer, breast cancer, cervical cancer, ovarian cancer, endometrial cancer, and bile duct cancer. As antibodies to MUC5AC, a mouse antibody (Non-Patent Literature 1) prepared using, as an antigen, a pancreatic cancer mucin fraction purified from xenograft of human pancreatic cancer cell line SW1990, and chimeric antibodies (Patent Literatures 1, 2, Non-Patent Literatures 2, 3) and humanized antibody (Patent Literatures 3, 4) produced based thereon have been reported.

An antibody is used as a reagent for detecting a target molecule, a diagnostic agent, or a pharmaceutical product for treating a disease by utilizing the specificity of the antibody for a target molecule. To further improve the detection performance and therapeutic effect, studies on antibodies bound to radionuclides and drugs are underway. Non-Patent Literature 1 has reported radioimmunotherapy of pancreatic carcinoma model mice using mouse antibody labeled with $^{131}$I which is a β-ray emitting nuclide. Non-Patent Literature 3 has reported SPECT imaging of pancreatic cancer patients using a chimeric antibody labeled with $^{111}$In which is a γ-ray emitting nuclide. Patent Literatures 3 and 4 describe particular MUC5AC-specific humanized antibodies and also describe that those labeled with $^{90}$Y, $^{111}$In, etc. are included. However, they only show the ADCC (antibody-dependent cellular cytotoxicity) activity of the MUC5AC-specific humanized antibody in the Examples.

[Citation List]

[Patent Literatures]

Patent Literature 1: JP-A-H7-203974
Patent Literature 2: JP-A-H11-5749
Patent Literature 3: WO 2013/157102
Patent Literature 4: WO 2013/157105

[Non-Patent Literatures]

Non-Patent Literature 1: Japanese Journal of Cancer Research, 87, 977-984, 1996
Non-Patent Literature 2: Japanese Journal of Clinical Medicine vol. 64 extra issue 1, 2006, p274-278
Non-Patent Literature 3: Japanese Journal of Cancer Research, 90, 1179-1186, 1999

[Summary of Invention]

The present invention aims to provide an anti-MUC5AC humanized antibody labeled with a radionuclide, that is superior in the specificity for mucin subtype 5AC (MUC5AC) and accumulation in tumor.

The present inventors have conducted intensive studies in view of the above-mentioned problems. As a result, they have succeeded in producing a conjugate of a nuclide that emits β-ray (β nuclide) and an anti-MUC5AC humanized antibody composed of a specific amino acid sequence, found that the conjugate is superior in specificity for MUC5AC

and accumulation in tumor, and confirmed the effects thereof, which resulted in the completion of the present invention.

According to the present invention, an anti-MUC5AC humanized antibody labeled with a nuclide that emits β-ray and superior in specificity for MUC5AC and accumulation in tumor, and use of the antibody can be provided.

[Brief Description of Drawings]

[Fig. 1]
Fig. 1 is a diagram showing the results of in vitro ARG using a section of a tumor highly expressing MUC5AC. For each condition, a filter paper was prepared on which 5 μL of a solution of each β-nuclide-labeled antibody diluted with phosphate buffered saline containing 1% (w/v) bovine serum albumin to 100-fold concentration as much as that used for administration to mouse was dropped on the entire surface. An image normalized using the average intensity of the ROI set over the entire filter paper as the upper limit of contrast (upper limit value is about 15000 to 20000) is displayed.

[Fig. 2]
Fig. 2 is a graph showing the binding ratio of each β-nuclide-labeled antibody to a MUC5AC high expression tumor section relative to a MUC5AC low expression tumor section. Error bars are standard deviations, n=3, the test was performed using the Tukey method.

[Fig. 3]
Fig. 3 is a graph showing confirmation results of the time-course changes in the tumor volume in tumor-bearing mice after administration of each β nuclide labeled antibody. Error bars are standard deviations, n=6 (in $^{131}$I-labeled antibody administration radioactivity 14.8 MBq group, autopsy was performed on two animals each on 13, 21, and 23 days after administration), the test was performed using the Tukey method.

[Fig. 4]
Fig. 4 is a graph showing the time-course changes in the body weight of tumor-bearing mice after administration of each β-nuclide-labeled antibody.

[Fig. 5]
Fig. 5 is a graph showing the examination results of thyroid toxicity in tumor-bearing mice after administration of each β-nuclide-labeled antibody. Plasma Thyroxine concentration was evaluated as thyroid toxicity. Error bars are standard deviations, n=6 (in $^{131}$I-labeled antibody administration radioactivity 7.4 MBq group and 14.8 MBq group, n=3, n=5, respectively), the test was performed using the Steel method.

[Fig. 6]
Fig. 6 shows the results of histopathological evaluation of tumor-bearing mice after administration of each β-nuclide-labeled antibody (typical hematoxylin and eosin staining image of femur). The black line at the bottom left of each Figure is a scale bar (200 μm).

[Fig. 7]
Fig. 7 shows the results of histopathological evaluation of tumor-bearing mice after administration of each β-nuclide-labeled antibody (typical hematoxylin and eosin staining image of thyroid gland). The black line at the bottom left of each Figure is a scale bar (200 μm).

[Fig. 8]
Fig. 8 is an image showing the results of PET-CT imaging 48 hr after administration of each $^{89}$Zr-labeled antibody.

[Fig. 9]
Fig. 9 shows graphs showing the results of VOI (volume of interest, three-dimensional ROI) analysis of the tumor (upper figure), heart (middle figure) and liver (lower figure) at each time point (12, 48, 84, 168 and 252 hr) in SPECT images of respective $^{89}$Zr-labeled antibodies prepared using $^{89}$Zr-labeled DOTA-Bn-DBCO (-■-) and $^{89}$Zr-labeled DOTAGA-DBCO (···●···).

[Fig. 10]
Fig. 10 is a graph showing the results of calculation of tumor-liver ratio from the accumulation at each time point (12, 48, 84, 168 and 252 hr) in the tumor and liver after administration of respective $^{89}$Zr-labeled antibodies prepared using $^{89}$Zr-labeled DOTA-Bn-DBCO (-●-) and $^{89}$Zr-labeled DOTAGA-DBCO (...8...).

[Fig. 11]
Fig. 11 is a diagram showing the results of SPECT-CT imaging using each $^{111}$In-labeled antibody. SPECT images of each $^{111}$In-labeled antibody are shown.

[Fig. 12]
Fig. 12 is a graph showing the results of VOI (volume of interest, three-dimensional ROI) analysis of the tumor and liver at each time point in SPECT images of each $^{111}$In-labeled antibody.

[Fig. 13]

Fig. 13 is a graph showing the results of confirmation of biodistribution and excretion pathway 168 hr after administration of respective $^{111}$In-labeled antibodies.

[Fig. 14]

Fig. 14 is an image showing the results of comparison of the binding ability of respective $^{111}$In-labeled antibodies by in vitro ARG.

[Fig. 15]

Fig. 15 shows graphs showing the results of the quantified and compared binding ability of respective $^{111}$In-labeled antibodies by in vitro ARG.

[Fig. 16]

Fig. 16 is a diagram showing the results of in vitro ARG using a section of a tumor (SW1990) highly expressing MUC5AC. A filter paper was prepared on which 5 μL of a solution of $^{177}$Lu-labeled anti-MUC5AC humanized antibody diluted with phosphate buffered saline containing 1% (w/v) bovine serum albumin to 100-fold concentration as much as used for administration to mouse was dropped on the entire surface. An image normalized using the average intensity of the ROI set over the entire filter paper as the upper limit of contrast (upper limit value is about 7000 to 10000) is displayed.

[Fig. 17]

Fig. 17 is a graph showing the binding ratio of $^{177}$Lu-labeled anti-MUC5AC humanized antibody to a MUC5AC high expression tumor section relative to a MUC5AC low expression tumor section. Error bars are standard deviations.

[Fig. 18]

Fig. 18 is a graph showing the results confirming the time-course changes in the tumor volume in tumor-bearing mice after administration of $^{177}$Lu-labeled antibody. Error bars are standard deviations, n=6 (in administration radioactivity 7.4 MBq group, n=5. One in administration radioactivity 16.7 MBq group was autopsied 19 days after administration), the test was performed using the Steel method.

[Fig. 19]

Fig. 19 is a graph showing the time-course changes in the body weight of tumor-bearing mice after administration of $^{177}$Lu-labeled antibody.

[Fig. 20]

Fig. 20 is a graph showing the examination results of thyroid toxicity in tumor-bearing mice after administration of $^{177}$Lu-labeled antibody. Plasma Thyroxine concentration was evaluated as thyroid toxicity. Error bars are standard deviations, n=6 (in administration radioactivity 7.4 MBq group, n=5) .

[Fig. 21]

Fig. 21 shows the results of histopathological evaluation of tumor-bearing mice after administration of $^{177}$Lu-labeled antibody (typical hematoxylin and eosin staining image of femur). The black line at the bottom left of each Figure is a scale bar (200 μm). The arrows in the Figure of Example 8-3 show the locations where bone marrow hematopoietic cells decreased.

[Fig. 22]

Fig. 22 shows the results of histopathological evaluation of tumor-bearing mice after administration of $^{177}$Lu-labeled antibody (typical hematoxylin and eosin staining image of thyroid gland). The black line at the bottom left of each Figure is a scale bar (200 μm).

[Description of Embodiments]

Unless otherwise specified, the terms used in the present specification can be used with the meanings generally used in the pertinent technique field.

(1) Conjugate

The present invention provides a conjugate of a radionuclide and an antibody, wherein the aforementioned radionuclide is a nuclide that emits β-rays, and the aforementioned antibody is a humanized antibody that specifically binds to MUC5AC (hereinafter to be also referred to as the conjugate of the present invention).

In another embodiment, the present invention provides a conjugate of a chelating agent chelated with a radionuclide (hereinafter to be also referred to as the chelating agent of the present invention) and an antibody, wherein the aforementioned radionuclide is a nuclide that emits β-rays and the aforementioned antibody is a humanized antibody that specifically binds to MUC5AC (hereinafter to be also referred to as the chelate conjugate of the present invention).

(1-1) Radionuclide

The radionuclide contained in the conjugate or chelate conjugate of the present invention is a nuclide that emits

β-ray. The nuclide only needs to be a nuclide that emits β-ray during the decay process of radionuclides. In detail, $^{67}$Cu (copper-67), $^{131}$I (iodine-131), $^{89}$Sr (strontium-89), $^{90}$Y (yttrium-90), or $^{177}$Lu (lutetium-177), and the like are preferably used, $^{177}$Lu, $^{131}$I or $^{90}$Y is more preferred, and $^{177}$Lu or $^{90}$Y is further preferred.

The nuclide that emits β-ray of the present invention can be produced or obtained by a known method. For example, $^{90}$Y, which is a daughter nuclide, can be generated by β-decaying $^{90}$Sr (strontium-90). $^{177}$Lu can be produced in a nuclear reactor using a neutron capture reaction of $^{176}$Lu(n,γ)$^{177}$Lu. $^{131}$I, which is a daughter nuclide, can be generated through β decay of $^{131}$Te produced through a neutron capture reaction of $^{130}$Te(n,γ)$^{131}$Te in a nuclear reactor. These β nuclides can also be obtained as commercially available products from Eckert & Ziegler, Thermo Fisher Scientific, Institute of Isotopes, POLATOM, and the like. The β nuclide produced or obtained in this way can be used for forming a conjugate with an antibody by chemically treating same as necessary into a chemical form suitable for binding with an antibody.

(1-2) antibody

The antibody to be contained in the conjugate or chelate conjugate of the present invention is a humanized antibody that specifically binds to MUC5AC (hereinafter also to be referred to as the humanized antibody used in the present invention). The antibody is a humanized antibody having the ability to specifically bind to MUC5AC, has stable physical properties, and is superior in tumor accumulation. The antibody may be used as an antigen-binding fragment thereof, and such embodiment is also encompassed in the present invention. Specifically, it contains a specific heavy chain variable region and a specific light chain variable region described below and, when desired, contains an appropriate heavy chain constant region and an appropriate light chain constant region. In the present specification, the "antigen-binding fragment" means an antibody fragment consisting of a part of the humanized antibody used in the present invention, and having the binding ability to MUC5AC. The number of amino acids contained in the polypeptide constituting the antigen-binding fragment is not particularly limited as long as it has the binding ability to MUC5AC.

Amino acid sequences preferred as the heavy chain variable region of the humanized antibody used in the present invention are shown below. The heavy chain variable region 1 (H01), heavy chain variable region 2 (H02), heavy chain variable region 3 (H03), and heavy chain variable region 4 (H04) respectively correspond to SEQ ID NOs: 1 to 4 in the Sequence Listing attached to the present specification. The underlined parts are CDR sites.

[heavy chain variable region (H01)]

E V Q L L E S G G G L V Q P G G S L R L S C A A S G F T F S N Y G M S W V R
Q A P G K G L E W V S T I S N S G R Y T Y F P D S V K G R F T I S R D N S K
N T L Y L Q M N S L R A E D T A L Y Y C T R H L D Y A N Y D A M D Y W G Q G
T L V T V S S

[heavy chain variable region (H02)]

L V Q L V E S G G G V V R P G G S L R L S C A A S G F T F S N Y G M S W I R
Q A P G K G L E W V S T I S N S G R Y T Y F P D S V K G R F T I S R D N A K
N S L Y L Q M N S L R A E D T A L Y Y C T R H L D Y A N Y D A M D Y W G Q G
T L V T V S S

[heavy chain variable region (H03)]

L V Q L V E S G G G V V Q P G R S L R L S C A A S G F T F S N Y G M S W V R
Q A P G K G L E W V A T I S N S G R Y T Y F P D S V K G R F T I S R D N S K
N T L Y L Q M N S L R A E D T A V Y Y C T R H L D Y A N Y D A M D Y W G Q G
T L V T V S S

[heavy chain variable region (H04)]

E V Q L L E S G G G L V Q P G G S L R L S C A V S G F T F S N Y G M S W V R Q A P G K G L E W V S T I S N S G R Y T Y F P D S V K G R F T I S R D N S R N T L Y L Q M N T L R A E D T A V Y Y C T R H L D Y A N Y D A M D Y W G Q G T P V T V S S

Amino acid sequences preferred as the light chain variable region of the humanized antibody used in the present invention are shown below. The light chain variable region 1 (L01), light chain variable region 2 (L02), light chain variable region 3 (L03), and light chain variable region 4 (L04) respectively correspond to SEQ ID NOs: 5 to 8 in the Sequence Listing attached to the present specification. The underlined parts are CDR sites.

[light chain variable region (L01)]

D I V M T Q S P S S L S A S V G D R V T I T C R A S K S V T T S D F S Y M H W Y Q Q K P G K A P K L L I Y L A S N L E S G V P S R F S G S G S G T D F T L T I S S L Q P E D F A T Y Y C Q H S R E F P W T F G G G T K V E I K

[light chain variable region (L02)]

D V V M T Q S P S T L S A S V G D R V T I T C R A S K S V T T S D F S Y M H W Y Q Q K P G Q A P K L L I Y L A S N L E S G V P S R F S G S G S G T D F T L T I S S L Q P E D F A T Y Y C Q H S R E F P W T F G Q G T K L E I K

[light chain variable region (L03)]

D I Q M T Q S P S S L S A S V G D R V T I T C R A S K S V T T S D F S Y M H W Y Q Q K P G K S P K L L I Y L A S N L E S G V P S R F S G S G S G T D F S L T I S S L Q P E D F A T Y Y C Q H S R E F P W T F G G G T K V E I K

[light chain variable region (L04)]

D I V M T Q S P D S L A V S L G E R A T I N C K A S K S V T T S D F S Y L H W Y Q Q K P G Q P P K L L I Y L A S N L E S G V P D R F S G S G S G T D F T L T I S S L Q A E D V A V Y Y C Q H S R E F P W T F G G G T K L E I K

In other words, the heavy chain variable region of the humanized antibody preferred in the present invention consists of the amino acid sequence shown in any one of SEQ ID NO: 1 to SEQ ID NO: 4, and the light chain variable region consists of the amino acid sequence shown in any one of SEQ ID NO: 5 to SEQ ID NO: 8. That is, the humanized antibody used in the present invention consists of a combination of any one of the above-mentioned four heavy chain variable regions (H01 - H04) and any one of the four light chain variable regions (L01 - L04).

A preferred humanized antibody in the present invention has heavy chain variable region H01, H03, or H04, and any one of L01 - L04 as the light chain variable region.

The most preferred humanized antibody in the present invention has heavy chain variable region H01 and light chain variable region L03.

The heavy chain variable region of the humanized antibody in the present invention is not limited to those defined by the amino acid sequence shown in SEQ ID NO: 1 to SEQ ID NO: 4 and also includes variants maintaining functions. That is, a mutated heavy chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 1 to SEQ ID NO: 4 is also used as the heavy chain variable region of the humanized antibody to be used in the present invention as long as it can bind to MUC5AC when combined with the light chain variable region in the present invention.

In the present specification, the identity of the amino acid sequence refers to the identity of the amino acid sequences between the two proteins of interest, and is shown by the percentage (%) of amino acid residues that match

in the optimal alignment of the amino acid sequences prepared using mathematical algorithms known in the pertinent technical field. The identity of an amino acid sequence can be determined by visual inspection and mathematical calculation, and can be calculated using a homology search program (e.g., BLAST, FASTA) or sequence alignment program (e.g., ClustalW) known to those skilled in the art, or genetic information processing software (e.g., GENETYX [registered trademark]), and the like. To be specific, the identity of the amino acid sequence in the present specification can be determined using systematic analysis program ClustalW (http://clustalw.ddbj.nig.ac.jp/index.php?lang=ja) published on the website of DDBJ (DNA DataBank of Japan) by the initial setting conditions (Version2.1, Alignment type: slow, DNA Weight Matrix: Gonnet, GAP OPEN: 10, GAP EXTENSION: 0.1).

In addition, as the heavy chain variable region of the humanized antibody to be used in the present invention, a mutated heavy chain variable region consisting of the amino acid sequence shown in SEQ ID NO: 1 to SEQ ID NO: 4, wherein not more than 10, preferably not more than 8, further preferably not more than 5, most preferably not more than 3 (3, 2 or 1), amino acids are deleted, substituted, or added, is also used as the heavy chain variable region of the humanized antibody to be used in the present invention as long as it can bind to MUC5AC when combined with the light chain variable region in the present invention.

The light chain variable region of the humanized antibody to be used in the present invention is not limited to the amino acid sequence shown in SEQ ID NO: 5 to SEQ ID NO: 8 and also includes variants maintaining functions. That is, a mutated light chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 5 to SEQ ID NO: 8 is also used as the light chain variable region of the humanized antibody to be used in the present invention as long as it can bind to MUC5AC when combined with the heavy chain variable region in the present invention.

In addition, as the light chain variable region of the humanized antibody to be used in the present invention, a mutated light chain variable region consisting of the amino acid sequence shown in SEQ ID NO: 5 to SEQ ID NO: 8, wherein not more than 10, preferably not more than 8, further preferably not more than 5, most preferably not more than 3 (3, 2 or 1), amino acids are deleted, substituted, or added, is also encompassed in the light chain variable region of the humanized antibody to be used in the present invention as long as it can bind to MUC5AC when combined with the heavy chain variable region in the present invention.

The humanized antibody to be used in the present invention can be produced by a method generally performed in the art or a method analogous thereto. Specifically, the following steps can be performed.

Since the amino acid sequences of the heavy chain variable region and the light chain variable region of the humanized antibody to be used in the present invention are disclosed in SEQ ID NO: 1 to SEQ ID NO: 8, a nucleic acid encoding the antibody obtained based on the amino acid sequence information is constructed and inserted into a suitable expression vector. The expression vector can optionally contain, in addition to the nucleic acid encoding the humanized antibody to be used in the present invention, Kozak sequence to improve translation efficiency, a signal sequence that promotes secretion of the humanized antibody to be used in the present invention into the medium when introduced into a host, a promoter sequence, and the like. The vector that can be used in the present invention can be selected from those generally used in the pertinent technical field, and plasmid vector pcDNA3.4 is preferred. Introduction of an expression vector into the host cell is not particularly limited. As a method for introducing a gene into a cell, a method conventionally used in the pertinent technical field, for example, a method known to those skilled in the art such as calcium phosphate method, electroporation method, lipofection method, and DEAE-dextran method can be used. An introduction method using the lipofection method is particularly preferred, as performed in the following Example. As the host cell used for this purpose, those conventionally used in the pertinent technical field can be used. Examples of such host cell include CHO cell, 293 cell, Escherichia coli, Pichia yeast, Sf9 cell and the like. Currently, an expression system kit for expressing the protein of interest is also commercially available. The ExpiCHO System (manufactured by Thermo Fisher Scientific) used in the following Example is particularly preferred for rapid and reliable expression of the protein of interest.

The humanized antibody to be used in the present invention can be obtained by inserting a nucleic acid encoding the humanized antibody to be used in the present invention into an expression vector, introducing the nucleic acid into a host cell by the expression vector containing the nucleic acid, culturing the host cell after introduction of the nucleic acid, and obtaining the humanized antibody of the present invention from the culture supernatant thereof by a purification means such as chromatography and the like. In this method, the humanized antibody to be used in the present invention is secreted in a culture supernatant by culturing the host cell. The humanized antibody or an antigen-binding fragment thereof to be used in the present invention can be obtained from the culture supernatant by using a purification means such as chromatography, and the like. As the means for chromatography, various means known in the pertinent technical field such as affinity chromatography, ion exchange chromatography, size-exclusion chromatography and the like can be used. Affinity chromatography with the protein A column used in the following Example is particularly preferred.

The above-mentioned humanized antibody may be a polyclonal antibody or a monoclonal antibody.

(1-3) Chelating agent

In the present invention, the chelating agent is not particularly limited as long as it has a site in the structure thereof where radionuclide is coordinated. Preferably, it has a chelate site, which is a site to which a radionuclide is coordinated, and a substituent that enables conjugating with an antibody. Examples of the chelate site include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-$\alpha,\alpha',\alpha'',\alpha'''$-tetramethyl-1,4,7,10-tetraazacyclododecane-1,4,7,10-tetraacetic acid), DOTAM (1,4,7,10-tetrakis (carbamoylmethyl)-1,4,7,10-tetraazacyclododecane), DOTA-GA ($\alpha$-(2-Carboxyethyl)-1,4,7,10-tetraazacyclododecane-1,4,7,10-tetraacetic acid), DOTA-GA-NHS, DOTP (((1,4,7,10-Tetraazacyclododecane-1,4,7,10-tetrayl)tetrakis(methylene))tetraphosphonic acid), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane($L^{py}$), p-SCN-Bn-DOTA (S-2-(4-Isothiocyanatobenzyl)-1,4,7,10-tetraazacyclododecane tetraacetic acid), MeO-DOTA-NCS (1-[(2-methoxy-5-isothiocyanato phenyl)-carboxymethyl]-4,7,10-triscarboxy 5 methyl-1,4,7,10-tetraazacyclododecane), EuK-106, DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), DTPA-BMA (5,8-Bis(carboxymethyl)-11-[2-(methylamino)-2-oxoethyl]-3-oxo-2,5,8,11-tetraazatridecan-13-oic acid), EDTA (2,2',2'',2'''-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid), TETPA (1,4,8,11-tetraazacyclotetradecane-1,4,8,11-tetrapropionic acid), TETA (1,4,8,11-Tetraazacyclotetradecane-N,N',N'',N'''-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N'',N'''-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N'',N''',N''''-penta-acetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N''-bis(6-carboxy-2-pyridylmethyl)-diethylenetriamine-N,N',N''-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), porphyrin, DO3A (1,4,7,10-Tetraazacyclododecane-1,4,7-triacetic acid trisodium salt), and DO3A-NHS (1,4,7,10-Tetraazacyclododecane-1,4,7,10-tetraacetic acid mono-N-hydroxysuccinimide ester). It is preferable to have a structure derived from a compound represented by the following formula (A).

(A)

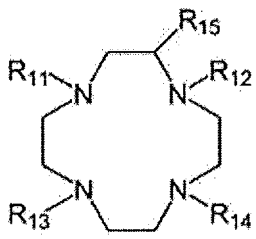

In the formula (A), $R_{11}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the aforementioned antibody, p is an integer of not less than 0 and not more than 3, $R_{15}$ is a hydrogen atom when $R_{12}$ is a substituent for conjugating with the aforementioned antibody, and $R_{15}$ is a substituent for conjugating with the aforementioned antibody when $R_{12}$ is not a substituent for conjugating with the aforementioned antibody.

Examples of the specific structure represented by the formula (A) include structures derived from the compounds represented by the following formulas (A-1) to (A-12).

(A-1)

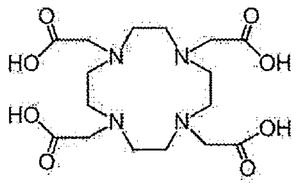

DOTA

(A-2)

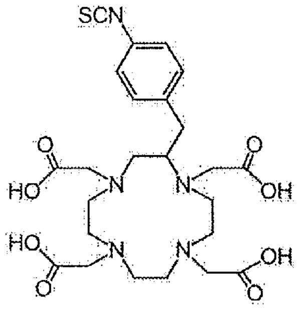

p-SCN-Bn-DOTA

(A-3)

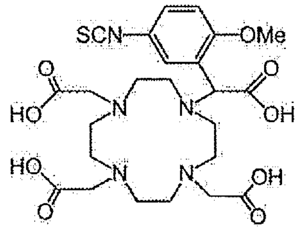

MeO-DOTA-NCS

(A-4)

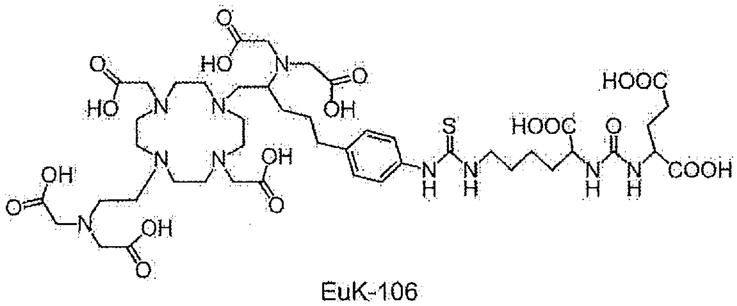

EuK-106

(A-5)

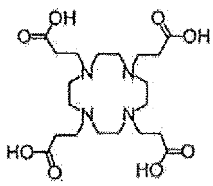

DOTPA

(A-6)

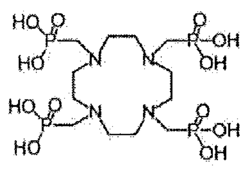

DOTMP

(A-7)

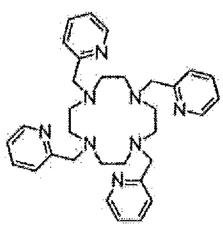

$L^{py}$

(A-8)

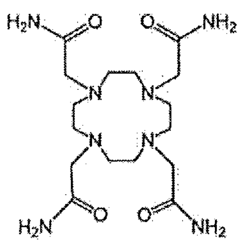

DOTAM

(A-9)

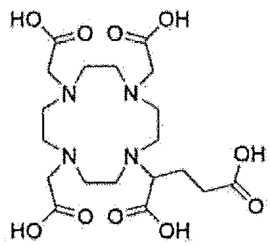

DOTA-GA

(A-10)

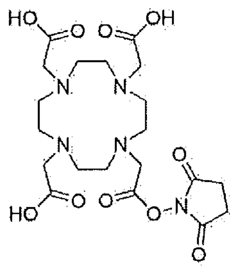

DO3A-NHS

(A-11)

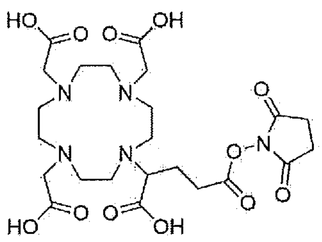

DOTA-GA-NHS

(A-12)

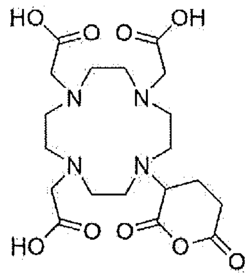

DOTA-GA-anhydride

The linkage site between the chelate site and the substituent that enables formation of a conjugate with the antibody is preferably an amide bond or a thiourea bond, more preferably an amide bond from the aspect of stability.

The amide bond can be formed, for example, by the reaction of an N-hydroxysuccinimide ester (NHS) group of the above-mentioned formula (A-10) or (A-11), or a 2,6-dioxo tetrahydro-2H-pyranyl group of the above-mentioned (A-12) with primary amine. The thiourea bond can be formed by the reaction of an isothiocyanate group of the compound of the above-mentioned formula (A-2), (A-3) with primary amine or a maleimide group.

In the chelate conjugate of the present invention, the chelating agent may be provided at least not less than one molecule, preferably not less than 1 molecule and not more than 8 molecules, per one molecule of the antibody. To maintain the activity of the antibody itself (antigen recognition action, neutralizing action, complement activating action and/or opsonin action), a chelating agent is preferably introduced site-specifically into the Fc region (constant region) of the antibody. In the present invention, the chelating agent is preferably provided at one or two molecules per one molecule of the antibody.

In the chelate conjugate of the present invention, the chelating agent may be connected to the antibody via a linker. Examples of the linker include substituted or unsubstituted alkyl group, substituted or unsubstituted heteroalkyl group, polyethylene glycol (PEG) group, peptides, sugar chain, disulfide group, combination of these and the like.

Preferably, the chelating agent modifies the antibody site-specifically, more preferably in the Fc region, via a linker. In this case, the linker contains a peptide consisting of not less than 13 and not more than 17 amino acid residues represented by the following formula (i) (hereinafter, to be also referred to as "antibody-modification peptide"), and one formed by a cross-linking reaction between the antibody-modification peptide modified with a crosslinking agent and the antibody can be used. In the explanation of the formula (i), the left side of the paper surface of the amino acid sequence indicates the N-terminal side, and the right side of the paper surface of the amino acid sequence indicates the C-terminal side. When the chelating agent is connected to the antibody via the antibody-modification peptide as a linker, the position where the chelating agent and the antibody-modification peptide are linked is not particularly limited. For example, it can be directly or indirectly linked at the N-terminal or C-terminal of the antibody-modification peptide, preferably at the N-terminal. In addition, the C-terminal of the antibody-modification peptide may be modified by, for example, amidation or the like to improve its stability and the like.

(Xa)-Xaa1-(Xb)-Xaa2-(Xc)-Xaa3-(Xd)··· (i)

In the formula (i), Xa, Xb, Xc and Xd are each continuous X in the number of a, continuous X in the number of b,

continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa1 and Xaa3 are linked, and
Xaa2 is a lysine residue, arginine residue, cysteine residue, aspartic acid residue, glutamic acid residue, 2-aminosuberic acid, or diamino propionic acid, and modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue

consisting of the same type of amino acid, or different types of amino acids.

In the formula (i), a, b, c and d are not particularly limited as long as they are numbers within the aforementioned range. From the aspect of the stability of binding between the peptide and antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

Xaa1 and Xaa3 are amino acid residues derived from an amino acid having a thiol group in the side chain, and Xaa1 and Xaa3 may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the wavy line indicates the binding part with the sulfide group.

(4) 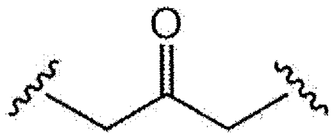

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Among these, the amino acid sequence of the antibody-modification peptide preferably has any one of the following sequences (1) to (14), more preferably the following sequence (1), (2), (13) or (14). In the following amino acid sequences (1) to (14), (Xaa2) is a lysine residue, a cysteine residue, an aspartic acid residue, a glutamic acid residue, a 2-aminosuberic acid, or a diamino propionic acid, and (Xaa1) and (Xaa3) are each a homocysteine residue. In the following amino acid sequences (1) to (14), the amino acids other than (Xaa1), (Xaa2) and (Xaa3) are indicated by one-letter abbreviations.

(1) DCAYH(Xaa2)GELVWCT (SEQ ID NO: 9)
(2) GPDCAYH(Xaa2)GELVWCTFH (SEQ ID NO: 10)
(3) RCAYH(Xaa2)GELVWCS (SEQ ID NO: 11)
(4) GPRCAYH(Xaa2)GELVWCSFH (SEQ ID NO: 12)
(5) SPDCAYH(Xaa2)GELVWCTFH (SEQ ID NO: 13)
(6) GDDCAYH(Xaa2)GELVWCTFH (SEQ ID NO: 14)
(7) GPSCAYH(Xaa2)GELVWCTFH (SEQ ID NO: 15)
(8) GPDCAYH(Xaa2)GELVWCSFH (SEQ ID NO: 16)
(9) GPDCAYH(Xaa2)GELVWCTHH (SEQ ID NO: 17)
(10) GPDCAYH(Xaa2)GELVWCTFY (SEQ ID NO: 18)
(11) SPDCAYH(Xaa2)GELVWCTFY (SEQ ID NO: 19)
(12) SDDCAYH(Xaa2)GELVWCTFY (SEQ ID NO: 20)
(13) RGNCAYH(Xaa2)GQLVWCTYH (SEQ ID NO: 21)
(14) G(Xaa1)DCAYH(Xaa2)GELVWCT(Xaa3)H (SEQ ID NO: 22)

(1-4A) Production method of conjugate

The conjugate of the present invention can be produced by forming a conjugate of a radionuclide and an antibody. The radionuclide used here is preferably used in a manner permitting ionization, more preferably in the form of an ion, from the viewpoint of increasing the conjugate formation efficiency. The order of addition of the radionuclide and the antibody does not matter as long as a conjugation of a radionuclide and an antibody can be formed. For example, a solution in which radionuclide ions are dissolved in a solvent mainly composed of water can be used as a radionuclide.

After conjugate formation, the obtained conjugate may be purified using a filtration filter, a membrane filter, a column filled with various fillers, chromatography or the like. The thus-obtained conjugate is a conjugate in which one

molecule of radionuclide or two or more molecules of radionuclide are modified at random positions for one molecule of antibody.

(1-4B) Production method of chelate conjugate

The production method of the chelate conjugate of the present invention includes two steps which are a conjugation step of conjugating a chelating agent and an antibody, and a complex formation step of forming a complex of a radionuclide and a chelating agent. The conjugation step may be performed before the complex formation step or after the complex formation step.

In the conjugation step, various methods for chemical modification of antibody are used. Specifically, the methods (a) - (f) can be mentioned:

(a) amine coupling method (a method for modifying the amino group of a lysine residue of an antibody by using a chelating agent or chelate having a carboxyl group activated by an N-hydroxysuccimidyl (NHS) group)
(b) method for modifying a sulfhydryl (SH) group generated by partially reducing a disulfide bond (SS bond) between polypeptide chains at the hinge site of an antibody with a chelating agent or linker having a maleimide group reactive with the SH group
(c) method for modifying cysteine newly introduced into an antibody by an amino acid mutation by genetic engineering with a chelating agent or linker having a maleimide group
(d) method for modifying an azide group of azidized lysine newly introduced into an antibody by an amino acid mutation by genetic engineering with a chelating agent or linker having alkyne (e.g., Dibenzylcyclooctyne: DBCO) by using a click reaction
(e) method for modifying glutamine introduced into a specific position of an antibody with a chelating agent or linker having a side chain of lysine by using transglutaminase
(f) method of site-specifically modifying the Fc region of an antibody with a chelating agent or linker having the antibody-modification peptide shown in the aforementioned (i).

In the complex formation step, the chelating agent is chelated with a radionuclide (complex formation). The radionuclide used here is preferably used in a manner permitting ionization, more preferably in the form of an ion, from the viewpoint of increasing the complex formation efficiency. In the complex forming step, the order of addition of the radionuclide to the chelating agent does not matter as long as a complex can be formed with the radionuclide. For example, a solution in which radionuclide ion is dissolved in a solvent mainly composed of water can be used as a radionuclide.

After complex formation, the obtained complex may be purified using a filtration filter, a membrane filter, a column filled with various fillers, chromatography or the like.

In the production method of the chelate conjugate of the present invention, a conjugation step is preferably performed after the complex formation step.

In a more preferred embodiment, in complex formation step (A), a complex is formed between a radionuclide and a chelating agent having a first atomic group capable of click reaction as a substituent for enabling conjugation with the antibody. Then, in conjugation step (B), using an antibody-modification peptide shown by the aforementioned (i) and an antibody-modification linker having a second atomic group capable of click reaction, a click reaction is performed between the peptide-modified antibody in which Fc region is site-specifically modified and the chelating agent with a formed complex which is obtained in step (A) to obtain the chelate conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker is preferably alkyne and the antibody-modification linker is preferably azide, or the chelate linker is preferably 1,2,4,5-tetrazine and the antibody-modification linker is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or a combination of an atomic group containing 1,2,4,5-tetrazine as the first atomic group (the formula (1b)) and

an atomic group containing trans-cyclooctene (TCO) as alkene of the second atomic group (the formula (2b)). Preferred is the combination of the formula (1a) and the formula (2a).

(1a) 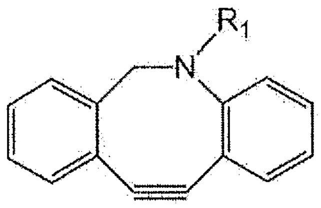

Dibenzylcyclooctyne

(2a) 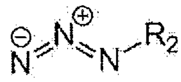

Azide

wherein $R_1$ is a linkage site with a chelating agent, and $R_2$ is a linkage site with an antibody-modification peptide in the antibody.

(1b) 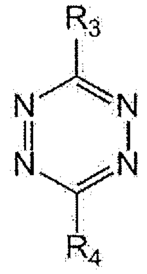

1,2,4,5-tetrazine

(2b) 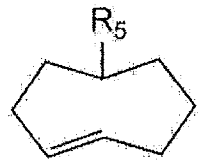

*trans*-cyclooctene

wherein one of $R_3$ and $R_4$ is a linkage site with a chelating agent or an antibody-modification peptide in the antibody, and the other is a hydrogen atom, a methyl group, a phenyl group or a pyridyl group, and $R_5$ is a linkage site with any one chelating agent or an antibody-modification peptide in the antibody depending on $R_3$ or $R_4$.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In step (A), more preferably, a chelating agent having a structure represented by the following formula (ii) is used.

A-B-C ··· (ii)

In the formula (ii), A is a chelate site represented by the following formula (iia).

(iia) 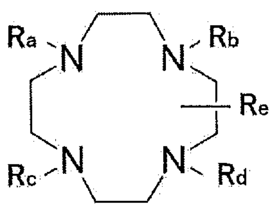

In the formula (iia), Ra, Rb, and Rc are each independently a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, p is an integer of not less than 0 and not more than 3, either Rd or Re is a binding site (*) to B, and the other is a hydrogen atom or a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, and p is an integer of not less than 0 and not more than 3.

In the formula (ii), B is represented by the following formula (iib):

(iib) 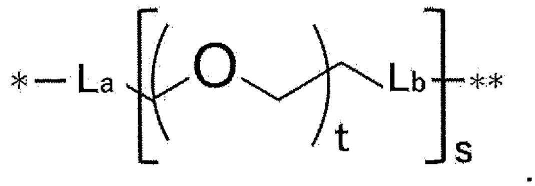

In the formula (iib), La and Lb are each independently a bond linker containing at least an amide bond or thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

(iic) 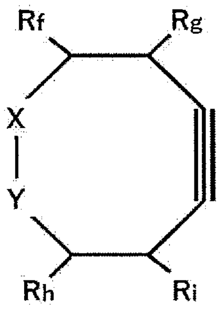 (iid) 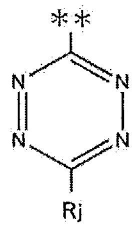

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (iia) wherein Ra to Rd are $-(CH_2)_pCOOH$, p is 1, Re is a binding site with B; or DO3A derivative or DOTAGA derivative wherein Ra to Rc are - $(CH_2)_pCOOH$, p is 1, Rd is a binding site with B, and Re is a hydrogen atom is more preferred.

In the formula (ii), a DOTA-PEGt-DBCO derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is an alkyne derivative represented by the formula (iic), wherein, in the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B; or a DOTA-PEGt-Tz derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is a tetrazine derivative represented by the formula (iid) is further preferred.

In the formula (ii), a DOSA-PEGt-DBCO derivative wherein A is the above-mentioned DO3A derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the formula (ii), a DOTAGA-PEGt-DBCO derivative wherein A is the above-mentioned DOTAGA derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf

and Rg are jointed to form a benzene ring, Rh and Ri are jointed to form a benzene ring, and ** is a binding site with B is further preferred.

In the molar ratio of the chelating agent and radionuclide, as chelate site/radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

In the reaction time, provided that the reaction temperature is as described above, lower limit is preferably not less than 5 minutes, more preferably not less than 10 minutes, further preferably not less than 20 minutes, further more preferably not less than 30 minutes, particularly preferably not less than 45 minutes, and the upper limit is preferably not more than 180 minutes, more preferably not more than 150 minutes, further preferably not more than 120 minutes, further more preferably not more than 90 minutes, and especially not more than 60 minutes, for example, and a range of not less than 10 minutes and not more than 150 minutes is preferred, and not less than 10 minutes and not more than 60 minutes is more preferred.

The antibody to be used in step (B) is a peptide-modified antibody in which Fc region (constant region) of humanized antibody as described in detail in the above-mentioned "(1-2) Antibody" is site-specifically modified using the antibody-modification peptide shown in the aforementioned (i), and an antibody-modification linker having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker may be one in which an antibody-modification peptide and a linker represented by the following formula (S1) are bonded.

$$*\text{-}((L_1)_m\text{-}Z)_k\text{-}L_2\text{-}AG_2 \ldots \quad (S1)$$

wherein * is a binding site with the N-terminal or C-terminal of peptide,

$L_1$ is a linker moiety of polyethylene glycol (PEG),
m is an integer of not less than 1 and not more than 50,
Z is a second linker moiety that binds $(L_1)_m$ and $L_2$,
k is 0 or 1,

$L_2$ is the second PEG linker moiety, and
$AG_2$ is a second atomic group.

In the aforementioned formula (S1), the structure of Z is not particularly limited as long as it is a linker structure that binds $(L_1)_m$ and $L_2$ to each other, and includes, for example, an amino acid sequence consisting of not less than 1 and not more than 5 amino acid residues. In this case, the amino acid sequence contained in Z preferably contains a cysteine residue, and is more preferably bonded to $L_2$ via a thioether group formed by the bond between the thiol group of the cysteine residue and a maleimide group.

In the present invention, the PEG linker moiety constituting $L_2$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

(P2) 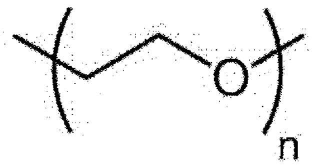

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker, an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an antibody to obtain a peptide-modified antibody can be performed, for example, using a crosslinking agent. A crosslinking agent is a chemical substance for linking an antibody-modification peptide and an antibody by a covalent bond. Examples thereof include a crosslinking agent preferably containing two or more succinimidyl groups such as disuccinimidyl glutarate (DSG), disuccinimidyl suberate (DSS) and the like, a crosslinking agent consisting of a compound containing two or more imidic acid moieties such as dimethyl adipimidate and the like, or a salt thereof, a crosslinking agent consisting of a compound having a disulfide bond such as dimethyl 3,3'-dithiobispropionimidate, dithiobissuccinimidylpropionic acid, and the like, or a salt thereof, and the like. Using such crosslinking agent, a crosslinking reaction can be caused between an amino acid residue of Xaa2 in the antibody-modification peptide and an antibody. When, for example, the humanized antibody of the present invention is used as the antibody, the crosslinking reaction in the antibody occurs site-specifically between an amino acid residue of Xaa2 and a Lys252 residue according to the Eu numbering in the humanized antibody of the present invention. These Lys residues are present in the Fc region of the humanized antibody of the present invention.

The method for binding the antibody-modification peptide to the antibody can be performed, for example, by dispersing the aforementioned antibody-modification peptide, an antibody, a crosslinking agent, and a catalyst as necessary in an appropriate buffer at not less than 10°C and not more than 30°C. The reaction time may be about not less than 10 min to 2 hr or less. The molar ratio at the time of reaction of the peptide and the antibody is preferably not less than 1/5, more preferably not less than 1/3, further preferably not less than 1/1.5 as the lower limit of the antibody/peptide, and the upper limit is preferably not more than 20/1, more preferably not more than 10/1, further preferably not more than 5/1, further more preferably not more than 1/1, particularly preferably not more than 1/1.7. For example, the range

of not less than 1/5 and not more than 20/1 is preferable, and not less than 1/1.5 and not more than 1/1.7 is more preferable.

The peptide-modified antibody obtained through the above steps is a mixture containing an antibody in which one molecule of antibody-modification peptide is bound to one molecule of an antibody (hereinafter to be referred to as "monovalent antibody") and an antibody in which two molecules of antibody-modification peptide are bound to one molecule of an antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is preferable to use a column packed with various fillers, and it is more preferable to use a column packed with a filler suitable for separation and purification of proteins such as antibody and the like.

The filler suitable for separation and purification of protein such as antibody and the like is not particularly limited as long as it is a filler in which an immunoglobulin-binding protein is immobilized on a carrier composed of a water-insoluble substrate, and which specifically binds to an antibody. Examples of the immunoglobulin-binding protein include protein A, protein G, protein L and the like. These immunoglobulin-binding proteins may be genetically engineered recombinants. Examples of the recombinant immunoglobulin-binding protein include genetically-engineered protein A, genetically-engineered protein G, and fused protein A domain and protein G domain. In the present invention, as a filler suitable for separation and purification of at least a monovalent antibody and a divalent antibody, protein A is more preferred, and genetically-engineered protein A is more preferred. As used herein, protein A and protein G are protein molecules that can specifically bind to an antibody molecule IgG, and classified as protein A (Staphylococcus aureus) or protein G (streptococcus: Streptococcus genus) depending on the difference in the origin of the isolated microorganisms. The genetically-engineered protein A is a protein A in which at least one amino acid mutation has been introduced into an amino acid residue of any of the IgG binding domains (E, D, A, B and C domains) of protein A. In the present invention, genetically-engineered protein A in which the domain into which at least one amino acid mutation has been introduced is multimerized is preferable, genetically-engineered protein A in which A, B or C domain into which at least one amino acid mutation of protein A has been introduced is multimerized is more preferable, and genetically-engineered protein A multimerized to not less than a dimer and not more than a pentamer is further more preferable. The amino acid mutation may be derived from any mutation such as substitution, deletion, insertion and the like of the amino acid sequence or the base sequence encoding the amino acid in the transcriptional translation step of the gene. Examples thereof that are not particularly limited include the genetically-engineered protein A described in WO 2003/080655, WO 2011/118699 and the like.

Examples of the water-insoluble substrate on which immunoglobulin-binding proteins are immobilized include inorganic carriers such as glass beads, silica gel and the like, organic carriers such as synthetic polymers (e.g., crosslinked polyvinyl alcohol, crosslinked polyacrylate, crosslinked polyacrylamide, crosslinked polystyrene) and polysaccharides (e.g., crystalline cellulose, crosslinked cellulose, crosslinked agarose, crosslinked dextran), and organic-organic, organic-inorganic conjugate carriers and the like obtained from combinations of these, and the like.

The column filled with the aforementioned genetically-engineered protein A as a filler is commercially available as, for example, KanCap (registered trademark) series (KANEKA KanCapA prepacked column) of KANEKA CORPORATION, HiTrap (registered trademark) series (HiTrap Mabselect, HiTrap Mabselect SuRe, HiTrap Mabselect Xtra) of GE Healthcare, HiScreen series (HiScreen Mabselect SuRe) of GE Healthcare, TOYOPEARL (registered trademark) series (TOYOPEARL AF-rProtein A-650F) of Tosoh Corporation, and the like.

The separation and purification of peptide-modified antibody used for click reaction in step (B) is explained below as an example.

A peptide-modified antibody is subjected to a click reaction in step (B) after an antibody modification step in which a modified antibody is obtained by site-specifically modifying an Fc region of an antibody by a linker provided with an antibody-modification peptide (antibody-modification linker), and an antibody purification step in which the modified antibody is purified using the aforementioned carrier with an immunoglobulin-binding protein immobilized thereon. In addition, the antibody purification step further includes a retention step of retaining the modified antibody retained on the carrier, a washing step of washing the modified antibody not retained on the carrier, and an elution step of eluting the modified antibody retained on the carrier in the retention step.

More specifically, in the antibody modification step, a modified antibody is obtained as a mixture containing an unmodified antibody not modified by an antibody-modification linker, a monovalent antibody, and a divalent antibody and, in the antibody purification step, the first antibody composition containing relatively large amounts of the unmodified antibody and the monovalent antibody and the second antibody composition containing a relatively large amount of the divalent antibody are respectively eluted utilizing the difference in the interaction of the unmodified antibody, monovalent

antibody and divalent antibody with immunoglobulin-binding proteins. That is, in the retention step and washing step among the antibody purification steps, the second antibody composition containing a relatively large amount of peptide-modified antibody (divalent antibody) having a low degree of interaction with immunoglobulin-binding proteins is eluted and, in the elution step among the antibody purification steps, the first antibody composition containing a relatively large amount of peptide-modified antibody (unmodified antibody and monovalent antibody) having a high degree of interaction with immunoglobulin-binding proteins is eluted. As used herein, "containing a relatively large amount of unmodified antibody and monovalent antibody" means that the total amount of unmodified antibody and monovalent antibody contained in the first antibody composition is larger than that of the divalent antibody contained in the antibody composition, preferably that the total amount of the unmodified antibody and monovalent antibody is not less than 55%, not less than 63%, not less than 70%, not less than 80%, or not less than 90%, of the total amount (100%) of the unmodified antibody and modified antibody contained in the antibody composition. In addition, "containing a relatively large amount of divalent antibody" means that the amount of divalent antibody contained in the second antibody composition is larger than that of the monovalent antibody contained in the antibody composition, preferably that the amount of divalent antibody is not less than 55%, not less than 63%, not less than 70%, not less than 80%, or not less than 90%, of the total amount (100%) of the unmodified antibody and modified antibody contained in the antibody composition.

In the retention step, a solution containing the mixture of the unmodified antibody, monovalent antibody and divalent antibody obtained in the antibody modification step is added to a column, and unmodified antibody and monovalent antibody retained on the carrier are retained on the column and the divalent antibody not retained on the carrier is allowed to pass through. The solution that passed through in the retention step constitutes a part of the second antibody composition. To facilitate retention of the unmodified antibody and monovalent antibody on the column and to prevent aggregation or denaturation of these, it is preferable to dilute the mixed solution of the peptide-modified antibody with an appropriate dilution solvent and add same to the column. The dilution solvent is not particularly limited as long as the peptide-modified antibody dissolves and does not easily aggregate or denature in the solvent, and water, saline, buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. When a buffer is used as a dilution solvent, the concentration of the buffering agent is not less than 10 mmol/L, preferably not less than 15 mmol/L, more preferably not less than 20 mmol/L as the lower limit, and not more than 1000 mmol/L, preferably not more than 500 mmol/L, more preferably not more than 100 mmol/L as the upper limit. In addition, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the washing step, the modified antibody remaining in the column is eluted from the column by using a wash solvent. The solution that passed through the column in the aforementioned retention step and the solution eluted from the column in the washing step contain a relatively large amount of the divalent antibody, and therefore, these can be combined and used as the second antibody composition.

The wash solvent is not particularly limited as long as the peptide-modified antibody dissolves and does not easily aggregate or denature in the solvent, and it is a buffer having appropriate pH buffering capacity, and buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. The concentration of the buffering agent used as the wash solvent is not less than 20 mmol/L, preferably not less than 30 mmol/L as the lower limit, and not more than 200 mmol/L, preferably not more than 70 mmol/L as the upper limit. The pH of the wash solvent is not less than 4.0, preferably not less than 4.5, more preferably not less than 4.8 as the lower limit, and not more than 7.4, preferably not more than 6.0, more preferably not more than 5.2 as the upper limit. Furthermore, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the elution step, the modified antibody retained on the carrier is eluted from the column by using an elution solvent. That is, the first antibody composition containing a relatively large amount of unmodified antibody and monovalent antibody is eluted from the column by using an elution solvent.

As the elution solvent, a buffer such as sodium acetate buffer, ammonium acetate buffer, citrate buffer and the like can be used. In addition, to reduce non-specific binding to the antibody-modification linker, unmodified antibody and modified antibody column carrier, the elution solvent may contain an additive such as sodium chloride, potassium chloride and the like. The concentration of the additive contained in the elution solvent is not particularly limited and may be, for example, 0.15 mol/L.

When the elution solvent contains a buffering agent, the concentration of the buffering agent is not less than

20 mmol/L, preferably not less than 30 mmol/L as the lower limit, and not more than 200 mmol/L, preferably not more than 70 mmol/L as the upper limit. In addition, to weaken the interaction between the unmodified antibody and monovalent antibody, and the immunoglobulin-binding protein, and to prevent denaturation and aggregation of the antibody, the pH of the elution solvent is not less than pH 3.0 as the lower limit, and not more than pH 4.2 as the upper limit.

The first antibody composition or the second antibody composition obtained in the antibody purification step may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the conjugate obtained in step (A) are capable of click reaction, the order of addition of these does not matter. For example, one of the conjugate and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the conjugate and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferred, and the range of not less than 0.1 mL and not more than 10 mL is more preferred.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferred, and the range of not less than 1 μmol/L and not more than 100 μmol/L is more preferred, from the aspect of the yield of the desired chelate conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferred, and the range of not less than 10 min and not more than 20 hr is more preferred.

The obtained chelate conjugate may be used as it is or purified using a filtration filter, a membrane filter, a column filled with various fillers, chromatography or the like.

In the chelate conjugate produced by steps (A) and (B), a specific site of a humanized antibody that specifically binds to MUC5AC (e.g., the lysine residue in the Fc region of antibody) is specifically modified with a chelating agent. This chelate conjugate comprises one or two molecules of the aforementioned chelating agent per one molecule of the antibody. The chelating agent site-specifically modifies the Fc region of the antibody of the present invention via a linker. The linker is constituted of a chelate linker that connects to a chelating agent, a first atomic group that connects to the linker, a second atomic group that can perform click reaction with the first atomic group, and an antibody-modification linker that connects to the second atomic group (including an antibody-modification peptide represented by the above-mentioned formula (i)). Therefore, the linker has a chemical structure derived from the first atomic group and the second atomic group. As such chemical structure, a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

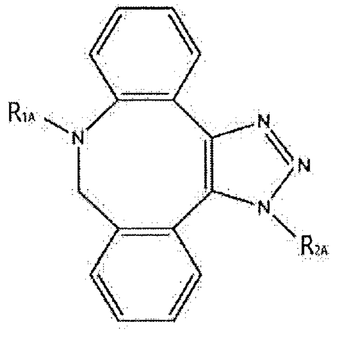

(10a)

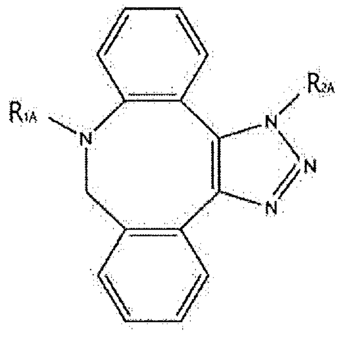

(10b)

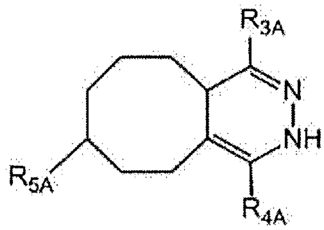

(10c)

In the formula (10a) and the formula (10b), $R_{1A}$ is a binding site with a chelate linker, and $R_{2A}$ is a binding site with an antibody-modification linker. In the formula (10c), one of $R_{3A}$ and $R_{4A}$ is a hydrogen atom, a methyl group, a phenyl group or a pyridyl group, and the other is a binding site with a chelate linker, and $R_{5A}$ is a binding site with an antibody-modification linker.

(1-5) Radiopharmaceutical

The conjugates produced by the method of the aforementioned (1-4A) and the chelate conjugates produced by the method of the aforementioned (1-4B) may also be used as they are or after purification for the preparation of radiopharmaceuticals containing the conjugate or the chelate conjugate as the active ingredient. A radiopharmaceutical refers to a composition that contains a conjugate of the present invention or a chelate conjugate of the present invention, i.e., anti-MUC5AC humanized antibody or a derivative thereof which is labeled with a radionuclide (nuclide that emits β-ray), and is in a form suitable for in vivo administration to a subject. A radiopharmaceutical can be produced, for example, by dissolving a conjugate of the present invention or a chelate conjugate of the present invention, which is produced by the aforementioned method, in a solvent mainly containing water and isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of a disease, diagnosis of a disease, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is cancer. The cancer to be treated or diagnosed in the present invention includes, for example, pancreatic cancer, thyroid cancer, liver cancer, colorectal cancer, gastric cancer, urothelial cancer, breast cancer, cervical cancer, ovarian cancer, and endometrial carcinoma, and particularly, application to pancreatic cancer is preferred.

Examples of the cancer to be treated and diagnosed by the present invention also include bile duct cancer.

There are plural reports stating that MUC5AC is an antigen carrier for CA19-9 (PLoS ONE (December 2011, Volume 6, Issue 12, e29180, p1-10)). Therefore, examples of the cancer to be treated by the present invention also include biliary tract cancer, uterine cancer, lung cancer, and esophageal cancer overexpressing CA19-9, and these can be treated efficiency.

As used herein, the "effective amount" is an amount that can afford useful therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (e.g., cancer), and the like. Physicians and veterinarians can consider these factors and determine the appropriate effective amount.

By selecting a radionuclide having a therapeutic effect, the conjugate of the present invention or the chelate conjugate of the present invention can be used for targeted radionuclide therapy (RI internal therapy). In RI internal therapy, a radiopharmaceutical is administered intravenously or orally, this radiopharmaceutical is accumulated in a lesion site such as a primary cancer lesion or a metastatic lesion, and the cancer cells in the lesion site are destroyed by radiation emitted from the radiopharmaceutical. Therefore, the conjugate of the present invention or the chelate conjugate of the present invention can be preferably used for RI internal therapy of cancer. In this case, the amount of radioactivity to be administered and dose of the pharmaceutical are appropriately selected according to the kind of the radionuclide, the effectiveness of the active ingredient, the form and route of administration, the stage of progression of the disease (particularly cancer), body shape, body weight, age of the patient, and the kind and amount of other therapeutic

agent to be used in combination for the disease.

Furthermore, as the dosage of the drug, a human equivalent dose (HED), which is calculated by converting the dose for a mouse to a human based on the body surface area, can also be used. HED is determined by the following formula. HED=animal dose in MBq/kg × (animal weight in kg/human weight in kg) $^{0.33}$

For example, when the HED for a human weighing 60 kg is calculated using the dose for a mouse weighing 20 g and when the radionuclide is iodine-131, it can generally be administered at not more than 100 MBq/kg per dose. The effect can be exhibited even at a dose of not more than 50 MBq/kg one time. For example, when the radionuclide is yttrium-90, it can generally be administered at not more than 50 MBq/kg per dose. The effect can be exhibited even at a dose of not more than 30 kBq/kg one time. For example, when radionuclide is lutetium-177, it can generally be administered at not more than 50 MBq/kg per dose. The effect can be exhibited even at a dose of not more than 40 MBq/kg one time. Such dosage is an example of one embodiment of the present invention, and those skilled in the art can make appropriate conversion according to the above-mentioned formula, based on the body weights of mouse and human.

When radionuclide is iodine-131, the toxicity to the thyroid gland, where iodine accumulates physiologically, can be reduced by pre-administration of a non-radioactive iodine preparation (stable iodine preparation). As a stable iodine agent, potassium iodide, potassium iodate, and the like can be used.

In addition, as another embodiment of the present invention, a radiopharmaceutical containing the aforementioned conjugate or chelate conjugate in which only the radionuclide is replaced with, from the β ray emitting nuclides, a radionuclide ($^{68}$Ga, $^{64}$Cu, $^{86}$Y, $^{89}$Zr, $^{111}$In, $^{123}$I, $^{125}$I, Al$^{18}$F) that emits positron or γ-ray as the active ingredient may be prepared and used for the diagnosis of cancer in the aforementioned RI internal therapy for cancer. The diagnostic radiopharmaceutical for cancer of the present invention may be used for diagnosis before performing RI therapy for cancer, or may be used for diagnosis after performing RI therapy for cancer. Using the radiopharmaceutical for diagnosis before conducting an RI therapy for cancer, selection of treatment can be performed as to whether or not to perform RI therapy for cancer using the conjugate or chelate conjugate of the present invention provided with a radionuclide that emits β-rays. Using the radiopharmaceutical for diagnosis after conducting an RI therapy for cancer, moreover, whether or not RI therapy for cancer using the conjugate or chelate conjugate of the present invention provided with a radionuclide that emits β-rays is effective can be judged, and a treatment plan including an increase or a decrease of dose and the like can be optimized.

According to the embodiment of the present invention described above, an anti-MUC5AC antibody, particularly a humanized antibody, labeled with a radionuclide, specifically, a nuclide that emits β-rays, which is superior in specificity for MUC5AC and accumulation in tumor is provided.

Moreover, according to the embodiment of the present invention, an RI-labeled anti-MUC5AC antibody which enables cancer diagnosis and/or cancer treatment for achieving theranostics is provided.

The above-mentioned embodiment of the present invention includes the following technical ideas.

[1] A conjugate of a radionuclide and an antibody, wherein the aforementioned radionuclide is a nuclide that emits β-rays and the aforementioned antibody is a humanized antibody that specifically binds to mucin subtype 5AC (MUC5AC) (anti-MUC5AC antibody) and that comprises a heavy chain variable region consisting of

(1) the amino acid sequence shown in SEQ ID NO: 1 (H01), an amino acid sequence having not less than 90% or not less than 95% sequence identity with the amino acid sequence shown in SEQ ID NO: 1, or an amino acid sequence shown in SEQ ID NO: 1 wherein 3, 2 or 1 amino acid is/are deleted, substituted or added,
(2) the amino acid sequence shown in SEQ ID NO: 2 (H02), an amino acid sequence having not less than 90% or not less than 95% sequence identity with the amino acid sequence shown in SEQ ID NO: 2, or an amino acid sequence shown in SEQ ID NO: 2 wherein 3, 2 or 1 amino acid is/are deleted, substituted or added,
(3) the amino acid sequence shown in SEQ ID NO: 3 (H03), an amino acid sequence having not less than 90% or not less than 95% sequence identity with the amino acid sequence shown in SEQ ID NO: 3, or an amino acid sequence shown in SEQ ID NO: 3 wherein 3, 2 or 1 amino acid is/are deleted, substituted or added, or
(4) the amino acid sequence shown in SEQ ID NO: 4 (H04), an amino acid sequence having not less than 90% or not less than 95% sequence identity with the amino acid sequence shown in SEQ ID NO: 4, or an amino acid sequence shown in SEQ ID NO: 4 wherein 3, 2 or 1 amino acid is/are deleted, substituted or added, and a light chain variable region consisting of
(5) the amino acid sequence shown in SEQ ID NO: 5 (L01), an amino acid sequence having not less than 90% or not less than 95% sequence identity with the amino acid sequence shown in SEQ ID NO: 5, or an amino acid sequence shown in SEQ ID NO: 5 wherein 3, 2 or 1 amino acid is/are deleted, substituted or added,
(6) the amino acid sequence shown in SEQ ID NO: 6 (L02), an amino acid sequence having not less than 90% or not less than 95% sequence identity with the amino acid sequence shown in SEQ ID NO: 6, or an amino acid sequence shown in SEQ ID NO: 6 wherein 3, 2 or 1 amino acid is/are deleted, substituted or added,

(7) the amino acid sequence shown in SEQ ID NO: 7 (L03), an amino acid sequence having not less than 90% or not less than 95% sequence identity with the amino acid sequence shown in SEQ ID NO: 7, or an amino acid sequence shown in SEQ ID NO: 7 wherein 3, 2 or 1 amino acid is/are deleted, substituted or added, or
(8) the amino acid sequence shown in SEQ ID NO: 8 (L04), an amino acid sequence having not less than 90% or not less than 95% sequence identity with the amino acid sequence shown in SEQ ID NO: 8, or an amino acid sequence shown in SEQ ID NO: 8 wherein 3, 2 or 1 amino acid is/are deleted, substituted or added.

[2] The conjugate of the above-mentioned [1], wherein the aforementioned antibody is a humanized antibody having a heavy chain variable region consisting of

(1) the amino acid sequence shown in SEQ ID NO: 1 (H01),
(2) the amino acid sequence shown in SEQ ID NO: 2 (H02),
(3) the amino acid sequence shown in SEQ ID NO: 3 (H03), or
(4) the amino acid sequence shown in SEQ ID NO: 4 (H04), and a light chain variable region consisting of
(5) the amino acid sequence shown in SEQ ID NO: 5 (L01),
(6) the amino acid sequence shown in SEQ ID NO: 6 (L02),
(7) the amino acid sequence shown in SEQ ID NO: 7 (L03), or
(8) the amino acid sequence shown in SEQ ID NO: 8 (L04).

[3] The conjugate of the above-mentioned [1] or [2], wherein the aforementioned antibody is a humanized antibody having a heavy chain variable region consisting of

(1) the amino acid sequence shown in SEQ ID NO: 1 (H01), and a light chain variable region consisting of
(7) the amino acid sequence shown in SEQ ID NO: 7 (L03).

[4] The conjugate of any of the above-mentioned [1] to [3], wherein the aforementioned nuclide that emits β-rays is iodine-131, yttrium-90, or lutetium-177.
[5] The conjugate of any of the above-mentioned [1] to [4], wherein the aforementioned conjugate comprises a chelating agent chelated with a radionuclide, and the aforementioned radionuclide is a metal nuclide that emits β-rays.
[6] The conjugate of the above-mentioned [5], wherein the aforementioned metal nuclide that emits β-rays is yttrium-90 or lutetium-177.
[7] The conjugate of the above-mentioned [5] or [6], comprising not less than 1 molecule and not more than 8 molecules of the chelating agent per 1 molecule of the antibody.
[8] The conjugate of any of the above-mentioned [5] to [7], wherein the chelating agent site-specifically modifies an Fc region of the antibody via a linker.
[9] The conjugate of the above-mentioned [8], wherein the aforementioned linker comprises an antibody-modification peptide represented by the following formula (i) which consists of not less than 13 and not more than 17 amino acid residues, and which is formed by a crosslinking reaction of the aforementioned peptide modified by a crosslinking agent and the aforementioned antibody:

(Xa)-Xaa1-(Xb)-Xaa2-(Xc)-Xaa3-(Xd) (i)

wherein Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, and they are linked via a disulfide bond or their sulfide groups are linked via a linker, or
one is an amino acid residue derived from an amino acid having a thiol group in the side chain and the other is an amino acid residue derived from an amino acid having a haloacetyl group in the side chain, and they are linked via a thioether bond, and Xaa2 is a lysine residue, an arginine residue, a cysteine residue, an aspartic acid residue, a glutamic acid residue, 2-aminosuberic acid, or diamino propionic acid, and modified with the aforementioned crosslinking agent.

[10] The conjugate of the above-mentioned [9], wherein the antibody-modification peptide is the formula (i) wherein Xaa2 is a lysine residue.
[11] The conjugate of the above-mentioned [9] or [10], wherein the antibody-modification peptide comprises an

antibody-modification peptide consisting of the amino acid sequence shown in SEQ ID NO: 10 (wherein Xaa2 is a lysine residue).

[12] The conjugate of any of the above-mentioned [5] to [11], wherein the chelating agent has a structure derived from a compound represented by the following formula (A) or a salt thereof:

(A)

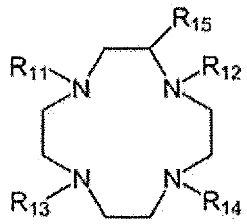

wherein in the formula (A), $R_{11}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the antibody, p is an integer of not less than 0 and not more than 3, $R_{15}$ is a hydrogen atom when $R_{12}$ is a substituent for conjugating with the antibody, and $R_{15}$ is a substituent for conjugating with the antibody when $R_{12}$ is not a substituent for conjugating with the antibody.

[13] The conjugate of any of the above-mentioned [5] to [12], wherein the chelating agent site-specifically modifies an Fc region of the antibody via a linker, the linker has a connected group formed by a click reaction.

[14] The conjugate of the above-mentioned [13], wherein the linker has a chelate linker that connects the chelating agent and the connected group formed by the click reaction, and an antibody-modification linker that connects the antibody and the connected group formed by the click reaction, and the connected group formed by the click reaction comprises a triazole skeleton-containing structure represented by the following formula (10a) or a pyridazine skeleton-containing structure:

(10a)

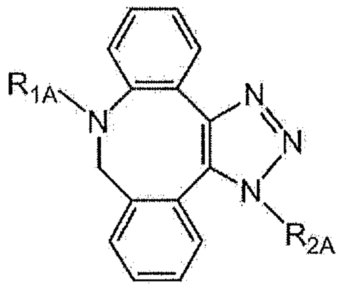

wherein $R_{1A}$ is a binding site with the chelate linker, and $R_{2A}$ is a binding site with the antibody-modification linker.

[15] A radiopharmaceutical comprising the conjugate of any of the above-mentioned [1] to [14] as the active ingredient.

[16] The radiopharmaceutical of the above-mentioned [15], that is used for RI internal therapy for cancer.

[17] The radiopharmaceutical of the above-mentioned [16], that is administered to a subject at a dose of not more than 100 MBq/kg one time in the aforementioned RI internal therapy, wherein the aforementioned radionuclide is iodine-131.

[18] The radiopharmaceutical of the above-mentioned [16], that is administered to a subject at a dose of not more than 50 MBq/kg one time in the aforementioned RI internal therapy, wherein the aforementioned radionuclide is yttrium-90.

[19] The radiopharmaceutical of the above-mentioned [16], that is administered to a subject at a dose of not more than 50 MBq/kg one time in the aforementioned RI internal therapy, wherein the aforementioned radionuclide is lutetium-177.

[20] A radiopharmaceutical for cancer diagnosis in RI internal therapy using the radiopharmaceutical of any of the above-mentioned [16] to [19] that is a radiopharmaceutical comprising a conjugate of a radionuclide or a chelating agent chelated with a radionuclide, and an antibody, wherein the antibody is a humanized antibody that specifically binds to MUC5AC.

[21] A method for producing the conjugate of any of the above-mentioned [1] to [4], comprising a conjugating step

of conjugating a radionuclide and an anti-MUC5AC antibody to produce a conjugate of the aforementioned radionuclide and the aforementioned anti-MUC5AC antibody.
[22] A method for producing the conjugate of any of the above-mentioned [5] to [14], comprising a conjugating step of conjugating a chelating agent chelated with a radionuclide and an anti-MUC5AC antibody to produce a conjugate of the chelating agent and the anti-MUC5AC antibody.
[23] The production method of the above-mentioned [22], wherein the chelating agent is connected to a chelate linker, the anti-MUC5AC antibody has an Fc region specifically modified by an antibody-modification linker provided with an antibody-modification peptide, and the chelate linker and the antibody-modification linker are connected by performing a click reaction in the conjugating step.
[24] A modified antibody with an Fc region of the antibody specifically modified by an antibody-modification linker provided with an antibody-modification peptide, wherein the aforementioned antibody is the humanized antibody of the above-mentioned [1] which specifically binds to MUC5AC, and the antibody-modification linker has an atomic group to connect to a chelate linker of a chelating agent chelated with a nuclide that emits β-rays by a click reaction.
[25] A method for producing a modified antibody in which an Fc region of the antibody is specifically modified by an antibody-modification linker provided with an antibody-modification peptide, comprising

an antibody modification step of site-specifically modifying an Fc region of the antibody with a linker provided with an antibody-modification peptide to give a modified antibody, and
an antibody purification step of purifying the antibody using a carrier with an immunoglobulin-binding protein immobilized thereon, wherein
the antibody is a humanized antibody of the above-mentioned [1] that specifically binds to MUC5AC, and
the aforementioned antibody-modification linker has an atomic group to connect to a chelate linker of a chelating agent chelated with a nuclide that emits β-rays by a click reaction.

[26] The production method of the above-mentioned [25], wherein the immunoglobulin-binding protein is protein A or genetically-engineered protein A.
[27] The production method of the above-mentioned [25] or [26], wherein the antibody purification step is performed using a column filled with the carrier.
[28] The production method of the above-mentioned [27], wherein the antibody purification step comprises a retention step of retaining the modified antibody on the carrier, and an elution step of eluting the modified antibody retained on the carrier.
[29] The production method of the above-mentioned [28], wherein the modified antibody is obtained as a mixture containing an unmodified antibody not modified by the antibody-modification linker, a monovalent antibody modified by one molecule of the antibody-modification linker with respect to one molecule of the antibody, and a divalent antibody modified by two molecules of the antibody-modification linker with respect to one molecule of the antibody in the antibody modification step, and a first antibody composition containing relatively large amounts of the unmodified antibody and the monovalent antibody and a second antibody composition containing a relatively large amount of the divalent antibody are respectively obtained utilizing the difference in the interaction of the unmodified antibody, the monovalent antibody and the divalent antibody with immunoglobulin-binding proteins in the antibody purification step.
[30] A method for producing a conjugate, comprising a modified antibody production step of performing the production method of any of the above-mentioned [25] to [29] to obtain a modified antibody, and a conjugating step of conjugating the modified antibody and a chelating agent chelated with a nuclide that emits β-rays to produce a conjugate of the chelating agent and the modified antibody.
[31] The production method of the above-mentioned [30], wherein the first antibody composition in which the proportion of a total of the unmodified antibody not modified by the antibody-modification linker and the monovalent antibody modified by one molecule of the antibody-modification linker with respect to one molecule of the antibody is larger than the divalent antibody modified by two molecules of the antibody-modification linker with respect to one molecule of the antibody is obtained in the modified antibody production step, and
the conjugate of the chelating agent and the monovalent antibody is formed in the conjugating step.
[32] The production method of the above-mentioned [31], wherein the second antibody composition in which the proportion of the divalent antibody modified by two molecules of the antibody-modification linker with respect to one molecule of the antibody is larger than a total of the unmodified antibody not modified by the antibody-modification linker and the monovalent antibody modified by one molecule of the antibody-modification linker with respect to one molecule of the antibody is obtained in the modified antibody production step, and
the conjugate of the chelating agent and the divalent antibody is formed in the conjugating step.
[33] The production method of any of the above-mentioned [30] to [32], wherein the chelating agent is connected to the chelate linker, and the chelate linker and the antibody-modification linker are connected by performing the

click reaction in the conjugating step.

[34] A kit for producing a conjugate of a radionuclide and an antibody, comprising (1) a radionuclide and (2) an anti-MUC5AC antibody, wherein the conjugate is the conjugate of any of the above-mentioned [1] to [4].

[35] A kit for producing a conjugate of a chelating agent chelated with a radionuclide, and an antibody, comprising (1) a chelating agent capable of chelating a radionuclide and (2) an anti-MUC5AC antibody, wherein the conjugate is the conjugate of any of the above-mentioned [5] to [13].

[36] The kit of [34] or [35], further comprising (1) a first atomic group capable of click reaction and (2) a second atomic group capable of click reaction.

[37] The kit of [35] or [36], further comprising a radionuclide capable of chelating with the chelating agent.

According to the radiopharmaceutical of the above-mentioned [15], since a conjugate containing a humanized antibody that specifically binds to MUC5AC and a nuclide that emits β-rays is contained as an active ingredient, when used for RI internal therapy of cancer, it accumulates specifically in tumor expressing MUC5AC, and can irradiate β-rays specifically on tumor cells, whereby higher therapeutic effect is obtained.

According to the above-mentioned method for producing a conjugate of the above-mentioned [21], since it includes a conjugate formation step of conjugating a radionuclide and an anti-MUC5AC antibody, the conjugate can be efficiently obtained by a more convenient operation.

According to the above-mentioned method for producing a conjugate of the above-mentioned [22], since it includes a conjugate formation step of conjugating a chelating agent chelated with a radionuclide and an anti-MUC5AC antibody, the conjugate can be efficiently obtained by preventing denaturation of the antibody without subjecting the anti-MUC5AC antibody to a chelating step, which is a more severe condition for the antibody.

According to the method for producing a conjugate of the above-mentioned [23], since the click reaction is included in the conjugate formation step, the conjugate can be formed in a buffer solution under extremely mild condition of room temperature, and the conjugate can be obtained efficiently without denaturing the anti-MUC5AC antibody.

According to the modified antibody of the above-mentioned [24], since the Fc region of the anti-MUC5AC antibody is specifically modified by the antibody-modification linker, the modified antibody can be used in a click reaction with respect to a chelating agent chelated with a radionuclide, without impairing the antigen-binding ability of the anti-MUC5AC antibody.

According to the method for producing a modified antibody according to the above-mentioned [25], since it includes an antibody modification step of site-specifically modifying the Fc region of an anti-MUC5AC antibody with a linker provided with an antibody-modification peptide to obtain a modified antibody, and an antibody purification step of purifying the aforementioned antibody by using a carrier with an immunoglobulin-binding protein immobilized thereon, the purity of the modified antibody can be further increased.

According to the method for producing a conjugate of the above-mentioned [31] or [32], since the conjugate formation step is performed using an antibody conjugate in which the proportion of either the monovalent antibody or the divalent antibody is higher than the proportion of the other, the number of chelating agents that bind to the anti-MUC5AC antibody can be adjusted according to the purpose, and a conjugate having a desired valence can be obtained with higher purity.

According to the kit of the above-mentioned [34], since the conjugate of any of [1] to [4] can be prepared at the time of use by reacting a conjugate of a chelating agent capable of chelating a radionuclide and an antibody with the radionuclide at a required timing, efficient treatment is possible without impairing both the half-life of the radionuclide and antibody activity.

According to the kit of the above-mentioned [35], since the conjugate of any of [5] to [13] can be prepared at the time of use by reacting a conjugate of a chelating agent capable of chelating a radionuclide and an antibody with the radionuclide at a required timing, efficient treatment is possible without impairing both the half-life of the radionuclide and antibody activity.

According to the kit of the above-mentioned [36], since a conjugate containing a chelating agent capable of chelating a radionuclide and an atomic group for click reaction and a conjugate containing an antibody and an atomic group for click reaction are separately provided, the conjugate of any of [5] to [14] can be prepared at the time of use by chelating the radionuclide with a chelating agent and click reaction thereof at a required timing, and efficient treatment is possible without impairing both the half-life of the radionuclide and antibody activity.

The present invention is explained in detail in the following by referring to Examples and the like. The present invention is not limited thereto.

[Example]

Production Example 1: Production of anti-MUC5AC humanized antibody

The amino acid sequences of various variable regions to which a signal sequence was added and the amino acid sequences of various constant regions were converted into base sequences while considering codon usage suitable for expression in CHO cells. A Kozak sequence was added to the initiation codon site of the signal sequence, and a stop codon was added to the C-terminal side of the constant region. Furthermore, restriction enzyme sites were added to the upstream of the Kozak sequence and downstream of the stop codon so that they could be introduced into the expression gene transfer site of a mammalian cell expression plasmid (pcDNA3.4). Each DNA fragment designed in this way was prepared by chemical synthesis. A DNA fragment containing a variable region to be a desired H chain and a desired L chain and a DNA fragment containing a constant region were ligated by fusion PCR.

The prepared various antibody genes were subjected to restriction enzyme treatment and then purified. Similarly, a mammalian cells transient expression plasmid (pcDNA3.4) was also treated with the same restriction enzyme and then purified. The both fragments were mixed at an appropriate mixing ratio and ligated. The ligation reaction solution was mixed with Escherichia coli DH5$\alpha$ competent cells to perform transformation. The resulting transformants were subjected to colony PCR, single colony isolation, plasmid extraction from small-scale culture medium, and nucleotide sequencing of the insert portion were performed. A plasmid (Escherichia coli clone) into which the full-length designed antibody gene was correctly inserted in the intended direction with the designed sequence (Escherichia coli clone) was selected. The selected Escherichia coli clone was subjected to large scale culture, and plasmid extraction and purification including an endotoxin removal step were performed. The concentration of the purified plasmid was calculated by measuring the absorbance at 260 nm.

Transient expression by CHO cells was performed using the ExpiCHO System (manufactured by Thermo Fisher Scientific). From each of the prepared H chain expression plasmids and each of the prepared L chain expression plasmid, one H chain and one L chain were selected to achieve the desired combination, transfected by the lipofection method, cultured, and fed. After 7 days - 13 days from transfection, the culture medium was recovered. The culture supernatants after centrifugation and filtration were added to Protein A column and the antibody was purified by general affinity column chromatography (washing after adsorption, elution with acidic buffer, neutralization of eluate). The concentration of the purified antibody was calculated by measuring the absorbance at 280 nm.

The following anti-MUC5AC humanized antibodies were prepared using the method described above. The antibody numbers assigned to the combinations of the heavy chain variable region and the light chain variable region are shown below.

antibody 1: H01L03
antibody 2: H01L04
antibody 3: H02L04
antibody 4: H04L04

As used herein, H01, H02 and H04 are heavy chain variable regions respectively shown in SEQ ID NO: 1, SEQ ID NO: 2 and SEQ ID NO: 4, L03 and L04 are light chain variable regions respectively shown in SEQ ID NO: 7 and SEQ ID NO: 8. The antibodies used in the following Examples were combinations of heavy chain constant region 1 (SEQ ID NO: 25) and light chain constant region 1 (SEQ ID NO: 26), and the heavy chain variable region and light chain variable region of the above-mentioned antibody 1 to antibody 4.

Production Example 2: Site-specific antibody modification by peptide linker

(1) Antibody modification step

An antibody-modified peptide was produced by the method described in WO 2017/217347 to obtain a peptide containing 17 amino acid residues represented by the following formula (P3). The amino acid sequence of this peptide was the same as the sequence in which Xaa2 of SEQ ID NO:10 was a lysine residue, and the side chain terminal amino group of the lysine residue was modified with the structure shown by $R_1$. In addition, two cysteine residues form a disulfide bond with each other, and to the N-terminal of the peptide was added ethyl azide as an atomic group containing an azide group, which is the second atomic group, via a linker structure having diglycolic acid and eight PEGs.

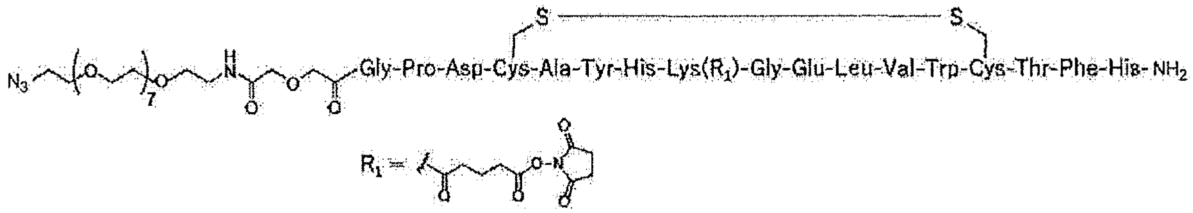

(P3)

In the formula (P3), Gly is glycine, Pro is proline, Asp is aspartic acid, Cys is cysteine, Ala is alanine, Tyr is tyrosine, His is histidine, Lys is lysine, Glu is glutamic acid, Leu is leucine, Val is valine, Trp is tryptophan, Thr is threonine, and Phe is phenylalanine.

A mixture of this peptide and anti-MUC5AC humanized antibody (antibody 1) produced in Production Example 1 in a sodium acetate buffer (pH 6.0) was reacted at room temperature for 30 min to give a solution containing a peptide-modified antibody. The peptide-modified antibody has an Fc region of the antibody site-specifically modified by the above-mentioned peptide.

(2) Peptide-modified antibody separation step

The peptide-modified antibody was diluted with 1 mol/L sodium acetate buffer (pH 6.0), added to Protein A column (manufactured by GE Healthcare, HiTrap MabSelect SuRe), and a 0.05 mol/L sodium acetate buffer (pH 5.7) containing 0.15 mol/L sodium chloride was flowed. A peptide-modified antibody (divalent antibody) to which two peptide molecules modify was recovered, and the concentration was adjusted such that the concentration of the divalent antibody contained in the recovered fraction was 15 mg/mL. Thereafter, 0.05 mol/L sodium acetate buffer (pH 3.5) containing 0.15 mol/L sodium chloride was flowed into Protein A column, a peptide-modified antibody (monovalent antibody) to which one molecule of peptide modify was recovered, and the concentration was adjusted such that the concentration of the monovalent antibody contained in the recovered fraction was 15 mg/mL.

Example 1: Production of $^{90}Y$-labeled anti-MUC5AC humanized antibody

(1) Antibody modification step

Separately, an antibody-modification peptide was produced by the method described in WO 2017/217347, and a peptide containing 17 amino acid residues represented by the following formula (P3) as obtained. The amino acid sequence of this peptide was the same as the sequence in which Xaa2 of SEQ ID NO: 10 was a lysine residue, and the side chain terminal amino group of the lysine residue was modified with the structure shown by $R_1$. In addition, two cysteine residues form a disulfide bond with each other, and to the N-terminal of the peptide was added ethyl azide as an atomic group containing an azide group, which is the second atomic group, via a linker structure having diglycolic acid and eight PEGs.

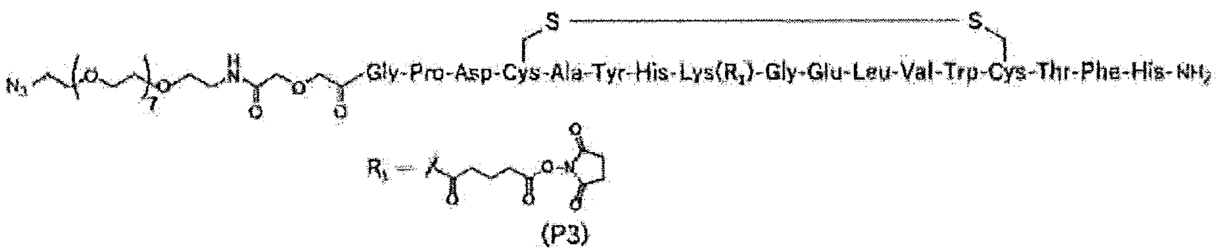

(P3)

A mixture of this peptide and the anti-MUC5AC humanized antibody (antibody 1) produced in Production Example 1 in a sodium acetate buffer (pH 6.0) was reacted at room temperature for 60 min to give a solution containing a peptide-modified antibody. The peptide-modified antibody has an Fc region of the antibody site-specifically modified by the above-mentioned peptide.

(2) Peptide-modified antibody separation step

The peptide-modified antibody was diluted with 20 mol/L sodium acetate buffer (pH 6.0), added to Protein A column (manufactured by GE Healthcare, HiTrap MabSelect SuRe), and a 0.05 mol/L sodium acetate buffer (pH 5.7)

containing 0.15 mol/L sodium chloride was flowed to recover a peptide-modified antibody with two peptide molecules modified. Thereafter, 0.05 mol/L sodium acetate buffer (pH 4.2) containing 0.15 mol/L sodium chloride was flowed to recover a peptide-modified antibody with one peptide molecule modified (monovalent antibody) in a container previously added with 1 mol/L sodium acetate buffer (pH 6.0). The buffer of each collected fraction was replaced with a 50 mmol/L histidine buffer (pH 6.1) containing 0.1 mol/L arginine using an ultrafiltration filter (manufactured by Merck, model number: UFC903096).

(3) Chelate modification step

The structure of the chelate site used in this Example is shown in the following formula (L1-5). The chelate site was dissolved in 0.1 mol/L arginine-containing 50 mmol/L histidine buffer (pH 6.1) as a solvent to give a dispersion containing 0.13 mmol/L chelate site.

This dispersion and a solution containing peptide-modified antibody were mixed and click reacted at 37°C for 90 min to give a chelate-modified antibody. The concentrations of the chelate site and the peptide-modified antibody were 56 μmol/L and 83 μmol/L, respectively, and the molar ratio of the first atomic group (DBCO) and the second atomic group (azide) was each about 1:1.3. The solution of the chelate-modified antibody was purified using an ultrafiltration filter (manufactured by Merck, model number: UFC903096), and replaced with 0.1 mol/L arginine-containing 50 mmol/L histidine buffer (pH 6.1).

L1-5

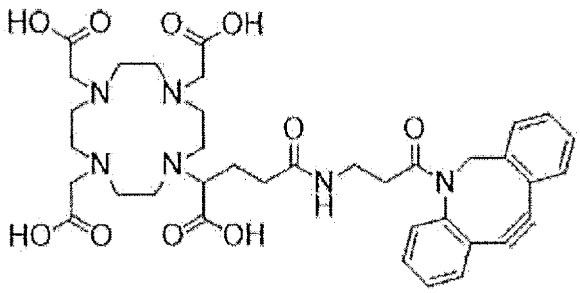

DOTAGA-DBCO

(4) Labeling step

The chelate-modified antibody solution obtained through each of the aforementioned steps was replaced with 156 mmol/L sodium acetate buffer (pH 6.0) using an ultrafiltration filter (manufactured by Merck, model number: UFC803008) to a concentration of 19.48 mg/mL. A reaction mixture of this solution (0.2 mL) and a $^{90}$Y ion-containing solution (0.04 mol/L aqueous hydrochloric acid solution, radioactivity concentration 8440 MBq/mL, manufactured by Eckert&Ziegler) (0.01 mL) as a source of radioactive metal was reacted under heating conditions to give a $^{90}$Y-labeled anti-MUC5AC humanized antibody solution. The molar ratio of the chelate site and the radioactive metal ion was chelate site:$^{90}$Y ion = about 28:1, and the heating temperature of the reaction mixture was set to 37°C, and the heating time was set to 30 min.

The obtained solution of the $^{90}$Y-labeled antibody was purified using an ultrafiltration filter (manufactured by Merck, model number: UFC803008), and subjected to the subsequent experiments. The radiochemical purity of the $^{90}$Y-labeled antibody after purification was 97.9%, and the radiochemical yield was 75.9%. As used herein, the radiochemical purity of the $^{90}$Y-labeled antibody is the ratio (%) of the radioactivity count of the peak corresponding to the $^{90}$Y-labeled antibody to the total radioactivity count of the thin layer plate when analyzed by thin layer chromatography, and the radiochemical yield of the $^{90}$Y-labeled antibody is the ratio (%) of the radioactivity of the $^{90}$Y-labeled antibody (no attenuation correction) to the radioactivity at the start of the labeling reaction.

Example 2: Production of $^{131}$I-labeled anti-MUC5AC humanized antibody

The anti-MUC5AC humanized antibody produced in Production Example 1 was replaced with Dulbecco phosphate buffered saline using an ultrafiltration filter (manufactured by Merck, model number: UFC803008), and the concentration was set to 5.07 mg/mL. 0.648 mL of Dulbecco's phosphate buffered saline and 0.185 mL of $^{131}$I ion-containing solution (sodium carbonate buffer, radioactivity concentration 2400 MBq/mL, manufactured by Institute of Isotopes) were

added to a tube coated with 1,3,4,6-tetrachloro-3α,6α-diphenylglycoluril (manufactured by Thermo Fisher Scientific, model number: 28601). 0.092 mL of the antibody solution was added to this tube and the mixed reaction solution was reacted at room temperature for 9 min to obtain a $^{131}$I-labeled anti-MUC5AC humanized antibody solution.

The obtained $^{131}$I-labeled antibody solution was purified using PD-10 column (manufactured by GE Healthcare, model number: 17-0851-01) equilibrated with 0.1 mol/L arginine-containing 0.05 mol/L histidine buffer (pH 6.1), and subjected to the subsequent experiments. The radiochemical purity of the $^{131}$I-labeled antibody after purification was 98.5%, and the radiochemical yield thereof was 86.5%. As used herein, the radiochemical purity of the $^{131}$I-labeled antibody is the ratio (%) of the radioactivity count of the peak corresponding to the $^{131}$I-labeled antibody to the total radioactivity count of the thin layer plate when analyzed by thin layer chromatography, and the radiochemical yield of the $^{131}$I-labeled antibody is the ratio (%) of the radioactivity of the $^{131}$I-labeled antibody (no attenuation correction) to the radioactivity at the start of the labeling reaction.

Example 3: Binding property and specificity of β nuclide-labeled anti-MUC5AC humanized antibody for MUC5AC

$^{90}$Y-labeled anti-MUC5AC humanized antibody was produced according to Example 1. In addition, $^{131}$I-labeled anti-MUC5AC humanized antibody was produced according to Example 2. Using these β-nuclide-labeled anti-MUC5AC humanized antibodies, the binding property and specificity of each β-nuclide-labeled antibody to MUC5AC was evaluated by in vitro ARG.

A section with a thickness of 7 μm was prepared using a cryostat (manufactured by Leica) from a frozen block obtained by freezing MUC5AC high expression tumor (SW1990 transplanted tumor tissue) or MUC5AC low expression tumor (MIA PaCa-2 transplanted tumor tissue) in liquid nitrogen and used for in vitro ARG.

A section stored at -80°C was returned to room temperature, dried for 30 min or longer, immersed in phosphate buffered saline for 30 min, and then in phosphate buffered saline containing 1%(w/v) bovine serum albumin for 30 min, whereby the section was hydrophilized.

To the hydrophilized cryosection was added dropwise about 100 μL of a solution of each β-nuclide-labeled antibody diluted with phosphate buffered saline containing 1% (w/v) bovine serum albumin to 100-fold concentration as much as that used for administration to tumor-bearing mice in the below-mentioned Examples 4-1 to 4-5, and the cryosection was left standing for 30 min. Then, the section was washed by immersing for 10 min in 1%(w/v) bovine serum albumin-containing phosphate buffered saline, 5 min in phosphate buffered saline, and 5 min in phosphate buffered saline. The section after washing was air dried, exposed in an imaging plate (BAS-SR2040, manufactured by FUJIFILM Wako Pure Chemical Corporation) for about 30 min to 2 hr, and an autoradiogram was obtained using a fluoroimage analyzer (Typhoon FLA 7000, manufactured by GE Healthcare).

The resulting images are shown in Fig. 1 and Fig. 2. A ROI was set on each whole section of a MUC5AC high expression tumor section and a low expression tumor section, and the ratio of the binding to the MUC5AC high expression tumor and to the low expression tumor was calculated using the calculated average intensity in the ROI of each tumor section. After determining the intensity ratio to the standard source as to high expression and low expression, the binding ratio was calculated by dividing the value in high expression by the value in low expression. As a result, the binding of the $^{90}$Y-labeled anti-MUC5AC humanized antibody to MUC5AC high expression tumor was about 570-fold binding ratio than the binding to the low expression tumor. The binding of the $^{131}$I-labeled anti-MUC5AC humanized antibody to MUC5AC high expression tumor was about 140-fold binding ratio than the binding to the low expression tumor, where the results were independent of the amount of radioactivity added. Binding to sections of MUC5AC low expression tumor could not be confirmed on the same scale as sections of MUC5AC high expression tumor. In order to confirm binding, it was necessary to lower the upper limit of contrast from about 15000 - 20000 to about 100 - 300. It was confirmed that the $^{90}$Y-labeled anti-MUC5AC humanized antibody and the $^{131}$I-labeled anti-MUC5AC humanized antibody both maintained the binding property and specificity to MUC5AC.

Examples 4-1 to 4-5 and Comparative Example 1: Drug efficacy evaluation of $^{90}$Y-labeled anti-MUC5AC humanized antibody and $^{131}$I-labeled anti-MUC5AC humanized antibody

The $^{90}$Y-labeled anti-MUC5AC humanized antibody and the $^{131}$I-labeled anti-MUC5AC humanized antibody were administered to tumor-bearing mice, and study was conducted to confirm the tumor growth suppressive effect. The $^{90}$Y-labeled anti-MUC5AC humanized antibody and the $^{131}$I-labeled anti-MUC5AC humanized antibody were respectively produced according to Examples 1 and 2.

A mixture of a suspension of human pancreatic cancer cell line SW1990 and an equal amount of Matrigel (manufactured by Corning) was subcutaneously administered at a cell count of $5 \times 10^6$ to BALB/c-nu/nu mouse (male, manufactured by Japan SLC Co., Ltd.) from the flank to the back thereof under isoflurane anesthesia. When the tumor volume reached about 150-300 mm$^3$ 9 days after transplantation of SW1990, each β-nuclide-labeled antibody was administered from the tail vein of the mice. The tumor volume and body weight when $^{90}$Y-labeled or $^{131}$I-labeled anti-

MUC5AC humanized antibody was administered are shown in Table 1. The tumor volume was calculated from the following calculation formula.

$$\text{tumor volume} = ((\text{tumor minor axis})^2 \times \text{tumor major axis})/2$$

$^{90}$Y-labeled anti-MUC5AC humanized antibody was administered to tumor-bearing mice at a radioactivity of 3.7 MBq/mouse (Example 4-1) or 7.4 MBq/mouse (Example 4-2), and the property of the $^{90}$Y-labeled anti-MUC5AC humanized antibody was evaluated. $^{131}$I-labeled anti-MUC5AC humanized antibody was administered to tumor-bearing mice at a radioactivity of 3.7 MBq/mouse (Example 4-3) or 7.4 MBq/mouse (Example 4-4) or 14.8 MBq/mouse (Example 4-5), and the property of the $^{131}$I-labeled anti-MUC5AC humanized antibody was evaluated. In addition, a group (antibody control group) in which a solution containing only the anti-MUC5AC humanized antibody in a 50 mmol/L histidine buffer containing 100 mmol/L arginine (pH 6.1) containing 0.07% polysorbate 80 was administered was established (Comparative Example 1). The radioactivity concentration of each labeled anti-MUC5AC humanized antibody administration group was adjusted such that the same amount of antibody as that of the antibody control group was administered to the tumor-bearing mice. Each group contained 6 mice, and observation of general condition and measurement of the body weight and tumor volume were performed for 7 weeks after administration.

[Table 1]

| | tumor volume ($mm^3$) | body weight (g) |
|---|---|---|
| Comparative Example 1 | 271±83 | 21.2±1.1 |
| Example 4-1 | 260±72 | 21.8±1.1 |
| Example 4-2 | 248±65 | 21.6±1.0 |
| Example 4-3 | 214±76 | 22.4±1.3 |
| Example 4-4 | 198±56 | 22.5±1.3 |
| Example 4-5 | 191±23 | 22.2±1.1 |
| mean±standard deviation, n=6 | | |

[Evaluation 1] Changes in tumor volume and weight

The results of confirming changes in tumor volume over time are shown in Fig. 3. When compared with the antibody control group 5 weeks after administration, it was observed that the tumor volume was statistically significantly lower in $^{90}$Y-labeled antibody 3.7 MBq administration group (Example 4-1), 7.4 MBq administration group (Example 4-2), $^{131}$I-labeled antibody 3.7 MBq administration group (Example 4-3), and 7.4 MBq administration group (Example 4-4), whereby the tumor growth suppressive effect by the administration of β-nuclide-labeled antibody was confirmed. Furthermore, in the $^{131}$I-labeled antibody-administered radioactivity 14.8 MBq group (Example 4-5), remarkable weight loss was observed after administration, and the mice were sequentially euthanized when the humane endpoint was reached.

Autopsy was performed on each β-nuclide-labeled antibody administration group on the final day of the observation period. Autopsy was performed in the antibody control group when the average tumor volume exceeded 2000 $mm^3$. In the $^{131}$I-labeled antibody-administered radioactivity 14.8 MBq group, autopsies were sequentially performed when an individual appeared with a relative ratio to body weight at the time of administration of less than 0.80. The tumor was collected, accumulation of fluid such as blood was removed, and the weight of the actual tumor tissue was measured. The comparison results of the tumor weight are shown in Table 2.

[Table 2]

| | tumor weight (g) |
|---|---|
| Comparative Example 1 | 1.43±0.49*1 |
| Example 4-1 | 1.38±0.66 |
| Example 4-2 | 0.26±0.09 |
| Example 4-3 | 0.73±0.39 |

(continued)

| | tumor weight (g) |
|---|---|
| Example 4-4 | 0.11±0.04 |
| Example 4-5 | 0.15±0.03*2 |
| mean±standard deviation, n=6 (in $^{131}$I-labeled antibody-administered radioactivity 3.7 MBq group, 7.4 MBq group, and 14.8 MBq group, n=5, n=3, n=5, respectively)<br>*1: In Comparative Example 1, autopsy was performed 4 weeks after administration and tumor was collected.<br>*2: In Example 4-5, two mice each were autopsied 13, 21, and 23 days after administration, and tumor was collected. | |

[Evaluation 2] Body weight change

The results of confirming the changes in body weight over time are shown in Fig. 4, and the lowest values of the relative ratio of body weight in each administered radioactivity group and time points thereof are shown in Table 3. In each β-nuclide-labeled antibody administration group, a decrease in the body weight was observed in an administered radioactivity dose-dependent manner for about 10 days after administration. However, in the groups other than Example 4-5, the relative ratio to the average body weight at the time of administration was not below 0.85, and toxicity reaching the humane endpoint was not observed. Moreover, from day 13 to day 36 after administration, the body weight recovered to the level more than before administration. On the other hand, in Example 4-5, the decrease continued even after 10 days from administration, and multiple individuals with a relative ratio of less than 0.80 to the body weight at the time of administration appeared. It was thus considered that toxicity reaching the humane endpoint was observed, and autopsy was performed sequentially.

[Table 3]

| | lowest value of relative ratio of body weight and time point thereof |
|---|---|
| Example 4-1 | 0.93 (day 8) |
| Example 4-2 | 0.86 (day 8) |
| Example 4-3 | 0.94 (day 9) |
| Example 4-4 | 0.87 (day 15) |
| Example 4-5 | 0.78 (day 20) |

[Evaluation 3] Thyroid toxicity

Plasma samples collected at the end of the observation period were used to evaluate thyroid toxicity (Thyroxine in plasma was measured using Thyroxine (T4) (Mouse/Rat) ELISA Kit (manufactured by BioVision)). In addition, in Example 4-5, plasma samples collected at the time when the humane endpoint was reached were used.

The results of thyroid toxicity are shown in Fig. 5. In Example 4-3 (n=6) and Example 4-5 (n=5), it was observed that the plasma Thyroxine concentration was statistically significantly lower than that in the antibody control group (n=6). In Example 4-4 (n=3), a decreasing tendency in plasma Thyroxine concentration was observed. The above results suggest that administration of the $^{131}$I-labeled antibody induced thyrotoxicity. On the other hand, in the $^{90}$Y-labeled antibody administration group (all n=6), the decrease in plasma Thyroxine concentration observed in the $^{131}$I-labeled antibody administration group was not observed, thus suggesting that the possibility of thyroid toxicity due to $^{90}$Y-labeled antibody administration is low.

[Evaluation 4] Pathological evaluation

At the end of the observation period, the thyroid gland and femur (including femoral bone marrow) were collected. The thyroid gland was collected from the area including the thyroid gland from the laryngopharynx to the upper part of the trachea. Regarding the femur, the left femur was collected. Thyroid glands were fixed in 20% neutral buffered formalin for over 1 week. The femur was fixed in Bouin's solution for over 1 week and decalcified. The thyroid gland was not excised and was embedded as was. The femur was longitudinally sectioned to include the knee joint surface and embedded. Thereafter, thin sections were prepared according to a conventional method, hematoxylin and eosin - stained specimens were prepared, and histopathological evaluation was performed. The results are shown in Fig. 6 and Fig. 7.

Regarding the femur, each β-nuclide-labeled antibody administration group and the antibody control group (Comparative Example 1) were compared (Fig. 6). In Example 4-3, a decrease in the cancellous bone under the epiphyseal cartilage plate was observed, and the degree thereof increased in the order of Example 4-4 and Example 4-5. That is, in the $^{131}$I-labeled antibody administration group, bonetoxicity dependent on the administered radioactivity was observed. On the other hand, in the $^{90}$Y-labeled antibody administration groups (Examples 4-1 and 4-2) and antibody control group (Comparative Example 1), bone change was not observed. In Example 4-4, a slight decrease in bone marrow hematopoietic cells was observed, and in Example 4-5, bone marrow hematopoietic cells were significantly reduced or disappeared. That is, in the $^{131}$I-labeled antibody administration group, bone marrow toxicity dependent on the administered radioactivity was observed. On the other hand, in the $^{90}$Y-labeled antibody administration groups (Examples 4-1 and 4-2) and antibody control group (Comparative Example 1), bone marrow change was not observed.

Regarding the thyroid gland, each β-nuclide-labeled antibody administration group and the antibody control group (Comparative Example 1) were compared (Fig. 7). In Example 4-3, atrophy of the thyroid follicles was observed, but obvious inflammatory cell infiltration and necrosis of the follicular epithelium were only observed here and there. In Examples 4-4 and 4-5, significant atrophy, necrosis, and disappearance of thyroid follicles, fibrotic replacement of thyroid tissue, and inflammatory cell infiltration were observed to varying degrees, and the levels thereof were stronger in Example 4-5. That is, in the $^{131}$I-labeled antibody administration group, administered radioactivity dependent thyroid toxicity was observed. On the other hand, in the $^{90}$Y-labeled antibody administration groups

(Examples 4-1 and 4-2) and antibody control group (Comparative Example 1), thyroid gland change was not observed.

Example 5: Production of $^{177}$Lu-labeled anti-MUC5AC humanized antibody

(1) Chelating agent synthesis step

The structure of the chelate site used in this Example is the same as the aforementioned formula (L1-5). This chelating agent was dispersed in 0.156 mol/L sodium acetate buffer (pH 5.5) as a solvent to give a dispersion containing 0.45 mmol/L chelating agent. A reaction mixture of the dispersion (0.004 mL), 0.156 mol/L sodium acetate buffer (pH 5.5, 0.004 mL) dissolving 0.225 mmol/L gentisic acid, and $^{177}$Lu ion-containing solution (0.04 mol/L aqueous hydrochloric acid solution, radioactivity concentration 3.4 GBq/mL, prepared from one produced by POLATOM, liquid amount 0.01 mL, amount of radioactivity 3.73 MBq) as a radioactive metal source was reacted under heating conditions to give a $^{177}$Lu complex solution. The molar ratio of the chelate site and the radioactive metal ion was chelate site:$^{177}$Lu ion = about 350:1, and the heating temperature of the reaction mixture was set to 70°C, and the heating time was set to for 5 min.

The radiochemical purity of the obtained $^{177}$Lu complex was measured in the same manner as in the measurement of radiochemical purity of the radioconjugate of Example 1. As a result, the radiochemical purity of the $^{177}$Lu complex was 47.4%. The obtained $^{177}$Lu complex solution was directly used for the labeling step.

(2) Labeling step

A solution of the unpurified $^{177}$Lu complex obtained in the aforementioned step (1), and a solution containing a peptide-modified antibody (monovalent antibody) of an anti-MUC5AC humanized antibody obtained in Example 1 were subjected to a click reaction at 37°C for 2 hr to give $^{177}$Lu-labeled anti-MUC5AC humanized antibody. The amount of the $^{177}$Lu complex and the amount of the peptide-modified antibody (monovalent antibody) were 1.8 nmol and 2.6 nmol, respectively, and the molar ratio of the DBCO group and the azide group was about 1:1.4. The reaction rate (%) of the unpurified $^{177}$Lu-labeled anti-MUC5AC humanized antibody is shown in the following Table 4.

In addition, the radiochemical purity (RCP) and radiochemical yield (RCY) of the $^{177}$Lu-labeled anti-MUC5AC humanized antibody after purification using an ultrafiltration filter in the same manner as in Example 1 are shown in the following Table 4.

The radiochemical purity and radiochemical yield of the $^{177}$Lu-labeled anti-MUC5AC humanized antibody were measured in the same manner as in Example 1.

[Table 4]

| | chelating agent (A) | antibody (B) | molar ratio (A) : (B) | reaction rate (%) by 37°C, 2 hr reaction | after purification | |
|---|---|---|---|---|---|---|
| | | | | | RCP (%) | RCY (%) |
| Example 5 | DOTAGA | Production Example 1 | 1:1.4 | 38.8 | 95.1 | 66.8 |

Example 6: Formulation step

To 33 μL of the $^{177}$Lu-labeled anti-MUC5AC humanized antibody solution obtained in Example 5 was diluted to 37 MBq/mL by adding a buffer for adjusting radioactivity concentration (50 mmol/L histidine buffer containing 100 mmol/L arginine containing 0.7 mass% polysorbate 80, pH 6.1).

[Evaluation 5] Stability evaluation

The $^{177}$Lu-labeled anti-MUC5AC humanized antibody obtained in Example 6 was stored at room temperature for 4 weeks, and the radiochemical purity was evaluated each time point (0 day point, 1 day point, 7 day point, 14 day point, 20 day point, 27 day point). Note that 7 days from the end of production corresponds to about 1 half-life of $^{177}$Lu, 14 days from the end of production corresponds to about 2 half-lives of $^{177}$Lu, 20 days from the end of production corresponds to about 3 half-lives of $^{177}$Lu, and 27 days from the end of production corresponds to about 4 half-lives of $^{177}$Lu.

[Evaluation 5-1]

The radiochemical purity was analyzed by thin layer chromatography (TLC). The TLC conditions were similar to those used in Example 1. The results are shown in Table 5.

[Table 5]

| | radiochemical purity (%) | | | | | |
|---|---|---|---|---|---|---|
| | 0 day point | 1 day point | 7 day point | 14 day point | 20 day point | 27 day point |
| Example 6 | 96.0 | 95.9 | 92.9 | 93.8 | 93.7 | 92.8 |

Example 7: Evaluation of binding property and specificity of $^{177}$Lu-labeled anti-MUC5AC humanized antibody to MUC5AC

The binding property and specificity of $^{177}$Lu-labeled anti-MUC5AC humanized antibody to MUC5AC was evaluated in the same manner as in Example 3. Note that the $^{177}$Lu-labeled anti-MUC5AC humanized antibody was produced according to Example 5.

The images and graphs of the results are shown in Fig. 16 and Fig. 17. The binding ratio between MUC5AC high expression tumor and low expression tumor was calculated in the same manner as in Example 3. As a result, the binding of the $^{177}$Lu-labeled anti-MUC5AC humanized antibody to MUC5AC high expression tumor showed about 200-fold binding ratio as compared with that to low expression tumor, and the results were independent of the added radioactivity. Note that binding to MUC5AC low expression tumor sections could not be confirmed on the same scale as high expression tumor sections, and it was necessary to lower the upper limit of contrast from about 7000 - 10000 to about 300 to confirm the binding. From these results, it was confirmed that the $^{177}$Lu-labeled anti-MUC5AC humanized antibody retained binding property and specificity to MUC5AC.

Examples 8-1 to 8-3 and Comparative Example 2: Drug efficacy evaluation of $^{177}$Lu-labeled anti-MUC5AC humanized antibody

The $^{177}$Lu-labeled anti-MUC5AC humanized antibody was administered to tumor-bearing mice, and a study was conducted to confirm the tumor growth suppressive effect. The $^{177}$Lulabeled anti-MUC5AC humanized antibody was produced according to Example 5.

Tumor-bearing mice were produced in the same manner as in Examples 4-1 to 4-5. $^{177}$Lu-labeled anti-MUC5AC humanized antibody was administered to tumor-bearing mice at administered radioactivity 7.4 MBq/mouse (Example 8-1), 11.1 MBq/mouse (Example 8-2), or 16.7 MBq/mouse (Example 8-3), and the property of the $^{177}$Lu-labeled anti-MUC5AC humanized antibody was evaluated. In addition, a group (antibody control group) in which a solution containing only the anti-MUC5AC humanized antibody in a 50 mmol/L histidine buffer containing 100 mmol/L arginine (pH 6.1) containing 0.07% polysorbate 80 was administered was established (Comparative Example 2). The radioactivity concentration of the $^{177}$Lu-labeled anti-MUC5AC humanized antibody administration group was adjusted such that the same amount of antibody as that of the antibody control group was administered to the tumor-bearing mice. The 7.4 MBq administration group (Example 8-1) contained 5 mice, and other groups contained 6 mice, and observation of general condition, measurement of the body weight and tumor volume were performed in the same manner as in Examples 4-1 to 4-5 for 7 weeks after administration. The tumor volume and body weight when $^{177}$Lulabeled anti-MUC5AC humanized

antibody was administered are shown in Table 6.

[Table 6]

| | tumor volume ($mm^3$) | body weight (g) |
|---|---|---|
| Comparative Example 2 | 188±24 | 21.3±1.1 |
| Example 8-1 | 187±32 | 21.1±1.4 |
| Example 8-2 | 196±43 | 21.1±1.2 |
| Example 8-3 | 181±28 | 20.7±1.5 |
| mean±standard deviation, n=6 (in Example 8-1, n=5) | | |

[Evaluation 1] Changes in tumor volume and weight

The results of confirming the changes in tumor volume over time are shown in Fig. 18. When compared with the antibody control group on the final day of the observation period, it was found that the tumor volumes were statistically significantly lower in all groups of the $^{177}$Lu-labeled anti-MUC5AC humanized antibody administration group, and a tumor growth suppressive effect by the administration of the $^{177}$Lulabeled anti-MUC5AC humanized antibody was confirmed. On the final day of the observation period, autopsies were performed on all groups, the tumors were collected, accumulation of fluid such as blood was removed, and the weight of the actual tumor tissue was measured. The comparison results of the tumor weight are shown in Table 7. In the groups administered with the $^{177}$Lu-labeled anti-MUC5AC humanized antibody, it was observed that the weight of actual tumor tissue was statistically significantly lower in all groups as compared with the antibody control group, and a tumor growth suppressive effect by the administration of the $^{177}$Lu-labeled anti-MUC5AC humanized antibody was confirmed. In addition, since one mouse in the 16.7 MBq administration group (Example 8-3) showed remarkable weight loss after administration, it was euthanized 19 days after administration and an autopsy was performed.

[Table 7]

| | tumor weight (g) |
|---|---|
| Comparative Example 2 | 1.48±0.54 |
| Example 8-1 | 0.62±0.19* |
| Example 8-2 | 0.31±0.06* |
| Example 8-3 | 0.18±0.05* |
| mean±standard deviation, n=6 (in Examples 8-1 and 8-3, n=5)<br>*p<0.05 vs Comparative Example 2, the test was performed using the Steel method. | |

[Evaluation 2] Body weight change

The results of confirming the changes in body weight over time are shown in Fig. 19, and the lowest values of the relative ratio of body weight to that at the time of administration in each administered radioactivity group and time points thereof are shown in Table 8. In each $^{177}$Lu-labeled anti-MUC5AC humanized antibody administration group, a decrease in the body weight was observed in an administered radioactivity dose-dependent manner for about 8 days after administration. However, the relative ratio to the average body weight of each group at the time of administration was not below 0.85. Thus, no toxicity was observed that would result in a weight loss of not less than 25% in 7 days, which is one guideline for the humane endpoint.

[Table 8]

| | lowest value of relative ratio of body weight | |
|---|---|---|
| | each individual | mean (time point of lowest value) |
| Example 8-1 | 0.90 - 0.95 | 0.94 (day 8) |
| Example 8-2 | 0.85 - 0.93 | 0.89 (day 8) |

(continued)

| | lowest value of relative ratio of body weight | |
|---|---|---|
| | each individual | mean (time point of lowest value) |
| Example 8-3 | 0.80 - 0.92 | 0.87 (day 8) |

[Evaluation 3] Thyroid toxicity

Thyroid toxicity was evaluated using plasma samples collected at the end of the observation period or at the time of euthanasia during the observation period. Tyroid toxicity was evaluated by measuring thyroxine in plasma using Thyroxine (T4) (Mouse/Rat) ELISA Kit (manufactured by BioVision). The results are shown in Fig. 20. In the anti-MUC5AC humanized antibody administration group, the decrease in plasma Thyroxine concentration observed in the $^{131}$I-labeled anti-MUC5AC humanized antibody administration groups in Examples 4-3 to 4-5 was not observed, which suggests that the possibility of thyroid toxicity due to the administration of anti-MUC5AC humanized antibody is low.

[Evaluation 4] Pathological evaluation

Thyroid gland and femur (including femoral bone marrow) were collected at the end of the observation period or at the time of euthanasia during the observation period. Histopathological evaluation was performed in the same manner as in "Examples 4-1 to 4-5, Comparative Example 1". The results are shown in Fig. 21 and Fig. 22. Regarding the femur, each anti-MUC5AC humanized antibody administration group and the antibody control group (Comparative Example 2) were compared (Fig. 21). The administered radioactivity-dependent bone toxicity observed in the $^{131}$I-labeled antibody administration groups in Examples 4-3 to 4-5 was not observed in the anti-MUC5AC humanized antibody administration group (Examples 8-1 to 8-3) and the antibody control group (Comparative Example 2). In the anti-MUC5AC humanized antibody 7.4 MBq (Example 8-1), 11.1 MBq (Example 8-2) administration groups and antibody control group (Comparative Example 2), bone marrow change was not observed. In the 16.7 MBq administration group (Example 8-3), a slight decrease in bone marrow hematopoietic cells was observed in one of the five cases autopsied at the end of the observation period. In one case that was euthanized during the observation period, a remarkable decrease in bone marrow hematopoietic cells was observed.

Regarding the thyroid gland, each anti-MUC5AC humanized antibody administration group was compared with the antibody control group (Fig. 22). The administered radioactivity-dependent thyroid toxicity observed in the $^{131}$I-labeled anti-MUC5AC humanized antibody administration group in Examples 4-3 to 4-5 was not observed in the anti-MUC5AC humanized antibody administration group (Examples 8-1 to 8-3) and antibody control group (Comparative Example 2).

Example 9: Production of $^{89}$Zr-labeled anti-MUC5AC humanized antibody using $^{89}$Zr-labeled DOTAGA-DBCO (HPLC purification)

(1-1. Chelating agent synthesis step)

In this Example, a chelate site same as the chelate site shown by the above-mentioned formula (L1-5) was used. This chelate site was dispersed in DMSO solution to give a dispersion containing 2.0 mmol/L chelate site. A reaction mixture of the dispersion (0.150 mL), and $^{89}$Zr ion-containing solution (0.1 mol/L aqueous hydrochloric acid solution, radioactivity concentration 1335 MBq/mL, prepared from one produced by Nihon Medi-Physics Co., Ltd., liquid amount: 0.100 mL) 134 MBq, and 300 mmol/L gentisic acid-containing 780 mmol/L acetate buffer (0.050 mL) as a source of radioactive metal was reacted under heating conditions to give a $^{89}$Z complex solution. The molar ratio of the chelate site and the radioactive metal ion was chelate site:$^{89}$Zr ion = about 3333:1, and the heating temperature of the reaction mixture was set to 70°C, and the heating time was set to 60 min.

The radiochemical purity of the obtained $^{89}$Zr complex was measured by the following method. That is, a part of the $^{89}$Zr complex solution was developed by thin layer chromatography (manufactured by Agilent, model number: SGI0001, eluent: acetonitrile/water mixed solution (volume ratio 1:1)), and then measured by radio γ-TLC Analyzer (manufactured by raytest, MODEL GITA Star PS). The percentage of the radioactivity (count) of the peak detected near the origin with respect to the detected total radioactivity (count) was defined as the radiochemical purity (%) of the $^{89}$Zr complex. As a result, the radiochemical purity of the $^{89}$Zr complex was 98%.

(1-2. $^{89}Zr$ complex purification step)

The $^{89}Zr$ complex solution obtained in the aforementioned (1-1. Chelating agent synthesis step) was collected by high performance liquid chromatography (HPLC), and unreacted DOTAGA-DBCO was removed. The solvent was evaporated from the obtained collected solution to about 30 μL of the solution and the solution was used in the labeling step. The method for measuring the radiochemical yield (HPLC recovery rate) in the step of removing the unreacted substance of the $^{89}Zr$ complex-labeled antibody was as follows. That is, the percentage of radioactivity in the collected solution with respect to the amount of radioactivity charged at the start of this step was defined as the HPLC recovery rate (%) in the unreacted substance removal step.

The HPLC conditions were as follows, and fractions with a retention time of around 27 min were collected.

<HPLC conditions>

detector: ultraviolet absorption spectrophotometer (measurement wavelength: 220 nm, 254 nm)/scintillation detector
column: XBridge C18 3.5 μm, 4.6 × 100 mm, manufactured by Waters
flow rate: 0.5 mL/min
area measurement range: 45 min after sample injection
mobile phase A: 10 mmol/L histidine buffer (pH 6.5)
mobile phase B: acetonitrile for liquid chromatography
mobile phase C: acetonitrile/water mixed solution (1:1)
mobile phase feed: concentration gradient was controlled by changing the mixing ratio of mobile phase A, mobile phase B and mobile phase C as follows

[Table 9]

| time (min) after injection | mobile phase A (vol%) | mobile phase B (vol%) | mobile phase C (vol%) |
|---|---|---|---|
| 0.0 - 40.0 | 90→450 | 10→50 | 0 |
| 40.0 - 40.1 | 50→1 | 50→99 | 0 |
| 40.1 - 45.0 | 1 | 99 | 0 |
| 45.1 - 46.0 | 1→0 | 99→10 | 0→90 |
| 46.0 - 50.0 | 0 | 10 | 90 |
| 50.0 - 50.1 | 0→90 | 10 | 90→0 |
| 50.1 - 55.0 | 90 | 10 | 0 |

(2. Labeling step)

A solution of the $^{89}Zr$ complex obtained by each of the aforementioned steps and a solution containing a peptide-modified antibody (monovalent antibody; H01L03) produced in the same manner as in Production Example 2 were mixed and click-reacted at 37°C for 1.5 hrs to give a $^{89}Zr$ complex-labeled antibody. The amounts of the chelate site containing the $^{89}Zr$-labeled complex, and the peptide-modified antibody (monovalent antibody) were 73 pmol and 50 nmol, respectively, and the molar ratio of the first atomic group (DBCO) and the second atomic group (azide) was about 1:685.

Furthermore, a solution of the $^{89}Zr$ complex-labeled antibody obtained by reacting at 37°C for 1.5 hr was purified using ultrafiltration filter (manufactured by Merck, model number: UFC505096). The radiochemical purity of the $^{89}Zr$ complex-labeled antibody after purification was 95%, and the radiochemical yield was 50%. The measurement method of the radiochemical purity and radiochemical yield of the $^{89}Zr$ complex-labeled antibody was as follows. That is, thin layer chromatography (manufactured by Agilent, model number: SGI0001, eluent was mixed solution of acetonitrile:0.1 mmol/L EDTA solution (volume ratio 1:1)) was measured by radio γ-TLC Analyzer (manufactured by raytest, MODEL GITA Star PS), and the percentage of the radioactivity (count) of the peak detected near the origin with respect to the detected total radioactivity (count) was defined as the radiochemical purity (%). In addition, the percentage of the radioactivity recovered after ultrafiltration purification with respect to the total radioactivity added at the start of the labeling step was defined as the radiochemical yield (%).

Example 10: PET-CT imaging of $^{89}Zr$-labeled anti-MUCSAC humanized antibodies prepared using $^{89}Zr$-labeled DOTAGA-DBCO

$^{89}Zr$-labeled antibodies were produced using $^{89}Zr$-labeled DOTA-Bn-DBCO and DOTAGA-DBCO according to Example 8, each was administered to a tumor-bearing mouse, and evaluation using PET-CT imaging was performed.

Human pancreatic cancer-derived cell line SW1990 ($0.7 \times 10^7$ cells) which is a MUC5AC high expression tumor cell line was subcutaneously administered to BALB/c-nu/nu mouse (male, manufactured by Japan SLC Co., Ltd.) from the flank to the back thereof. When the tumor volume reached about 150-300 $mm^3$ after transplantation, the $^{89}Zr$-labeled anti-MUC5AC humanized antibodies were administered from the tail vein of the mice. The tumor volume was calculated from the following calculation formula.

$$\text{tumor volume} = ((\text{tumor minor axis})^2 \times \text{tumor major axis})/2$$

The radiochemical purity of the administered the $^{89}Zr$-labeled anti-MUC5AC humanized antibodies and animal information are shown in Table 7.

[Table 10]

| Administered radioactivity amount and animal information (on administration of various $^{89}Zr$-labeled antibodies) | | |
|---|---|---|
| name of antibody | $^{89}Zr$-labeled anti-MUC5AC humanized antibody | |
| | DOTA-Bn | DOTAGA |
| tumor volume ($mm^3$) | 337±142 | 335±129 |
| body weight (g) | 21.6±1.2 | 22.1±0.8 |
| radiochemical purity (%) | 92% | 94% |
| administered radioactivity amount (MBq) | 4.6±0.2 | 4.6±0.0 |
| (mean±standard deviation, n=4) | | |

PET-CT imaging (small animal PET-CT apparatus: Si78, manufactured by Bruker) was performed under the conditions in the following Table. The time points for PET and CT imaging were 12, 48, 84, 168, 252 hr after administration. Image reconstruction was performed by the MLEM method for PET and the FBP method for CT. VOI analysis of the SUV (standardized uptake value) of the tumor and heart (blood), liver at each time point was performed, and the SUV profile was compared using the time activity curve.

[Table 11]

| PET imaging and reconstruction conditions | |
|---|---|
| Isotope | 89-Zr |
| acquisition time | 600 sec |
| Energy window | 30% (357.7-664.3keV) |
| PET image reconstruction | MLEM GPU 32x32 0.25 (Iterations:12) |
| correction | Scatter, Randoms, Decay, Partial volume, Attenuation |

[Table 12]

| CT imaging conditions | |
|---|---|
| Acquisition Mode | Static |
| Scanning Mode | Continuous (7 deg/s) |
| X-ray Source Filter | AL-1.0 mm |
| Pixel Size | 50 um |

(continued)

| CT imaging conditions | |
|---|---|
| X-ray tube current | 771 uA |
| X-ray tube voltage | 65 kV |

The results of performing PET-CT imaging 48 hr after $^{89}$Zr-labeled antibody administration are shown in Fig. 8. The results of VOI analysis of the tumor and heart (blood), liver at each time point are shown in Fig. 9. In addition, the results of tumor to liver ratio at each time point are shown in Fig. 10.

The maximum value of accumulation in tumor was not less than 2.8 as SUV in all cases, and the maximum value of tumor to liver ratio 84 hr after administration was 3.6 for the $^{89}$Zr-labeled antibody prepared using $^{89}$Zr-labeled DOTA-Bn-DBCO, and 3.4 for the $^{89}$Zr-labeled antibody prepared using $^{89}$Zr-labeled DOTAGA-DBCO. A statistically significant difference was not found in each value. It was confirmed that the accumulation of the $^{89}$Zr-labeled antibodies in the heart (blood) decreased with time, and that it almost disappeared from the blood 252 hr after administration. In addition, no statistically significant difference was found in accumulation in heart (blood) at each time point among the $^{89}$Zr-labeled antibodies. Similarly, it was confirmed that the accumulation of the $^{89}$Zr-labeled antibodies in the liver and muscle decreased with time, and no statistically significant difference was found in accumulation in each tissue at each time point among the $^{89}$Zr-labeled antibodies.

Example 11: Screening of humanized antibody using In-111($^{111}$In)-labeled antibody

Example 11-1: Preparation of $^{111}$In-labeled antibody

To find an anti-MUC5AC antibody with high tumor accumulation ability and high maximum tolerated dose, various antibodies were labeled with $^{111}$In, administered to tumor-bearing mice, and SPECT-CT imaging was performed. Cumulative radioactivity and absorbed dose were calculated and compared using the obtained images.

The antibody used were 4 types of anti-MUC5AC humanized antibodies prepared in Production Example 1 and 1 type of anti-MUC5AC chimeric antibody disclosed in Patent Literature 1, and they were labeled with $^{111}$In.

The amino acid sequences of the heavy chain variable region and the light chain variable region (SEQ ID NOs: 23 and 24, respectively) of the chimeric antibody disclosed in Patent Literature 1 are as follows.

The $^{111}$In labeling was performed on conjugates of a labeling precursor having a structure represented by the following formula and various antibodies.

[heavy chain variable region]

E V K L V E S G G V L V K S G G S L K L S C A V S G F T F S N Y G M S W V R
Q T P E K R L E W V A T I S N S G R Y T Y F P D S V K G P F A I S R D N A K
N N L Y L Q M S S L R S A D T A L Y Y C T R H L D Y A N Y D A M D Y W G Q G
T S V T V S S

[light chain variable region]

D I V L T Q S P A S L A V S L G Q R A T I S C R A S K S V T T S D F S Y M H
W Y Q Q K P G Q P P K L L L Y L A S N L E S G V P D R F S G S G S G Y D F Y
L N I H P V E E E D A A T Y Y C Q H S R E F P W T F G G G T K L E I K

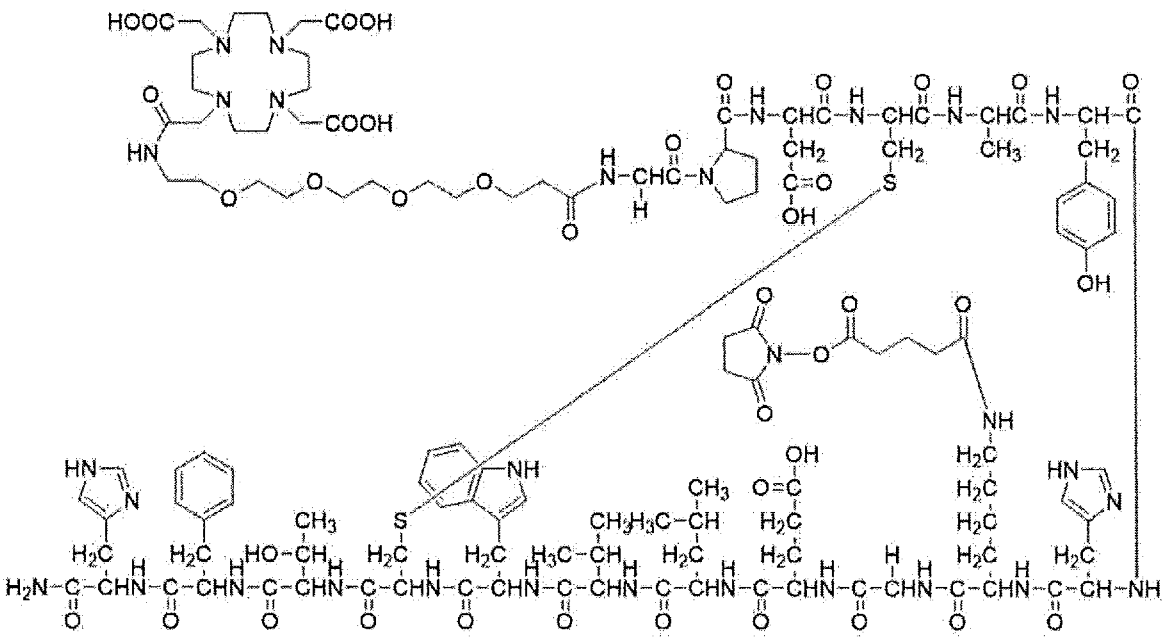

The above-mentioned labeling precursor had a structure in which DOTA as a chelate site is linked to the N-terminal of an antibody-modification peptide (peptide containing 17 amino acid residues having the same sequence as the sequence of SEQ ID NO: 10 in which Xaa2 is a lysine residue) via 8 polyethylene glycols, and linked to the 252nd lysine residue by EU numbering in various antibodies via an N-hydroxysuccinimide ester group in the structure.

This labeling precursor (450 μg) was dissolved in 100 mmol/L 4-(2-hydroxyethyl)-1-piperazine ethane sulfonic acid buffer (pH 5.5) as a solvent. This solution was mixed with a $^{111}$In ion-containing solution (indium ($^{111}$In) chloride Injection, manufactured by Nihon Medi-Physics Co., Ltd.) as a radioactive metal source at 10 MBq as a radioactive amount, and a labeling reaction was performed at 45°C for 2 hr.

The radiochemical yield, radiochemical purity, and radioactivity administered to animal of various $^{111}$In-labeled antibodies are shown in Table 13. The radiochemical yield here refers to the radioactivity of the $^{111}$In-labeled antibody with respect to the radioactivity of $^{111}$In used. For the radioactivity measurement, a radioisotope Doze Calibrator (manufactured by CAPINTEC, model number: CRC-15R) was used. The radiochemical purity refers to the ratio (%) of the peak radioactivity count corresponding to the $^{111}$In-labeled antibody to the total radioactivity count of the filter paper as analyzed by filter paper chromatography. For filter paper chromatography, filter: manufactured by ADVANTEC, model number: No. 590, developing solvent: 0.01% EDTA, 50 mM citric acid-sodium citrate aqueous solution was developed, and the radioactivity count was detected using a radio γ-TLC analyzer (manufactured by raytest, MODEL GITA Star).

[Table 13]

| $^{111}$In labeling information | | | | | |
|---|---|---|---|---|---|
| name of antibody | chimeric antibody | humanized antibody | | | |
| | | H01L03 | H01L04 | H02L04 | H04L04 |
| radiochemical yield (%) | 99.0 | 96.3 | 93.6 | 91.0 | 94.0 |
| radiochemical purity (%) | 100 | | | | |
| administered radioactivity amount (MBq) | 5.3±1.0 | 5.5±0.1 | 4.7±0.2 | 4.6±0.2 | 4.6±1.3 |
| (mean±standard deviation, n=4) | | | | | |

Example 11-2: Biodistribution using tumor-bearing mice

(Production method of tumor-bearing mouse)

Human pancreatic cancer cell line SW1990 ($0.7\times10^7$ cells) were subcutaneously administered to BALB/c-nu/nu mice (male, manufactured by Japan SLC Co., Ltd.) from the flank to the back thereof.

When the tumor size reached about 150-300 $mm^3$ 14 days after transplantation of SW1990, various $^{111}In$-labeled antibodies prepared in Example 10-1 were administered from the tail vein of the mice. The tumor volume was calculated from the following calculation formula.

$$\text{tumor volume} = ((\text{tumor minor axis})^2 \times \text{tumor major axis})/2$$

[Table 14]

| Animal information (on administration of each $^{111}In$-labeled antibody) | | | | | |
|---|---|---|---|---|---|
| name of antibody | chimeric antibody | humanized antibody | | | |
| | | H01L03 | H01L04 | H02L04 | H04L04 |
| tumor volume (mm3) | 203±110 | 201±45 | 285124 | 209±83 | 193±80 |
| body weight (g) | 23.2±1.3 | 23.4±0.6 | 23.5±0.7 | 22.1±1.7 | 21.2±1.8 |
| (mean±standard deviation, n=4) | | | | | |

(Evaluation method)

SPECT-CT imaging (small animal SPECT-CT apparatus: FX-3000, manufactured by Trifoil) was performed under the conditions in the following Table. The time points for imaging were 1, 6, 24, 48, 72, and 168 hr after administration. Image reconstruction was performed by the OSEM method for SPECT and the FBP method for CT. A VOI (volume of interest, three-dimensional ROI) analysis of the tumor and liver at each time point was performed. The count number per organ volume was corrected to %ID/g, the physical half-life was converted from $^{111}In$ to $^{225}Ac$, the difference in physical half-life was corrected, and the biological half-life added to obtain the time activity curve with. The cumulative radioactivity was calculated from the integrated value of this time activity curve, and the absorbed dose was calculated in a sphere model (OLINDA/EXM ver2.0), and this was compared and examined for each antibody. However, for H01L03, a decrease in the time activity curve was not observed 168 hr after administration. Thus, the biological half-life was not considered and only the physical half-life was added.

[Table 15]

| SPECT imaging conditions | |
|---|---|
| Isotope | Indium-111 medium +high energy |
| Collimator | MMP952 |
| Radius of Rotation | 50 mm |
| Projection Limit | 150 sec |
| Projection Count | 16 |
| Rotation Angle | 90 degree |

[Table 16]

| CT imaging conditions | |
|---|---|
| Projection count | 207 views |
| Frames averaged | 1 frames |
| Detector binding | 2 × 2 |
| X-ray tube current | 270 uA |
| X-ray tube voltage | 60 kV |
| Exposure time | 230 ms |

(continued)

| CT imaging conditions | |
|---|---|
| Magnification | 2.0 |

(Results)

The results of performing SPECT-CT imaging are shown in Fig. 11. The results of VOI analysis of the tumor and liver at each time point are shown in Fig. 12. Graphs showing the results of confirming the biodistribution and the excretion route after the completion of SPECT-CT imaging 168 hr after administration are shown in Fig. 13.

The accumulation in the tumor was highest when the humanized antibody (H01L03) was administered, and lowest when the chimeric antibody was administered. The accumulation in the liver was highest when chimeric antibody was administered, and lower when any humanized antibody was administered as compared to the chimeric antibody. When the humanized antibody (H01L03) was administered, the excretion was the slowest and the blood retention was high. Regardless of which antibody was administered, the accumulation in the liver and spleen was high among the normal organs, followed by the accumulation in the lung and kidney.

When the threshold dose of liver was assumed to be 30 Gy, the maximum tolerated dose was estimated to be high value of 3.90 MBq with a humanized antibody (H01L03) and low value of 0.43 MBq with a chimeric antibody. The maximum tolerated dose (MBq) was calculated by threshold dose (Gy)/absorbed dose (Gy/MBq).

Example 11-3: In vitro autoradiography

The results of images evaluating the binding property and specificity of various antibodies for MUC5AC by in vitro autoradiography (ARG) using various $^{111}$In-labeled antibodies prepared in Example 11-1 are shown in Fig. 14. In addition, graphs of the numerical values of the radioactivity density (Bq/mm$^2$) in the ROI calculated by setting the region of interest (ROI) in the entire section are shown in Fig. 15.

A section with a thickness of 10 μm was prepared using a cryostat (manufactured by Leica) from a frozen block obtained by freezing MUC5AC high expression tumor (SW1990 transplanted tumor tissue) or MUC5AC low expression tumor (MIAPaCa2 transplanted tumor tissue) in liquid nitrogen and used for in vitro ARG.

A section stored at -80°C was returned to room temperature, dried for 30 min or longer, immersed in phosphate buffered saline for 30 min, and then in phosphate buffered saline containing 1 % by volume bovine serum albumin for 30 min, whereby the section was hydrophilized.

The hydrophilized cryosection was immersed in 1% by volume bovine serum albumin-containing phosphate buffered saline containing 5 kBq/mL various $^{111}$In-labeled antibodies for 30 min each. Then, the section was washed by immersing for 5 min in each of 1% by volume bovine serum albumin-containing phosphate buffered saline, phosphate buffered saline, and phosphate buffered saline in this order. The section after washing was air dried, exposed in an imaging plate (BAS-SR2040, manufactured by FUJIFILM Wako Pure Chemical Corporation) for about 15 hr, and an autoradiogram was obtained using a fluoroimage analyzer (Typhoon FLA 7000, manufactured by GE Healthcare). Using the analysis software "Image Quant TL" attached to the fluoroimage analyzer, the obtained autoradiogram was used to set the ROI over the entire section, and the radioactivity density (Bq/mm$^2$) in the ROI was calculated.

It was confirmed that all $^{111}$In-labeled humanized antibodies retained binding property and specificity for MUC5AC (Fig. 15, data of MUC5AC high expression tumor). It was confirmed that various humanized antibodies had less nonspecific binding as compared with the chimeric antibody (Fig. 15, data of MUC5AC low, unexpressed tumor).

From the results of Examples 11-2 and 11-3, the humanized antibody has higher specificity for MUC5AC than the chimeric antibody, and has high accumulation in tumor and low accumulation in normal organs such as liver and the like. It was clarified that it provides a more superior delivery technique relating to RI-labeled antibodies.

The results of this Example are summarized in the following Table. In the Table, the absorbed dose of SPECT was calculated assuming the tumor volume of 150 mm$^3$. The absorbed dose of the liver was calculated based on the mean (1.15±0.14 g, n=19) of the weight of the mouse liver used in this Example. The numerical value of the biodistribution was expressed by the mean±standard deviation of n=4 (however, n=3 for H02L04).

[Table 17]

| | | name of antibody | | | | |
|---|---|---|---|---|---|---|
| | | chimeric antibody | humanized antibody | | | |
| | | | H01L03 | H01L04 | H02L04 | H04L04 |
| SPECT absorbed dose | tumor (Gy/MBq) | 9.5 | 35.0 | 15.3 | 13.7 | 15.0 |
| | liver (Gy/MBq) | 70.1 | 7.7 | 33.4 | 27.0 | 35.4 |
| bio-distribution at 168 hr after administration | tumor (%ID/g) | 11.6±0.9 | 24.2±3.9 | 12.9±2.6 | 15.6±6.2 | 17.2±4.2 |
| | liver (%ID/g) | 7.9±1.9 | 3.6±1.7 | 3.1±0.9 | 2.7±0.7 | 3.9±1.4 |
| | spleen (%ID/g) | 5.0±1.8 | 5.4±0.8 | 3.8±1.2 | 4.0±3.0 | 3.4±0.3 |
| | kidney (%ID/g) | 1.8±0.3 | 2.5±0.4 | 1.1±0.3 | 1.4±1.0 | 1.6±0.2 |
| | blood (%ID/g) | 2.2±0.6 | 5.3±1.3 | 2.1±0.8 | 2.6±2.3 | 3.8±0.7 |

While the present invention has been described above with reference to the embodiments, the present invention is not limited to the above-mentioned embodiments. Various changes that can be understood by those skilled in the art can be made to the constitution and details of the present invention within the scope of the present invention.

The contents disclosed in any publication cited herein, including patents and patent applications, are hereby incorporated in their entireties by reference, to the extent that they have been disclosed herein.

[Industrial Applicability]

The RI-labeled anti-MUC5AC humanized antibody of the present invention is superior in specificity and accumulation in tumor. Therefore, it is extremely useful for the treatment and/or diagnosis of diseases in which MUC5AC is overexpressed, particularly cancer.

This application is based on a patent application No. 2021-072201 filed in Japan (filing date: April 21, 2021), the contents of which are incorporated in full herein.

## Claims

**1.** A conjugate of a radionuclide and an antibody, wherein the radionuclide is a nuclide that emits β-rays and the antibody is a humanized antibody that specifically binds to mucin subtype 5AC and that comprises
a heavy chain variable region consisting of

(1) the amino acid sequence shown in SEQ ID NO: 1 (H01), or an amino acid sequence having not less than 95% sequence identity with the amino acid sequence shown in SEQ ID NO: 1;
(2) the amino acid sequence shown in SEQ ID NO: 2 (H02), or an amino acid sequence having not less than 95% sequence identity with the amino acid sequence shown in SEQ ID NO: 2;
(3) the amino acid sequence shown in SEQ ID NO: 3 (H03), or an amino acid sequence having not less than 95% sequence identity with the amino acid sequence shown in SEQ ID NO: 3; or
(4) the amino acid sequence shown in SEQ ID NO: 4 (H04), or an amino acid sequence having not less than 95% sequence identity with the amino acid sequence shown in SEQ ID NO: 4; and a light chain variable region consisting of
(5) the amino acid sequence shown in SEQ ID NO: 5 (L01), or an amino acid sequence having not less than 95% sequence identity with the amino acid sequence shown in SEQ ID NO: 5;
(6) the amino acid sequence shown in SEQ ID NO: 6 (L02), or an amino acid sequence having not less than 95% sequence identity with the amino acid sequence shown in SEQ ID NO: 6;
(7) the amino acid sequence shown in SEQ ID NO: 7 (L03), or an amino acid sequence having not less than

95% sequence identity with the amino acid sequence shown in SEQ ID NO: 7; or
(8) the amino acid sequence shown in SEQ ID NO: 8 (L04), or an amino acid sequence having not less than 95% sequence identity with the amino acid sequence shown in SEQ ID NO: 8.

**2.** The conjugate according to claim 1, wherein the antibody is a humanized antibody having a heavy chain variable region consisting of

(1) the amino acid sequence shown in SEQ ID NO: 1 (H01),
(2) the amino acid sequence shown in SEQ ID NO: 2 (H02),
(3) the amino acid sequence shown in SEQ ID NO: 3 (H03), or
(4) the amino acid sequence shown in SEQ ID NO: 4 (H04), and a light chain variable region consisting of
(5) the amino acid sequence shown in SEQ ID NO: 5 (L01),
(6) the amino acid sequence shown in SEQ ID NO: 6 (L02),
(7) the amino acid sequence shown in SEQ ID NO: 7 (L03), or
(8) the amino acid sequence shown in SEQ ID NO: 8 (L04).

**3.** The conjugate according to claim 1, wherein the antibody is a humanized antibody having

a heavy chain variable region consisting of

(1) the amino acid sequence shown in SEQ ID NO: 1 (H01), and

a light chain variable region consisting of
(7) the amino acid sequence shown in SEQ ID NO: 7 (L03).

**4.** The conjugate according to claim 1, wherein the nuclide that emits β-rays is iodine-131, yttrium-90, or lutetium-177.

**5.** The conjugate according to claim 1, wherein the conjugate comprises a chelating agent chelated with a radionuclide, and the radionuclide is a metal nuclide that emits β-rays.

**6.** The conjugate according to claim 5, wherein the metal nuclide that emits β-rays is yttrium-90.

**7.** The conjugate according to claim 5, wherein the metal nuclide that emits β-rays is lutetium-177.

**8.** The conjugate according to claim 5, comprising not less than 1 molecule and not more than 8 molecules of the chelating agent per 1 molecule of the antibody.

**9.** The conjugate according to claim 5, wherein the chelating agent site-specifically modifies an Fc region of the antibody via a linker.

**10.** The conjugate according to claim 9, wherein the linker comprises a peptide represented by the following formula (i) which consists of not less than 13 and not more than 17 amino acid residues, and which is formed by a crosslinking reaction of the peptide modified by a crosslinking agent and the antibody:

(Xa)-Xaa1-(Xb)-Xaa2-(Xc)-Xaa3-(Xd)··· (i)

wherein Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or
one is an amino acid residue derived from an amino acid having a thiol group in the side chain and the other is an amino acid residue derived from an amino acid having a haloacetyl group in the side chain, and Xaa1 and Xaa3 are linked, and
Xaa2 is a lysine residue, an arginine residue, a cysteine residue, an aspartic acid residue, a glutamic acid residue, 2-aminosuberic acid, or diamino propionic acid, and modified with the crosslinking agent.

**11.** The conjugate according to claim 5, wherein the chelating agent has a structure derived from a compound represented by the following formula (A) or a salt thereof:

(A)

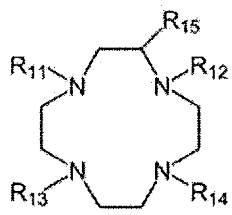

wherein in the formula (A), $R_{11}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the antibody, p is an integer of not less than 0 and not more than 3, $R_{15}$ is a hydrogen atom when $R_{12}$ is a substituent for conjugating with the antibody, and $R_{15}$ is a substituent for conjugating with the antibody when $R_{12}$ is not a substituent for conjugating with the antibody.

**12.** A radiopharmaceutical comprising the conjugate according to any one of claims 1 to 11 as the active ingredient.

**13.** The radiopharmaceutical according to claim 12, that is used for RI internal therapy for cancer.

**14.** The radiopharmaceutical according to claim 13, that is administered to a subject at a dose of not more than 100 MBq/kg one time in the RI internal therapy, wherein the radionuclide is iodine-131.

**15.** The radiopharmaceutical according to claim 13, that is administered to a subject at a dose of not more than 50 MBq/kg one time in the RI internal therapy, wherein the radionuclide is yttrium-90.

**16.** The radiopharmaceutical according to claim 13, that is administered to a subject at a dose of not more than 50 MBq/kg one time in the RI internal therapy, wherein the radionuclide is lutetium-177.

[Fig. 1]

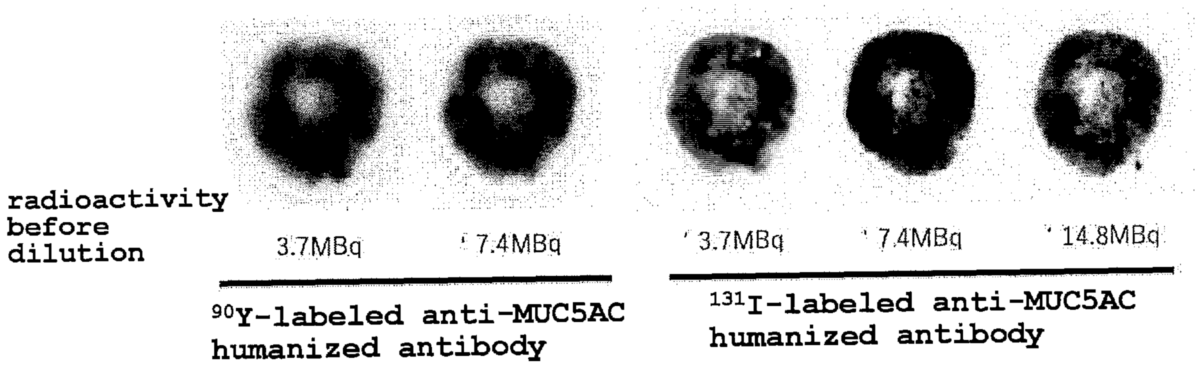
radioactivity before dilution
3.7MBq
7.4MBq
3.7MBq
7.4MBq
14.8MBq
$^{90}$Y-labeled anti-MUC5AC humanized antibody
$^{131}$I-labeled anti-MUC5AC humanized antibody

[Fig. 2]

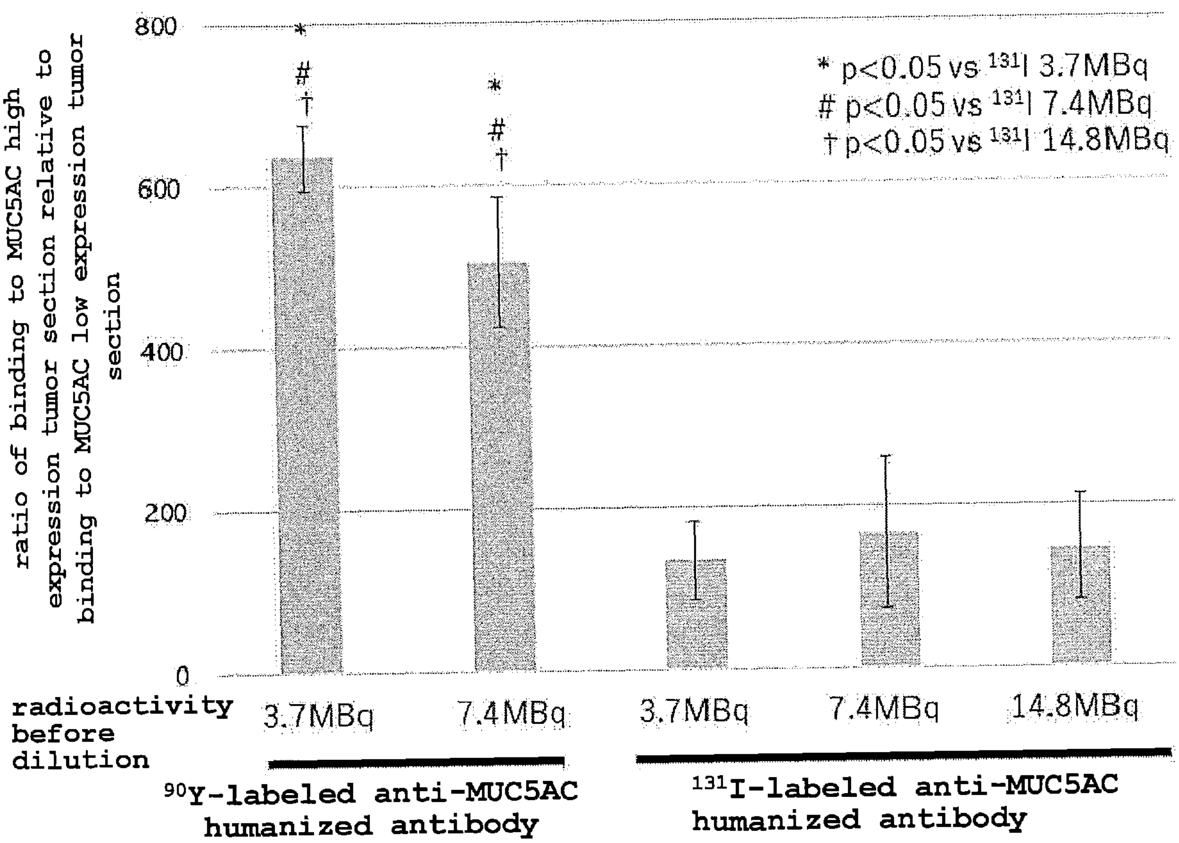
ratio of binding to MUC5AC high expression tumor section relative to binding to MUC5AC low expression tumor section
800
600
400
200
0
* p<0.05 vs $^{131}$I 3.7MBq
# p<0.05 vs $^{131}$I 7.4MBq
† p<0.05 vs $^{131}$I 14.8MBq
radioactivity before dilution
3.7MBq
7.4MBq
3.7MBq
7.4MBq
14.8MBq
$^{90}$Y-labeled anti-MUC5AC humanized antibody
$^{131}$I-labeled anti-MUC5AC humanized antibody

[Fig. 3]

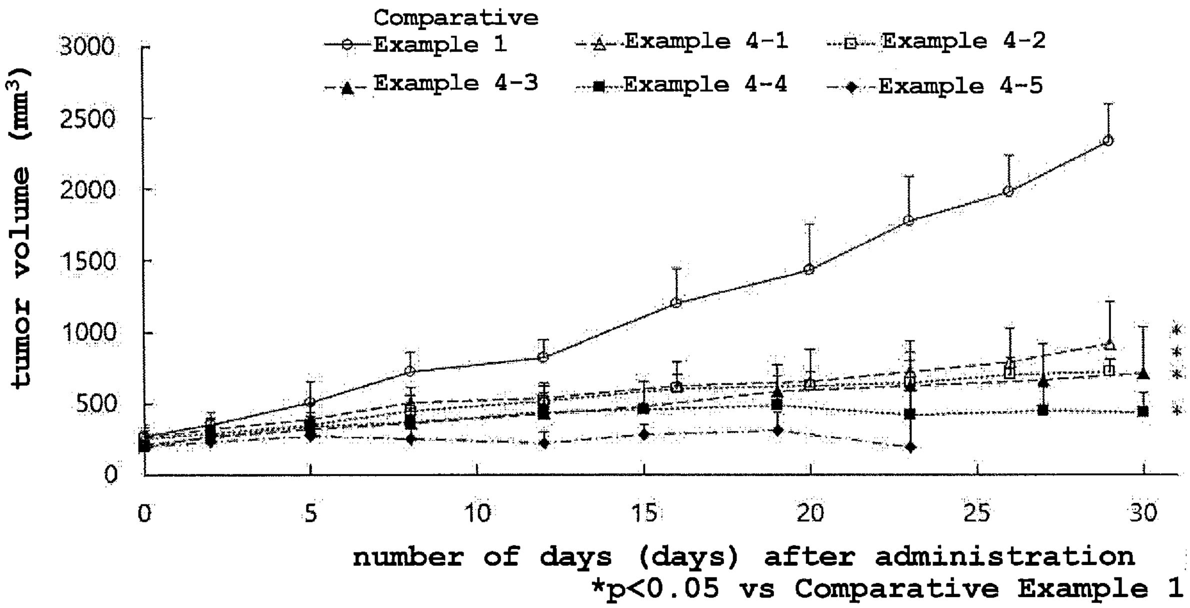
Comparative Example 1
Example 4-1
Example 4-2
Example 4-3
Example 4-4
Example 4-5
tumor volume (mm3)
3000
2500
2000
1500
1000
500
0
*
*
*
*
0
5
10
15
20
25
30
number of days (days) after administration
*p<0.05 vs Comparative Example 1

[Fig. 4]

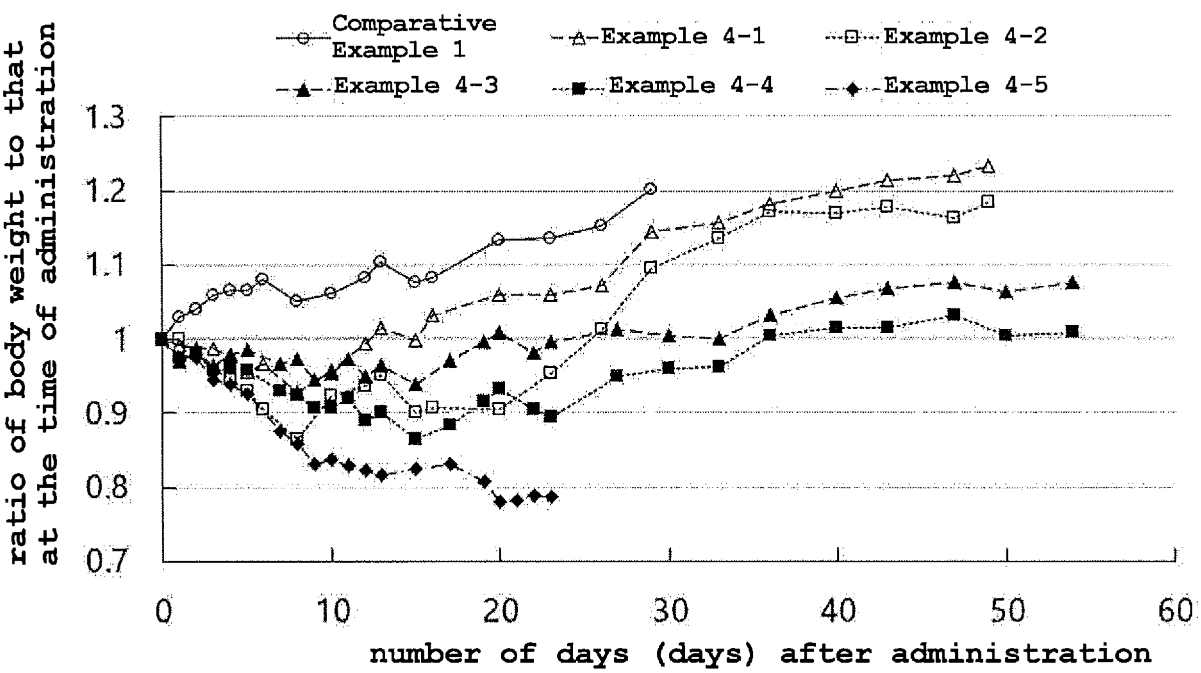
Comparative Example 1
Example 4-1
Example 4-2
Example 4-3
Example 4-4
Example 4-5
ratio of body weight to that at the time of administration
1.3
1.2
1.1
1
0.9
0.8
0.7
0
10
20
30
40
50
60
number of days (days) after administration

[Fig. 5]

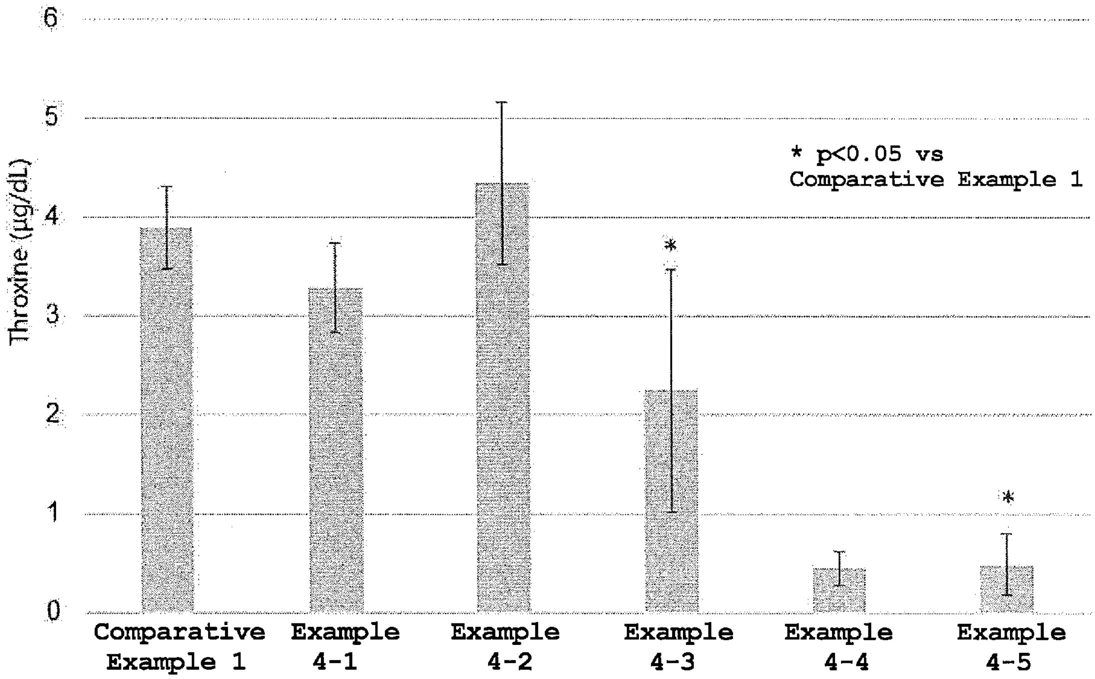
Throxine (μg/dL)
6
5
4
3
2
1
0
* p<0.05 vs
Comparative Example 1
*
*
Comparative Example 1
Example 4-1
Example 4-2
Example 4-3
Example 4-4
Example 4-5

[Fig. 6]

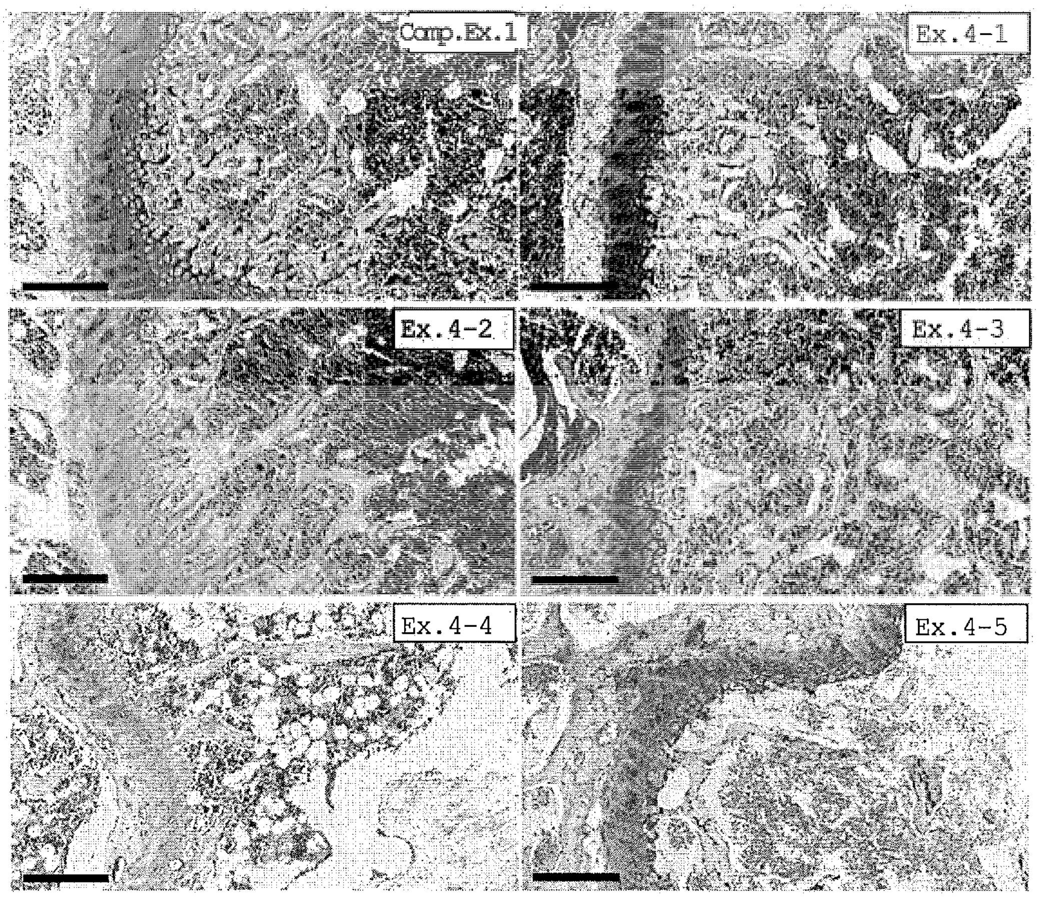
Comp.Ex.1
Ex.4-1
Ex.4-2
Ex.4-3
Ex.4-4
Ex.4-5

[Fig. 7]

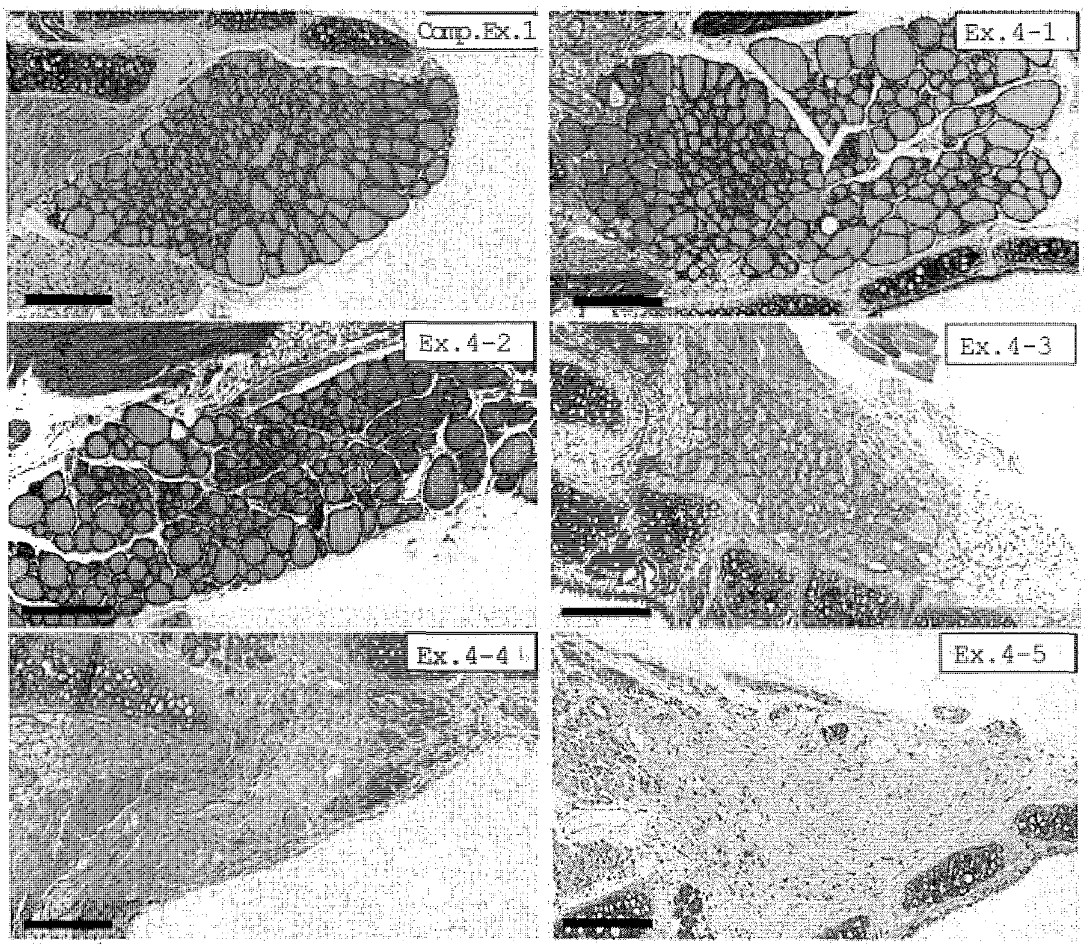
Comp.Ex.1
Ex.4-1
Ex.4-2
Ex.4-3
Ex.4-4
Ex.4-5

[Fig. 8]

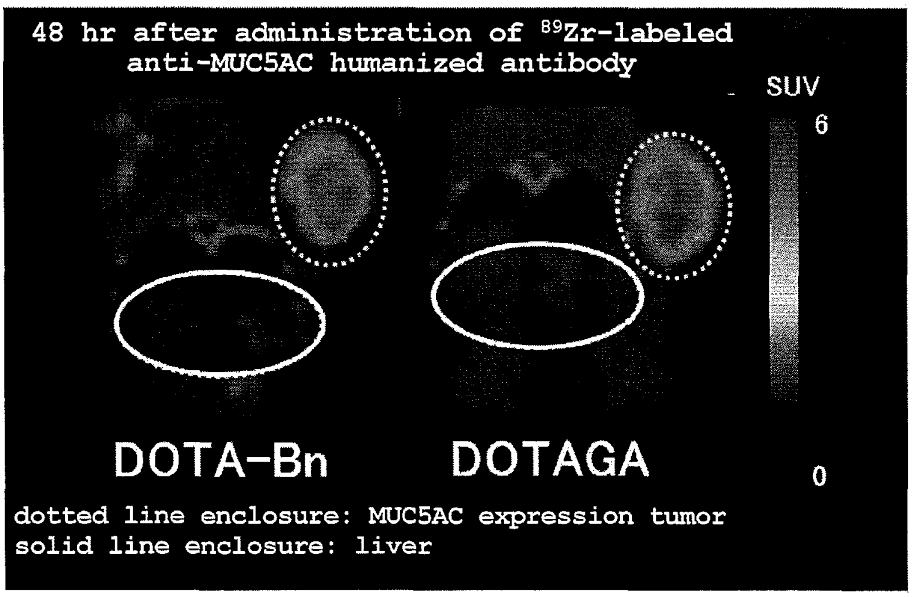
48 hr after administration of 89Zr-labeled anti-MUC5AC humanized antibody
SUV
6
0
DOTA-Bn
DOTAGA
dotted line enclosure: MUC5AC expression tumor
solid line enclosure: liver

[Fig. 9]

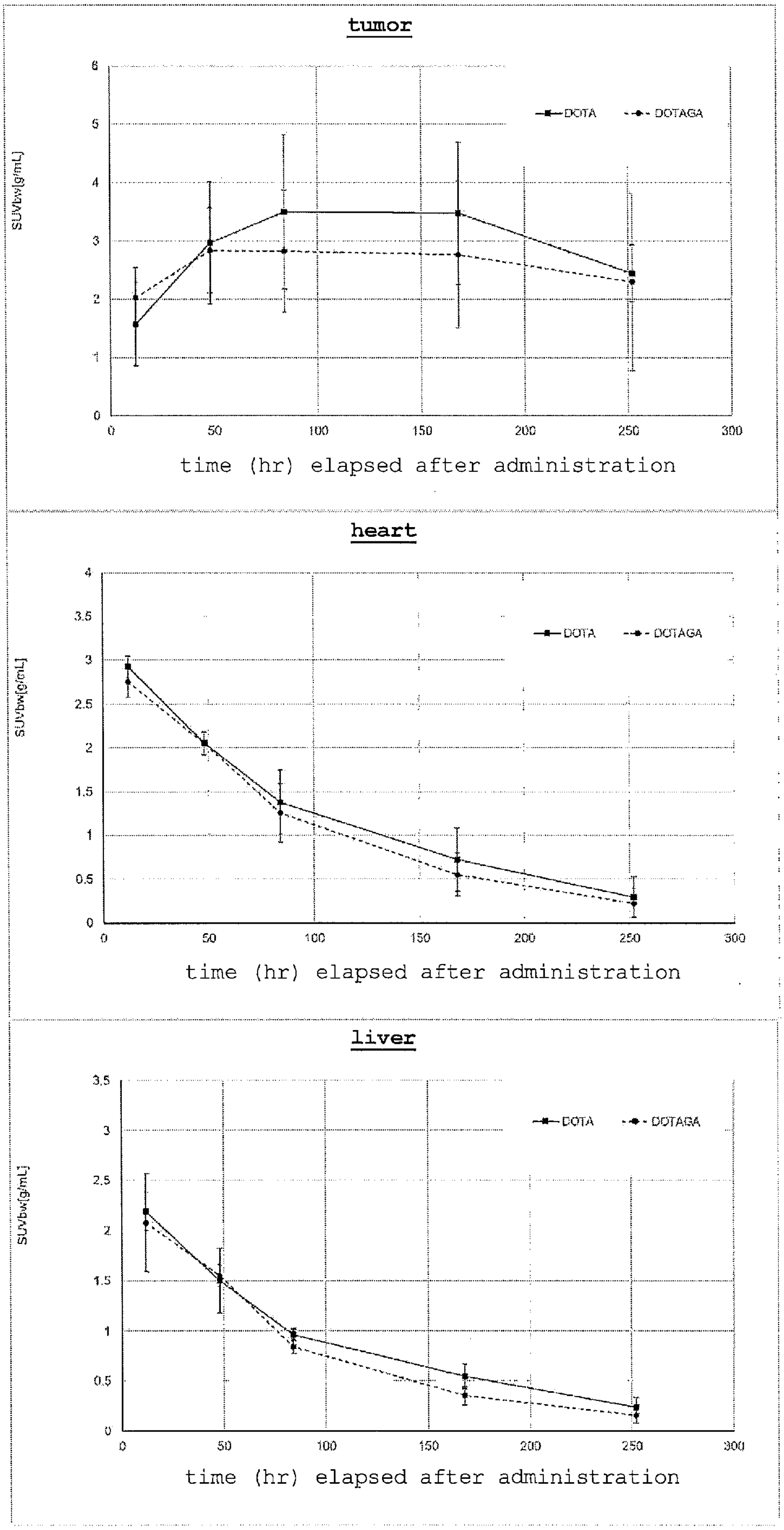
tumor
SUVbw[g/mL]
DOTA
DOTAGA
time (hr) elapsed after administration
heart
SUVbw[g/mL]
DOTA
DOTAGA
time (hr) elapsed after administration
liver
SUVbw[g/mL]
DOTA
DOTAGA
time (hr) elapsed after administration

[Fig. 10]

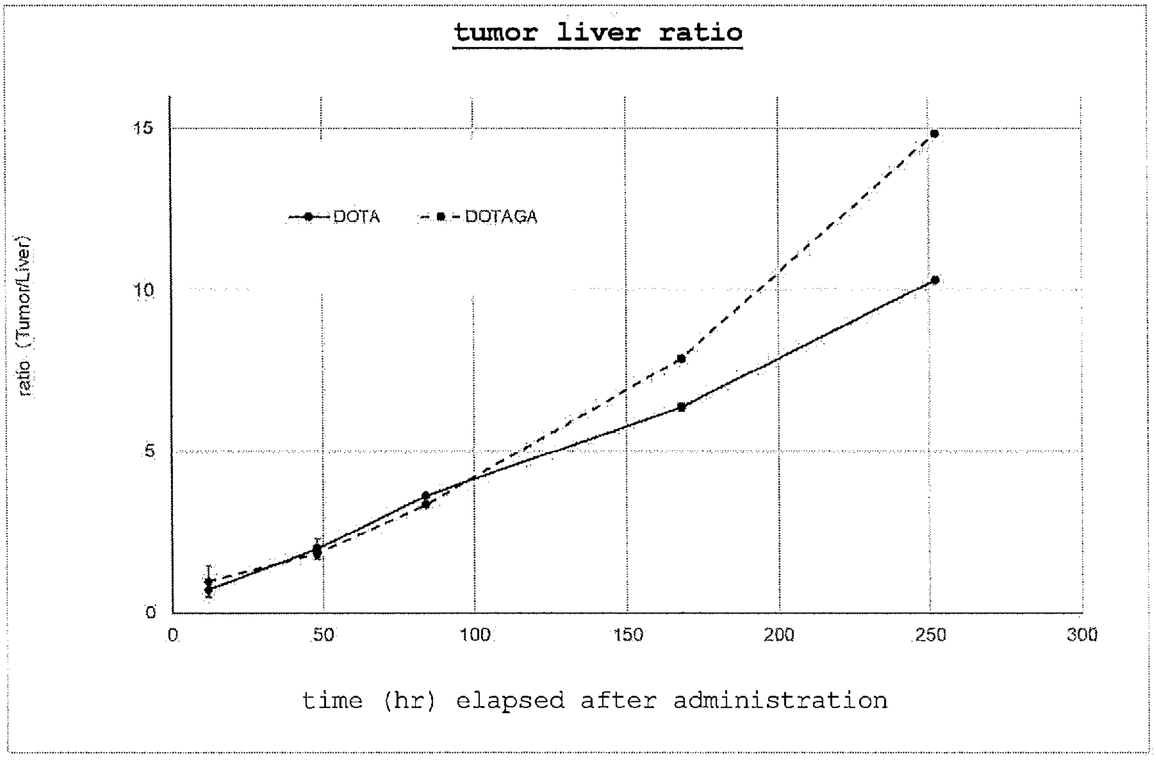
tumor liver ratio
ratio (Tumor/Liver)
15
10
5
0
DOTA
DOTAGA
0
50
100
150
200
250
300
time (hr) elapsed after administration

[Fig. 11]

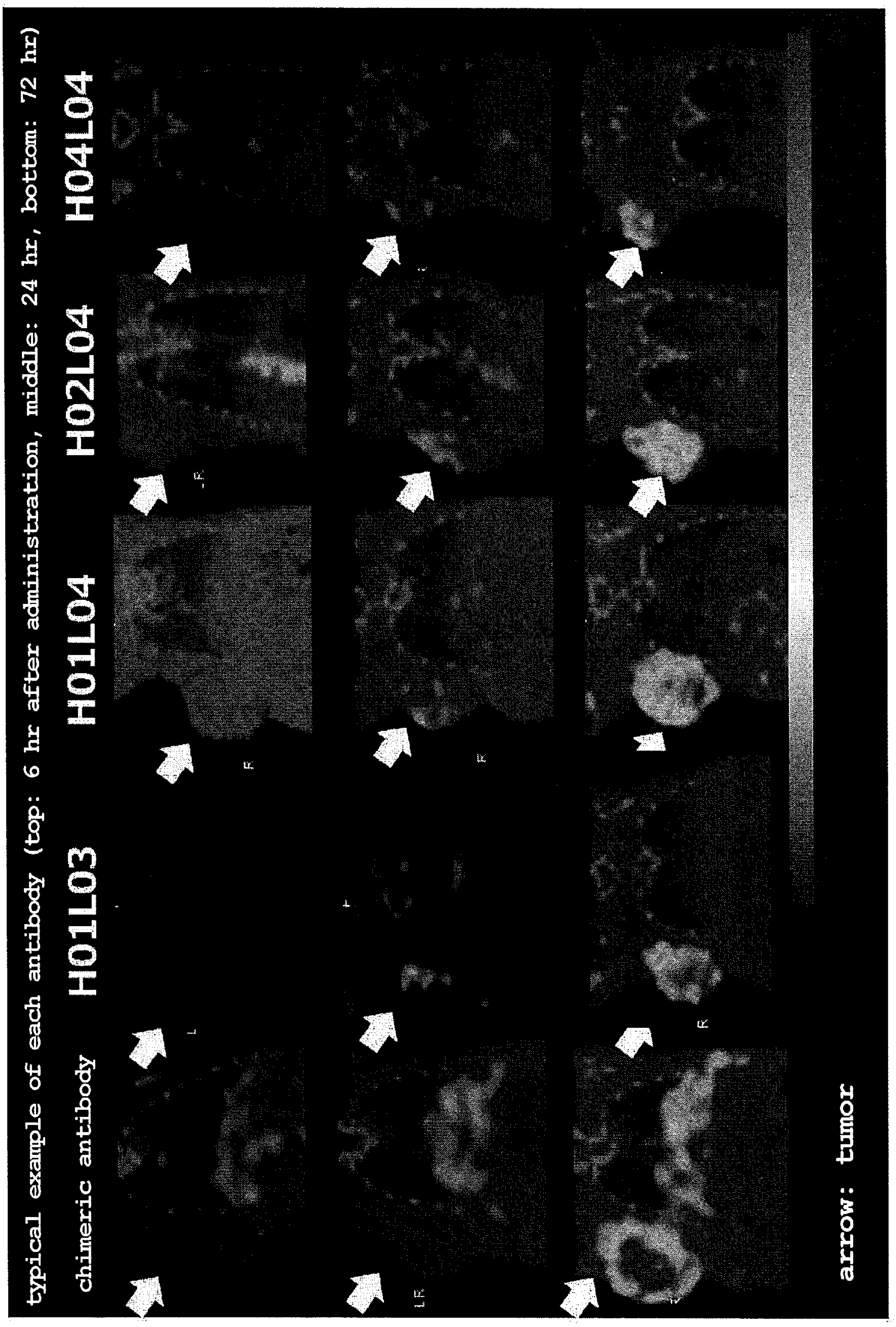
typical example of each antibody (top: 6 hr after administration, middle: 24 hr, bottom: 72 hr)
chimeric antibody
H01L03
H01L04
H02L04
H04L04
arrow: tumor

[Fig. 12]

(tumor)

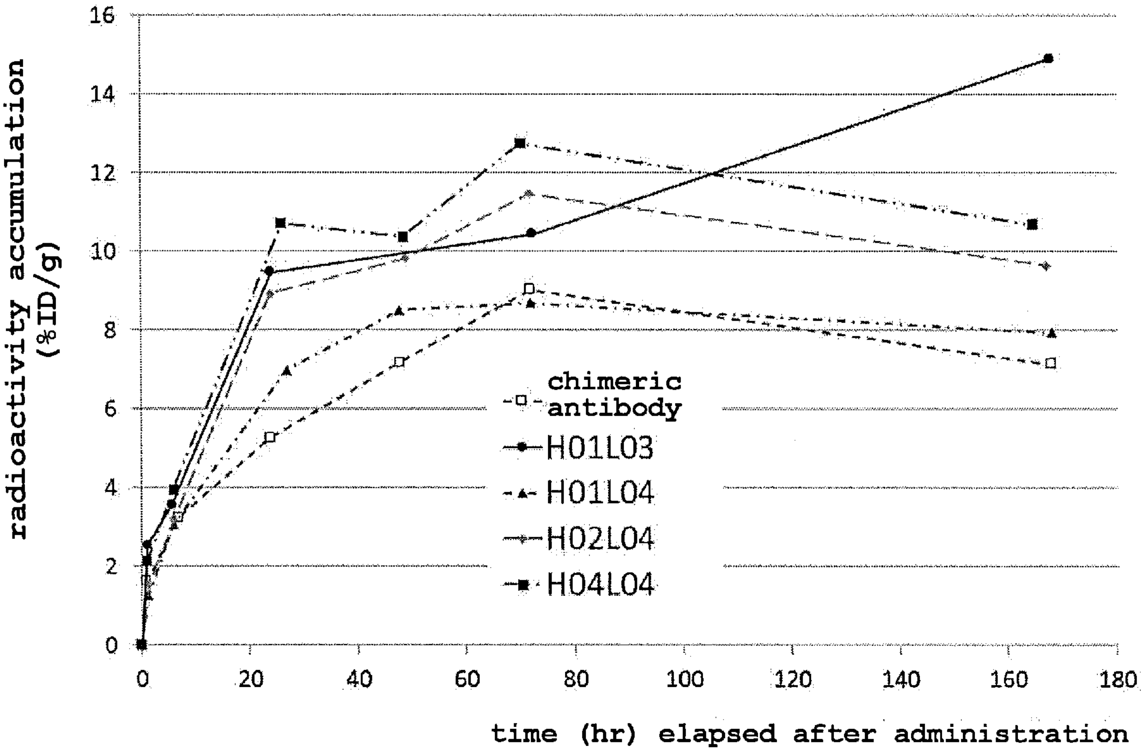


(liver)

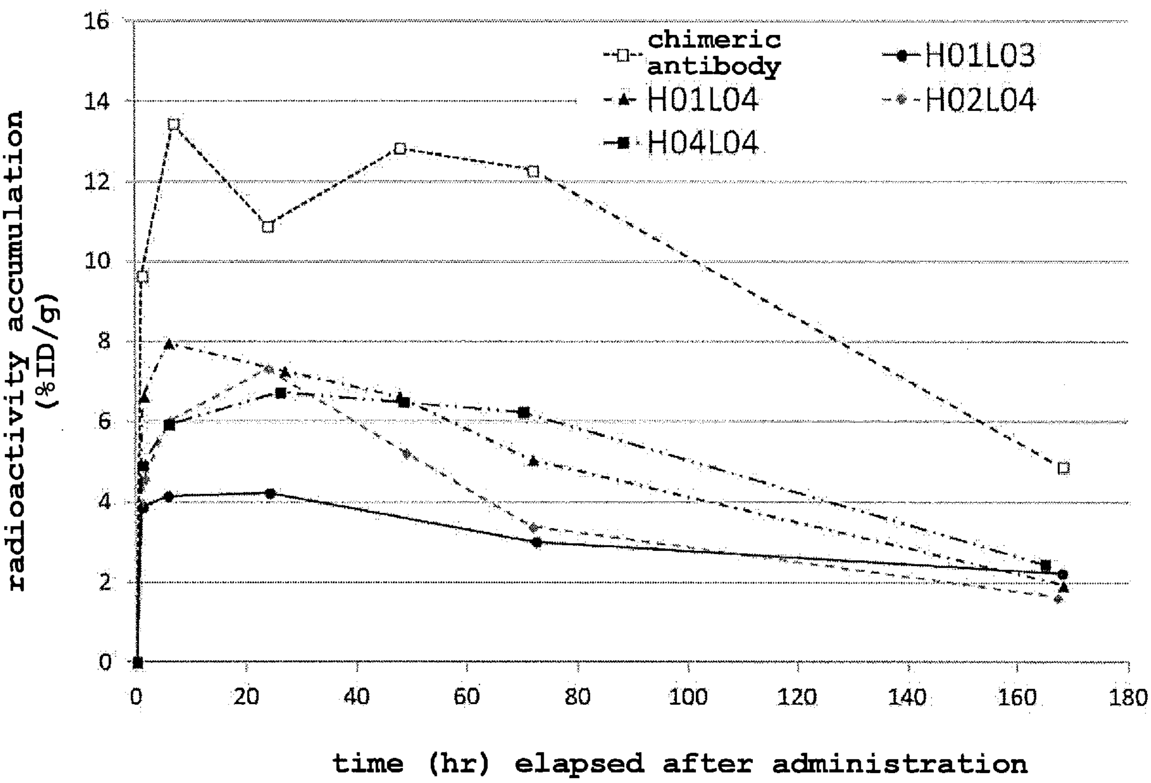

[Fig. 13]

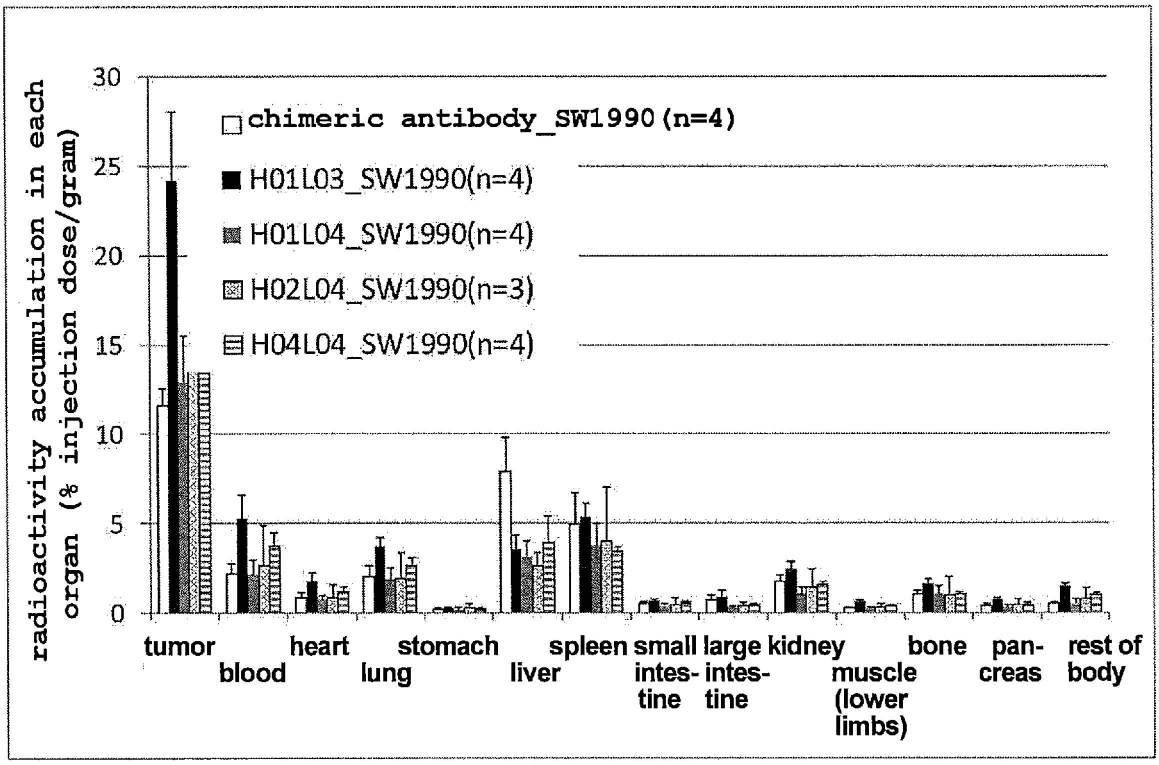
radioactivity accumulation in each organ (% injection dose/gram)
30
25
20
15
10
5
0
chimeric antibody_SW1990 (n=4)
H01L03_SW1990(n=4)
H01L04_SW1990(n=4)
H02L04_SW1990(n=3)
H04L04_SW1990(n=4)
tumor
blood
heart
lung
stomach
liver
spleen
small intes-tine
large intes-tine
kidney
muscle (lower limbs)
bone
pan-creas
rest of body

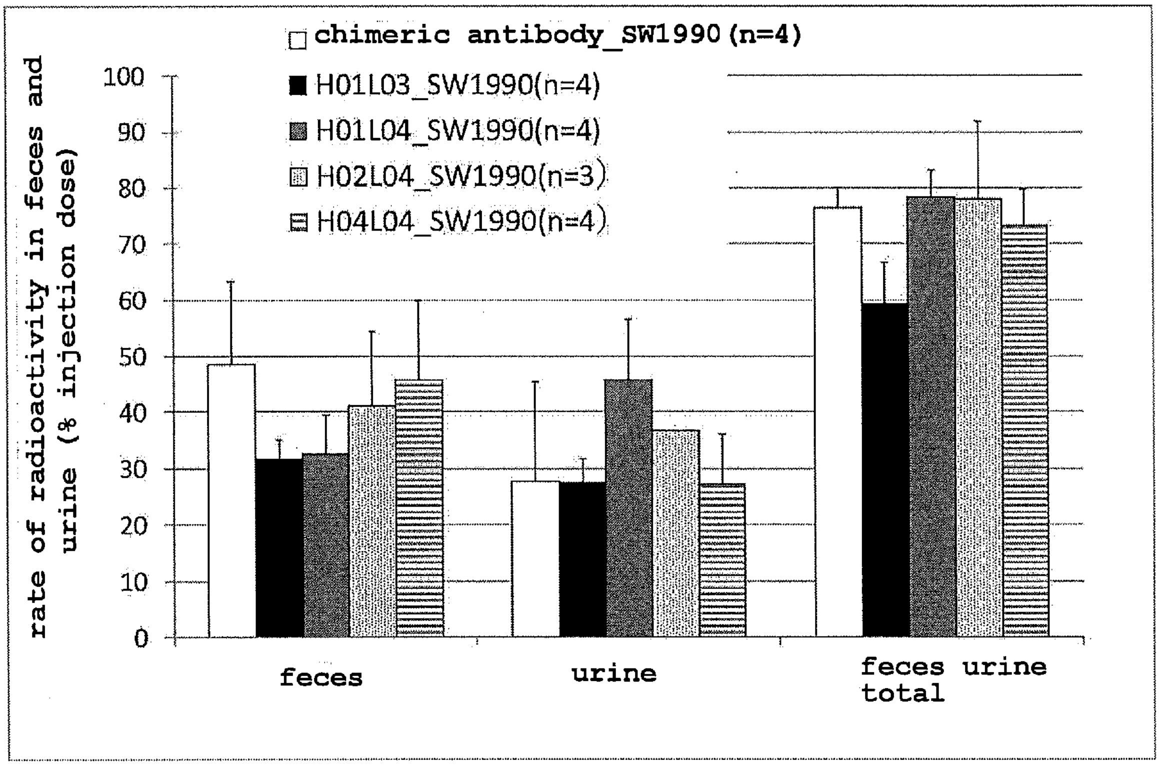
rate of radioactivity in feces and urine (% injection dose)
100
90
80
70
60
50
40
30
20
10
0
chimeric antibody_SW1990 (n=4)
H01L03_SW1990(n=4)
H01L04_SW1990(n=4)
H02L04_SW1990(n=3)
H04L04_SW1990(n=4)
feces
urine
feces urine total

[Fig. 14]

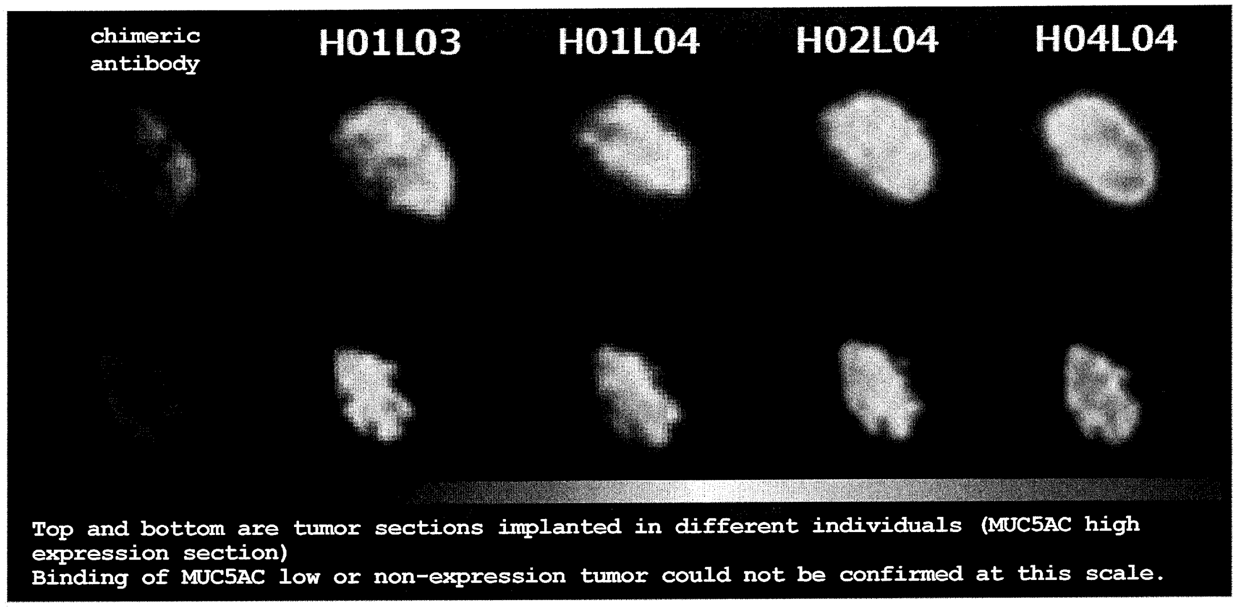
chimeric antibody
H01L03
H01L04
H02L04
H04L04
Top and bottom are tumor sections implanted in different individuals (MUC5AC high expression section)
Binding of MUC5AC low or non-expression tumor could not be confirmed at this scale.

[Fig. 15]

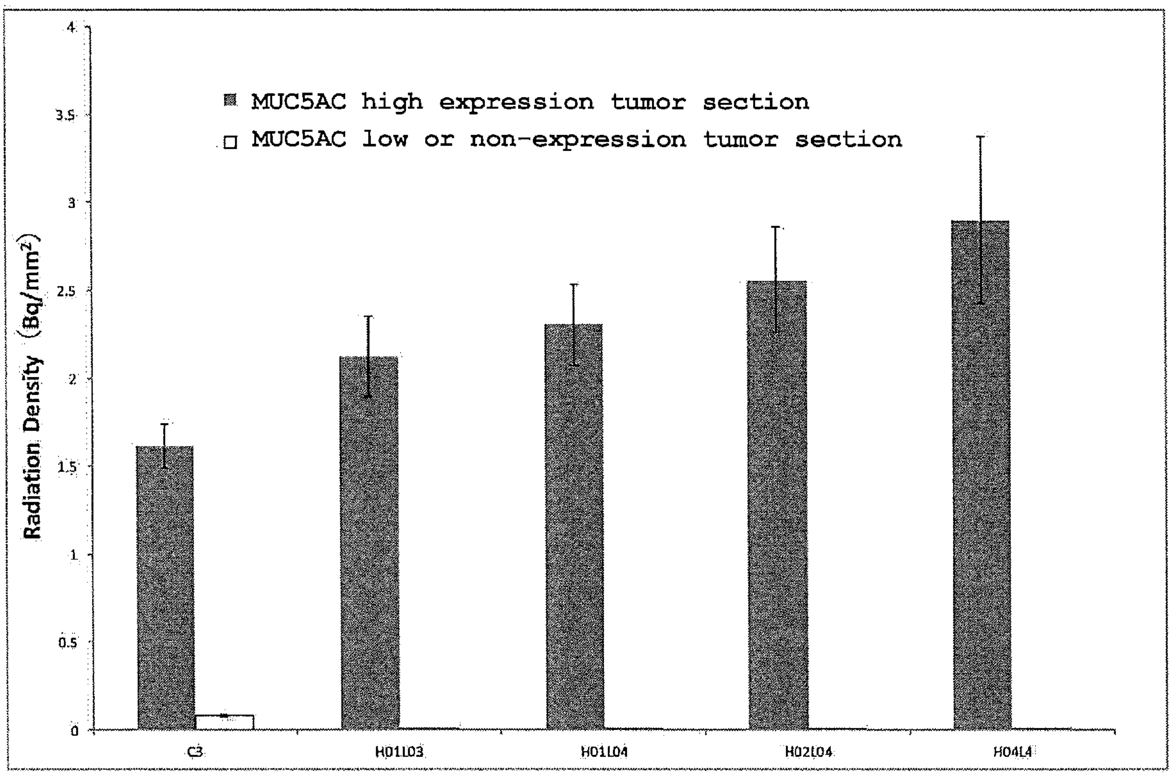
MUC5AC high expression tumor section
MUC5AC low or non-expression tumor section
Radiation Density (Bq/mm²)
4
3.5
3
2.5
2
1.5
1
0.5
0
C3
H01L03
H01L04
H02L04
H04L4

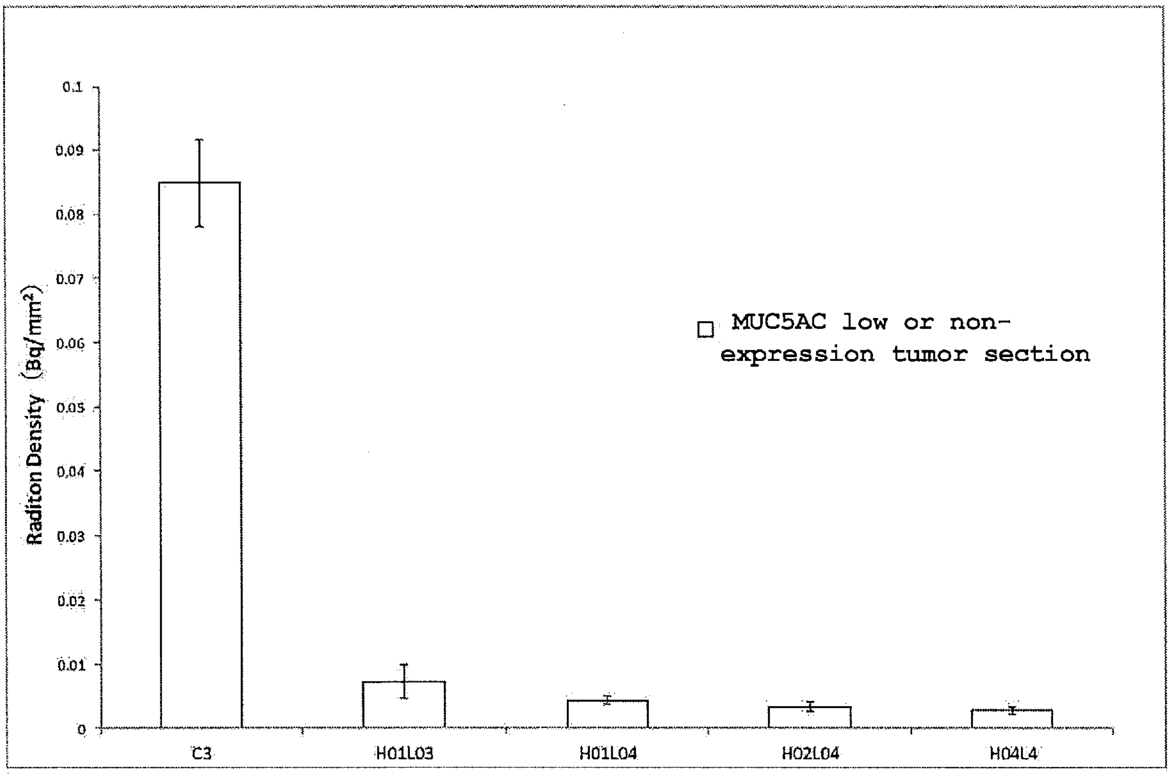
MUC5AC low or non-expression tumor section
Raditon Density (Bq/mm²)
0.1
0.09
0.08
0.07
0.06
0.05
0.04
0.03
0.02
0.01
0
C3
H01L03
H01L04
H02L04
H04L4

[Fig. 16]

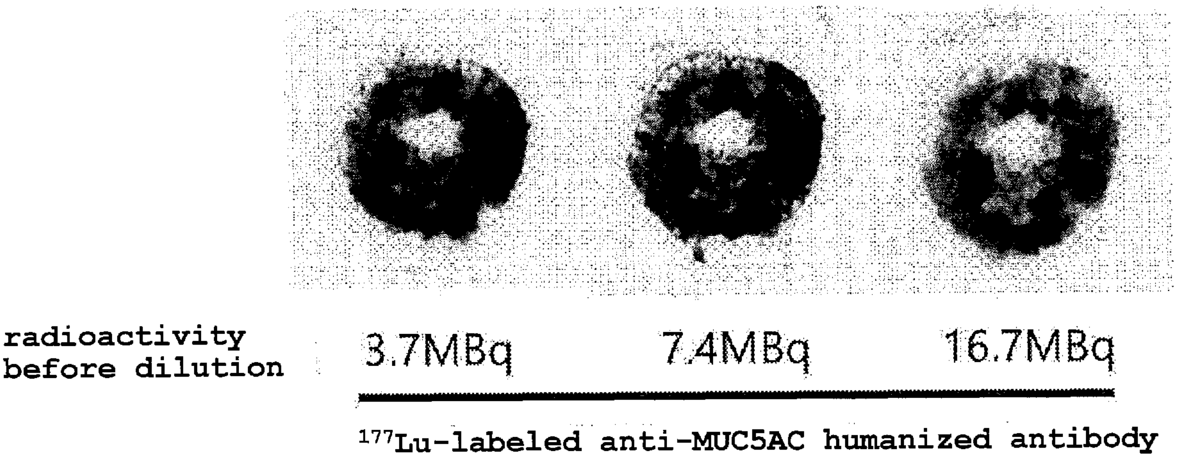
radioactivity before dilution
3.7MBq
7.4MBq
16.7MBq
$^{177}$Lu-labeled anti-MUC5AC humanized antibody

[Fig. 17]

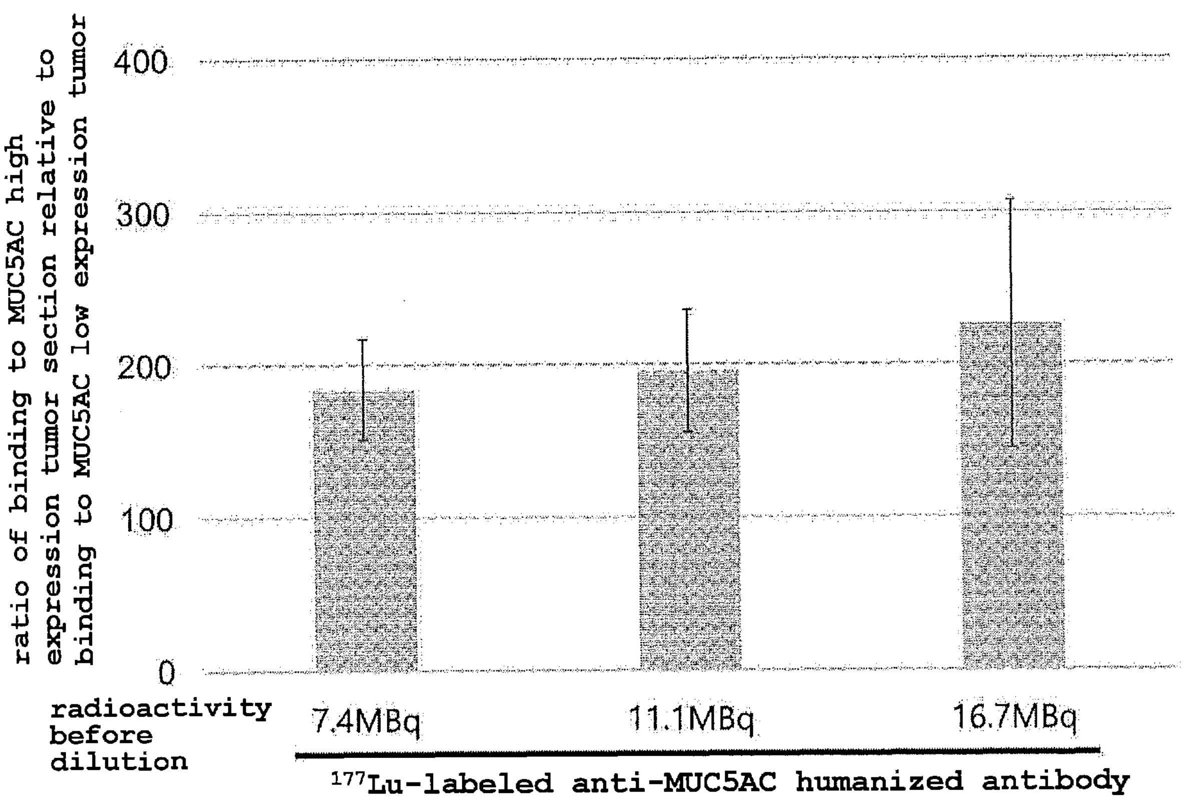
ratio of binding to MUC5AC high expression tumor section relative to binding to MUC5AC low expression tumor
400
300
200
100
0
radioactivity before dilution
7.4MBq
11.1MBq
16.7MBq
$^{177}$Lu-labeled anti-MUC5AC humanized antibody

[Fig. 18]

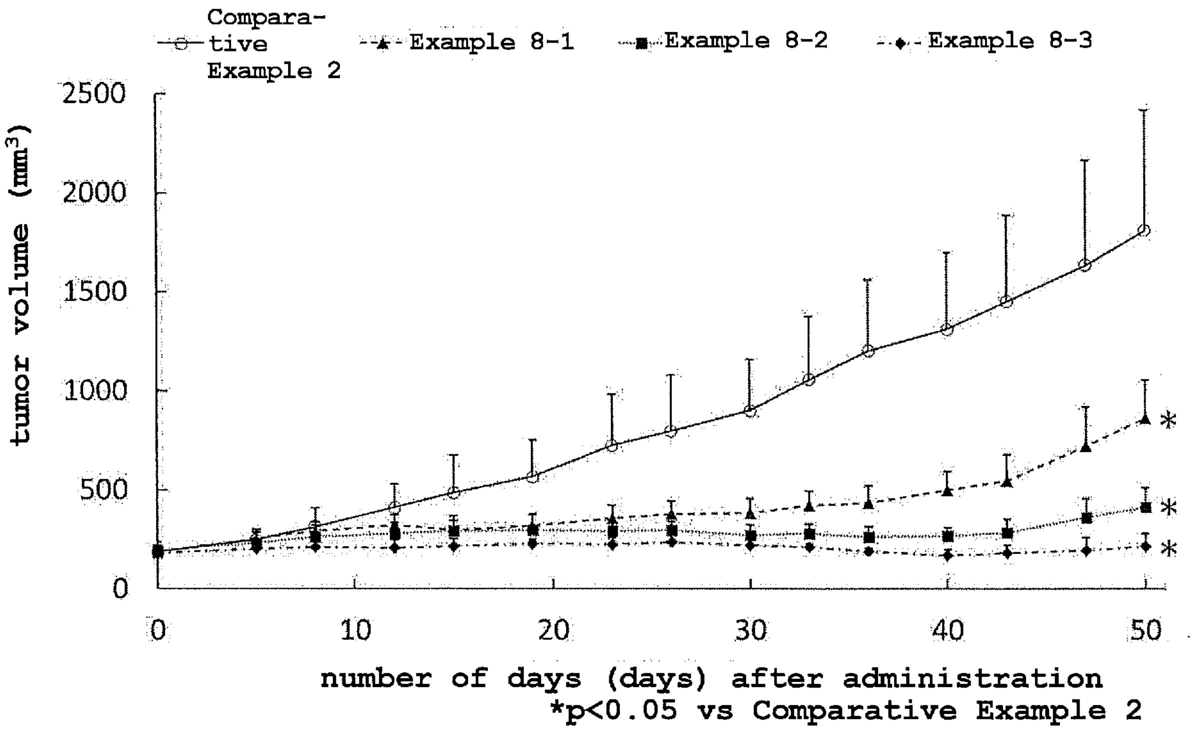
Comparative Example 2
Example 8-1
Example 8-2
Example 8-3
tumor volume (mm3)
2500
2000
1500
1000
500
0
10
20
30
40
50
*
number of days (days) after administration
*p<0.05 vs Comparative Example 2

[Fig. 19]

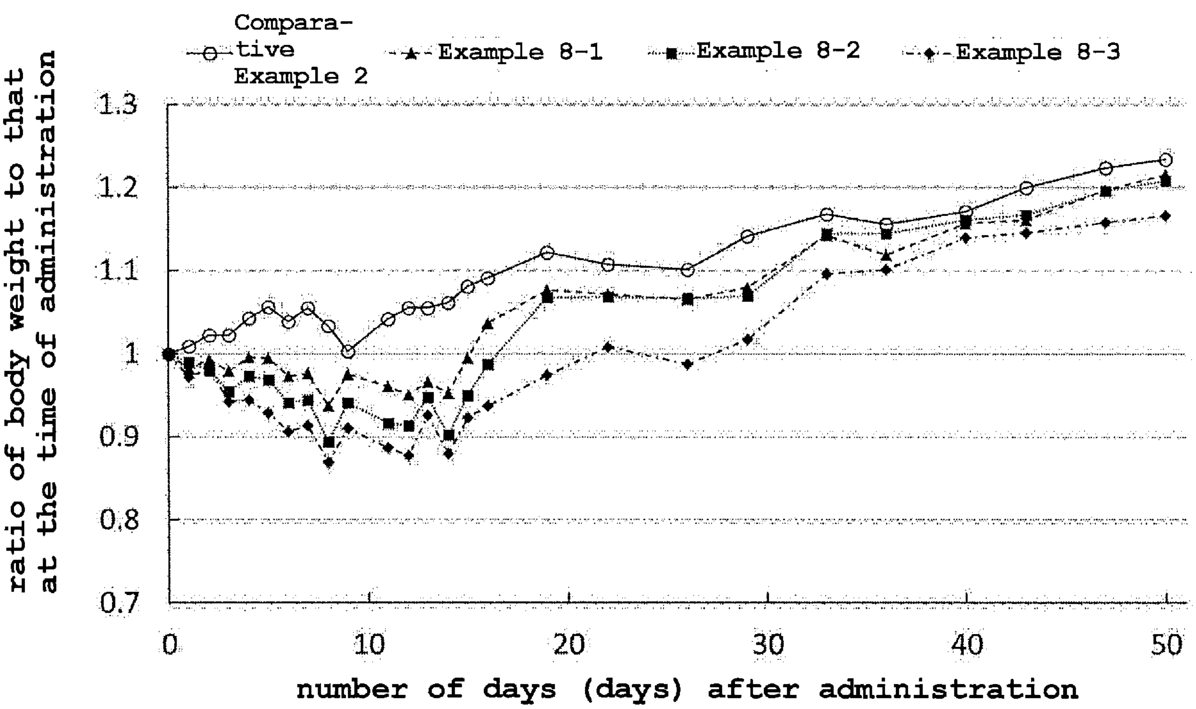
Comparative Example 2
Example 8-1
Example 8-2
Example 8-3
ratio of body weight to that at the time of administration
1.3
1.2
1.1
1
0.9
0.8
0.7
0
10
20
30
40
50
number of days (days) after administration

[Fig. 20]

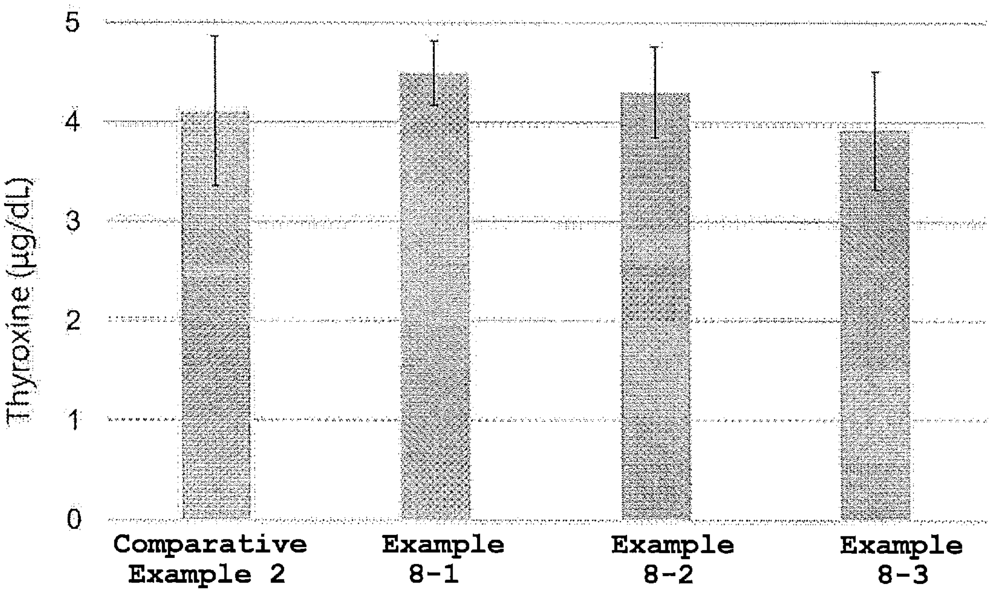
Thyroxine (μg/dL)
5
4
3
2
1
0
Comparative Example 2
Example 8-1
Example 8-2
Example 8-3

[Fig. 21]

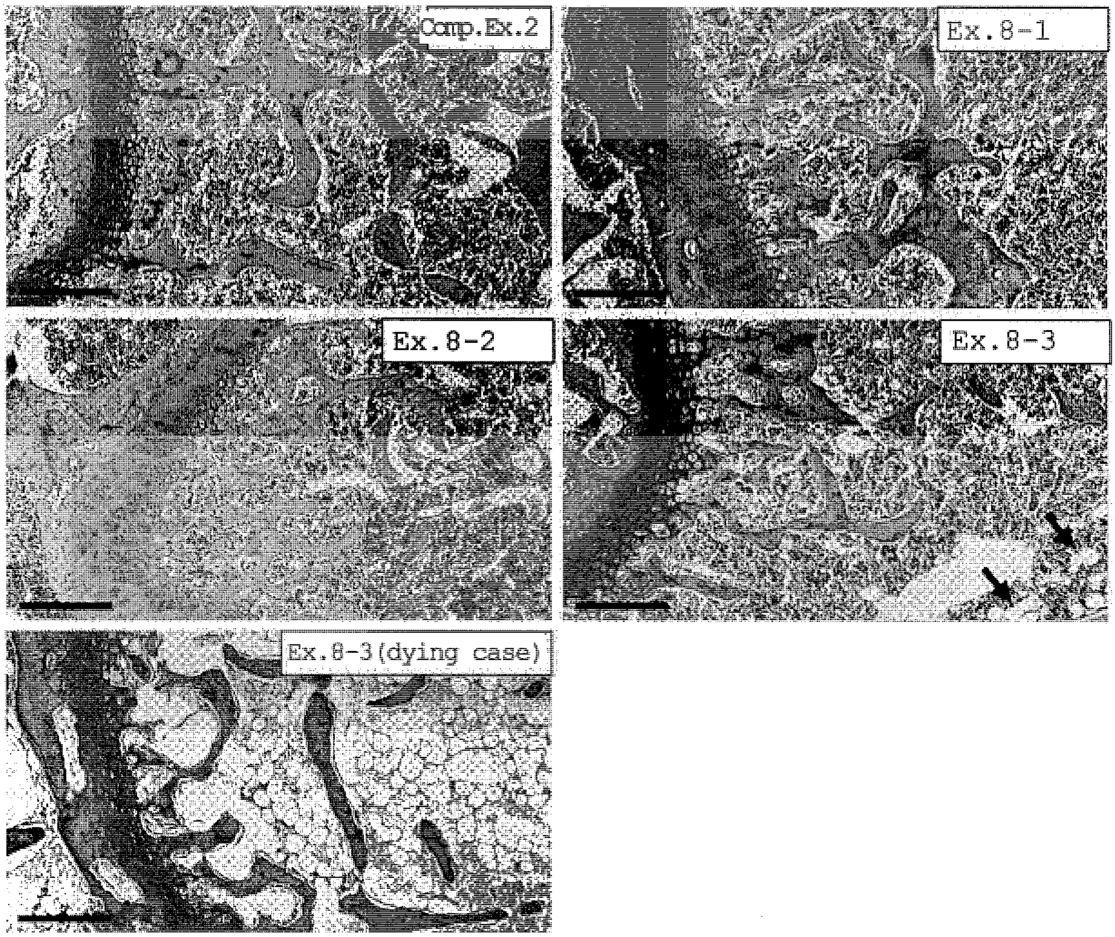
Comp.Ex.2
Ex.8-1
Ex.8-2
Ex.8-3
Ex.8-3(dying case)

[Fig. 22]

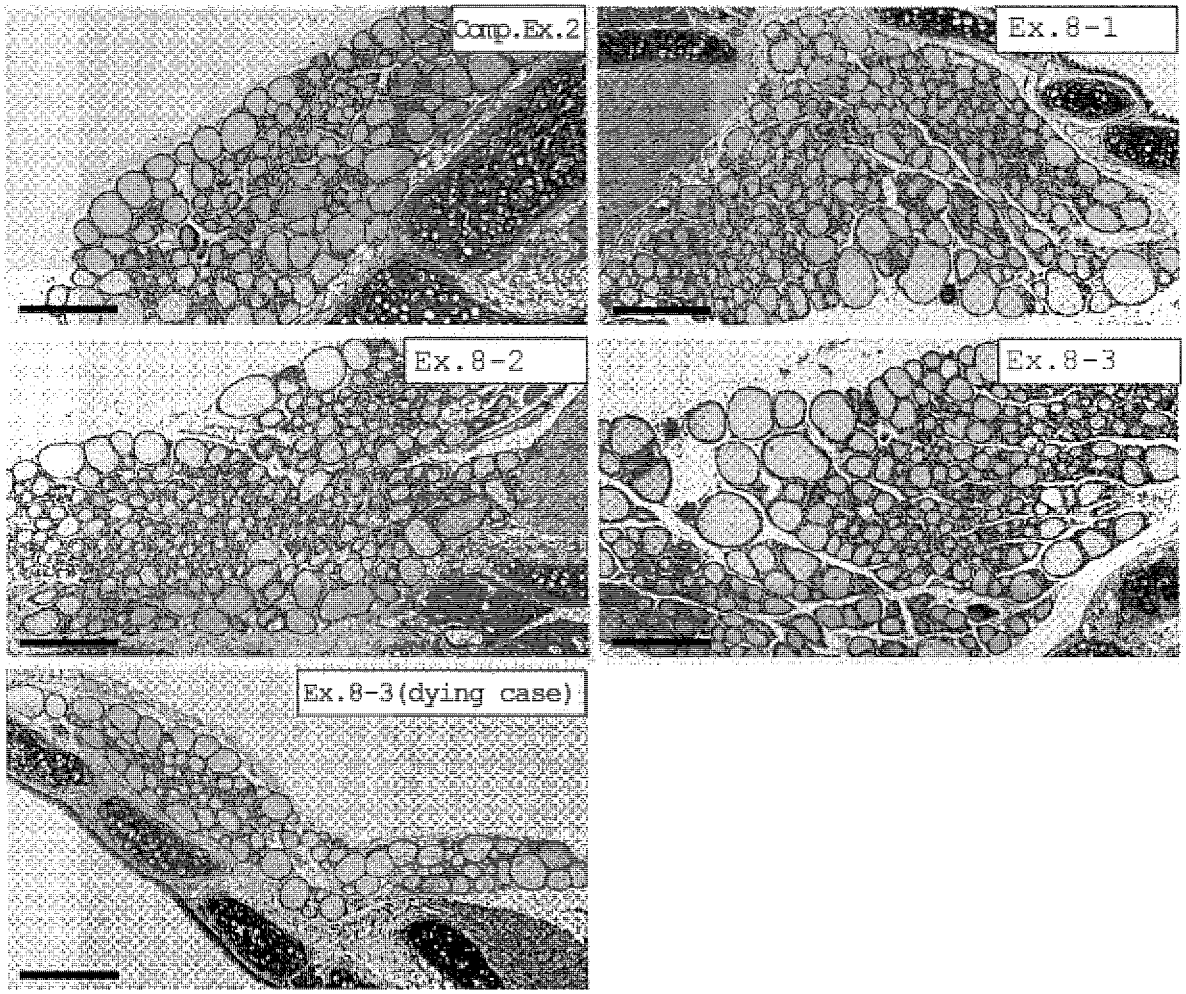

## INTERNATIONAL SEARCH REPORT

International application No.
**PCT/JP2022/018413**

**A. CLASSIFICATION OF SUBJECT MATTER**

***C07K 16/30***(2006.01)i; ***A61K 39/395***(2006.01)i; ***A61K 47/68***(2017.01)i; ***A61K 51/02***(2006.01)i; ***A61K 51/10***(2006.01)i; ***A61P 35/00***(2006.01)i; ***A61P 35/04***(2006.01)i; ***C07K 16/46***(2006.01)i; ***C12N 15/13***(2006.01)i
FI: C07K16/30; C07K16/46; A61P35/00; A61K39/395 N; A61K51/10 100; A61K47/68; A61K51/02 100; A61P35/04; C12N15/13 ZNA

According to International Patent Classification (IPC) or to both national classification and IPC

**B. FIELDS SEARCHED**

Minimum documentation searched (classification system followed by classification symbols)

C07K16/30; A61K39/395; A61K47/68; A61K51/02; A61K51/10; A61P35/00; A61P35/04; C07K16/46; C12N15/13

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2013/157102 A1 (OSAKA CITY UNIVERSITY) 24 October 2013 (2013-10-24) paragraphs [0001]-[0037] | 1-3, 12, 13 |
| Y | | 4-16 |
| Y | 遠藤啓吾，渡辺直行, ＲＩ標識モノクローナル抗体を用いた放射免疫診断の方法論とその臨床的有用性, 日本臨牀, 01 June 1996, vol. 54, no. 6, pp. 138-143 pp. 139, 140, (ENDO, Keigo. WATANABE, Naoyuki. Methodology and clinical usefulness of radioimmunoimaging using radiolabeled monoclonal antibodies. Japanese Journal of Clinical Medicine.) | 4-16 |
| A | | 1-3 |
| Y | JP 2016-504370 A (PRECISION BIOLOGICS, INC) 12 February 2016 (2016-02-12) paragraph [0282] | 4-16 |
| A | | 1-3 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 May 2022** | **31 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/018413**

**C. DOCUMENTS CONSIDERED TO BE RELEVANT**

| Category* | Citation of document, with indication, where appropriate, of the relevant passages | Relevant to claim No. |
|---|---|---|
| Y<br><br><br>A | JP 2017-503763 A (MILLENNIUM PHARMACEUTICALS, INC.) 02 February 2017 (2017-02-02)<br>paragraphs [0133], [0189]-[0222] | 10-16<br><br><br>1-9 |
| P, A | WO 2021/075544 A1 (NIHON MEDIPHYSICS CO LTD) 22 April 2021 (2021-04-22)<br>entire text, all drawings | 1-16 |

Form PCT/ISA/210 (second sheet) (January 2015)

**INTERNATIONAL SEARCH REPORT**

International application No.
**PCT/JP2022/018413**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/018413**

| Patent document cited in search report | Publication date (day/month/year) | Patent family member(s) | Publication date (day/month/year) |
|---|---|---|---|
| WO 2013/157102 A1 | 24 October 2013 | (Family: none) | |
| JP 2016-504370 A | 12 February 2016 | US 2016/0194398 A1<br>paragraph [0309]<br>WO 2014/106176 A1<br>EP 2938633 A1 | |
| JP 2017-503763 A | 02 February 2017 | US 2016/0303258 A1<br>paragraphs [0198], [0253]-[0277]<br>WO 2015/084996 A1<br>EP 3077407 A1 | |
| WO 2021/075544 A1 | 22 April 2021 | US 2021/0338852 A1<br>entire text, all drawings | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H7203974 A
- JP H115749 A
- WO 2013157102 A
- WO 2013157105 A
- WO 2016186206 A
- WO 2017217347 A
- WO 2018230257 A
- WO 2003080655 A
- WO 2011118699 A
- JP 2021072201 A


### Non-patent literature cited in the description


- *Japanese Journal of Cancer Research,* 1996, vol. 87, 977-984
- *Japanese Journal of Clinical Medicine,* 2006, vol. 64 (1), 274-278
- *Japanese Journal of Cancer Research,* 1999, vol. 90, 1179-1186
- *PLoS ONE,* December 2011, vol. 6 (12), 1-10